# EUROPEAN PATENT APPLICATION

(11) **EP 4 763 835 A1**
(43) Date of publication of application: **24.06.2026**
(21) Application number: 24307227.9
(22) Date of filing: 20.12.2024
(51) Int. Cl.: C07D 305/04, C07D 405/12, C07C 50/00, A61P 33/00, A61K 31/337, A61K 31/4427

(54) **POLYAROMATIC OR HETEROAROMATIC COMPOUNDS EXHIBITING ANTI-PLASMODIAL ACTIVITIES**

(71) Applicant: Centre National de la Recherche Scientifique, 75016 Paris (FR); Swiss Tropical And Public Health Institute, 4123 Allschwil (CH); Universität Basel, 4003 Basel (CH); Institut National de la Santé et de la Recherche Médicale, 75013 Paris (FR); Université de Strasbourg, 67000 Strasbourg (FR); Université de Haute Alsace, 68093 Mulhouse Cedex (FR)
(72) Inventor: DAVIOUD-CHARVET, Elisabeth, 59840 Pérenchies (FR); RICHARD, Jimmy, 67000 Strasbourg (FR); BLANDIN, Stéphanie, 67100 Strasbourg (FR); GOETZ, Alice-Anne, 67100 Strasbourg (FR); FEUFACK DONFACK, Lionel Brice, 67100 Strasbourg (FR); MÄSER, Pascal, 4153 Reinach (CH); ROTTMANN, Matthias, 79589 Binzen (DE)
(74) Representative: Novagraaf Technologies

(57) **Abstract**

Compounds of formula (I): 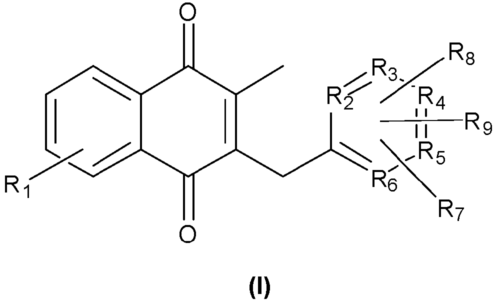 or their pharmaceutically acceptable salts, hydrates, solvates, esters or their optical isomers, racemates, diastereoisomers, enantiomers, tautomers, or a mixture thereof; said compounds exhibits anti-plasmidal activities, in particular for the treatment of malaria diseases.

## Description

### FIELD OF THE INVENTION

The present invention relates to compounds comprising a naphthoquinone (CAS:130-15-4) scaffold, and more particularly but not exclusively, to compounds comprising similarities with the known compound named plasmodione and encompassing the compounds in which the naphthoquinone group bears a methyl function and a benzyl skeleton. The invention relates also to the use of such compounds for the treatment of diseases involving parasites, and preferably parasites of the genus *Plasmodium.* The invention also relates to pharmaceutical compositions comprising said compounds as active ingredient. In particular the compounds of the invention comprising the naphthoquinone scaffold are used for the treatment of malaria.

### BACKGROUND OF THE INVENTION

Malaria remains one of the most severe infectious diseases that disproportionally affects the public health and economic welfare of the world's poorest communities. The causative agents of malaria are protozoan parasites of the genus *Plasmodium.* Our current reliance on Artemisinin-based Combination Therapy (ACT) is the firstline therapy for malaria worldwide. A decrease in artemisinin sensitivity, mainly in Southeast Asia, has recently been reported, thus underlining the need for further knowledge of artemisinin's molecular mechanism of action and drug resistance (World malaria report 2023, Featured publication, 30 November 2023, https://www.who.int/teams/global-malaria-programme/reports/world-malaria-report-2023). Recent investigations on mechanisms for artemisinin resistance (art-R) evidenced a role of mutations in the propeller domain of the Kelch13 protein (Ashley EA, et al., Tracking Resistance to Artemisinin Collaboration (TRAC). Spread of artemisinin resistance in Plasmodium falciparum malaria. N Engl J Med. 2014, 371(5):411-23. doi: 10.1056/NEJMoa1314981. Erratum in: N Engl J Med 2014; 371:786. doi: 10.1056/NEJMx140047). Furthermore, artemisinins have been proposed as potent inhibitors of P. *falciparum* phosphatidyl inositol-3-kinase, whereas mutated PfKelch13 reduces the natural degradation of its ligand PfPI3K after polyubiquitination (Mbengue A, et al. A molecular mechanism of artemisinin resistance in Plasmodium falciparum malaria. Nature 2015, 520(7549):683-7. doi: 10.1038/nature14412). The relative fitness of art-R parasites is of critical importance, given a broad decreased efficacy of partner drugs in ACTs, and clinical resistance to them developing at the early ring stage. The drug-induced ring stages are known as dormant rings given their ability to persist for days to weeks in a non-replicating state before recovery and normal asexual growth resumes. All previous studies suggest a more broadly adaptive fitness advantage of ring-stage dormancy for P. *falciparum* survival to stress, especially from antimalarial drugs (a- Witkowski B, et al. Increased tolerance to artemisinin in Plasmodium falciparum is mediated by a quiescence mechanism. Antimicrob Agents Chemother. 2010, 54(5):1872-7. doi: 10.1128/AAC.01636-09; b- Witkowski B, et al. Reduced artemisinin susceptibility of Plasmodium falciparum ring stages in western Cambodia. Antimicrob Agents Chemother. 2013, 57(2):914-23. doi: 10.1128/AAC.01868-12). Previous studies suggested an enhanced transmission potential of resistant parasites by earlier commitment to produce gametocytes as well a competitive advantage of resistant parasites to survive drug exposure long enough to transmit the traits to the next generation. Clearly, if art-R parasites are more fit to transmit to mosquitoes, the observed effects in the laboratories will dramatically be amplified in the field. Consequently, the potential role of second-cycle dormancy and recrudescence in resistance development should be investigated with additional novel antimalarial drugs and functional tools of epidemiological diagnostic.

Reported works evidenced that the antimalarial modes of action and resistance of artemisinin involve the production of reactive oxygen species (ROS) that are initiated by iron bioactivation of endoperoxides and/or catalysed by iron-dependent oxidative stress. In parallel, studies on artemisinin toxicity in human cells indicate that respiring mitochondria play an essential role in endoperoxide-induced cytotoxicity *via* the generation of ROS. Also, mature gametocytes are sensitive to pro-oxidative drugs, and the potential of redox-active compounds to block the transmission of *Plasmodium* to *Anopheles* mosquitoes was demonstrated. Recently, activity of nutrient permeability channels (NPCs) in Art-resistant parasites was demonstrated to influence the sensitivity to antimalarial drugs. Less Art bioactivation by Fe^{II} species was correlated with less endocytosed Hb. New redox-active drugs as partners of ACTs will certainly be essential to cure malaria and block parasite transmission to mosquitoes in the future.

Previous studies on the identification and mode of action of the antimalarial early lead compound 3-[4-(trifluoromethyl)benzyl]-menadione, henceforth called plasmodione, revealed that this drug disturbs the redox balance of the parasitized erythrocytes (a-Müller, T. et al. Glutathione reductase-catalyzed cascade of redox reactions to bioactivate potent antimalarial 1,4-naphthoquinones - A new strategy to combat malarial parasites. J. Am. Chem. Soc. 2011, 133, 11557-71. doi: 10.1021/ja201729z; b- Ehrhardt, K., et al. The redox-cycler plasmodione is a fast acting antimalarial lead compound with pronounced activity against sexual and early asexual blood-stage parasites. Antimicrob. Agents Chemother. 201660, 5146-58. doi: 10.1128/AAC.02975-15). Based on detailed physico-biochemical studies, the antimalarial selectivity of plasmodione has been proposed to come largely from its specific bioactivation involving a cascade of redox reactions starting from a benzylic oxidation (Cichocki B., et al. Plasmodium falciparum ferredoxin-NADP+ reductase-catalyzed redox cycling of plasmodione generates both predicted key drug metabolites: Implication for antimalarial drug development. ACS Infect. Dis. 2021, 7, 1996-2012, doi: 10.1021/acsinfecdis.1c00054).

The aim of the present invention is to develop the synthesis of novel compounds of the benzylmenadione family, and to investigate their activity as potential new drugs, in particular for the treatment of malaria.

### BRIEF DESCRIPTION OF THE DRAWINGS

**Figure 1** represents some compounds of formula (I);
**Figures 2A-D** represent the evaluation of the toxicity of the compounds on glucose-6-phosphate dehydrogenase (G6PD) -normal or -deficient hemizygotes;
**Figures 3A-B** represent the evaluation the transmission-blocking properties of the compounds using *P. falciparum* indirect Standard Membrane Feeding Assay;
**Figures 4A-C** represent the evaluation of the compounds on the evolution of the parasitemia of mice, and on the ability of these mice to transmit malaria parasites to mosquitoes;
**Figures 5A-B** represent the evaluation of the cross-resistance between DHA and the compounds using a series of Artemisinin-resistant parasite strains;
**Figure 6** represents drug partners tested together with compounds of the invention;
**Figure 7** represents the evaluation of drug combinations of the compounds of the invention together with drug partners on an Artemisinin-resistant parasite strain (IPC5202);
**Figure 8** represents the evaluation of drug combinations of DHA together with drug partners on an Artemisinin-resistant parasite strain (IPC5202);
**Figure 9** represents the evaluation of transmission blocking activity of drug combinations of MD40 together with drug partners using *P. falciparum* indirect Standard Membrane Feeding Assay.
**Figure 10** represents the evaluation of the *in vivo* efficacy of the orally-delivered drug combination **MD40** and artesunate on P. *falciparum* in a humanized mouse model.

### DEFINITIONS

As used hereabove or hereafter:
"Alkyl" means an aliphatic hydrocarbon group which may be straight or branched having 1 to 6 carbon atoms in the chain. "Branched" means that one or more lower alkyl groups such as methyl, ethyl or propyl are attached to a linear alkyl chain. Exemplary alkyl groups include methyl, ethyl, n-propyl, i-propyl, n-butyl, t-butyl, n-pentyl, 3-pentyl.
"Alkoxyl" means an O-alkyl group, the alkyl group being as described above.
"Alken" or "alkenyl" means an aliphatic hydrocarbon group containing a carbon-carbon double bond and which may be straight or branched having 2 to 6 carbon atoms in the chain. Preferred alkenyl groups have 2 to 6 carbon atoms in the chain; and more preferably about 2 to 4 carbon atoms in the chain. Exemplary alkenyl groups include ethenyl, propenyl, *n*-butenyl, *i*-butenyl, 3-methylbut-2-enyl, *n*-pentenyl.
"Aryl" means an aromatic monocyclic or multicyclic hydrocarbon ring system of 6 to 14 carbon atoms, preferably of 6 to 10 carbon atoms, substituted or not. Exemplary aryl groups include phenyl or naphthyl.
"Halogen atom" refers to fluorine, chlorine, bromine or iodine atom; preferably fluorine atom.
"Haloalkyl" refers to an alkyl group as described above substituted by at least one halogen atom as described above, for example substituted by 1 to 6 halogen atoms, preferably by 1 to 3 halogen atoms. Exemplary haloalkyl group includes trifluoromethyl group.
"Haloalkoxyl" refers to an alkoxyl group as described above substituted by at least one halogen atom as described above, for example substituted by 1 to 6 halogen atoms, preferably by 1 to 3 halogen atoms, for example -OCF₃.

As used herein, the term "heteroaryl" refers to a 5 to 14, preferably 5 to 10-membered aromatic hetero, mono-, bi- or multicyclic ring comprising at least one heteroatom preferably chosen among: O, N or S, preferably from 1 to 5 heteroatoms, for example from 1 to 4 heteroatoms, expecially one, two or three heteroatoms. Examples include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazoyl, tetrazolyl, isoquinolyl, benzothienyl, isobenzofuryl, pyrazolyl, carbazolyl, benzimidazolyl, isoxazolyl, pyridyl-N-oxide, as well as the fused systems resulting from the condensation with a phenyl group.

"Alkyl", "aryl", "alkoxyl", "haloalkoxyl", "haloalkyl", "heteroaryl" and the likes refers also to the corresponding "alkylene", "arylene", "alkoxylene", "haloalkoxylene", "haloalkylene" "heteroarylene" and the likes which are formed by the removal of two hydrogen atoms. Alkyl and alkylene are used herein interchangeably.

As used herein, the expression "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like, including mono, di or tri-salts thereof; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 20th ed., Mack Publishing Company, Easton, PA, 2000, the disclosure of which is hereby incorporated by reference.

The compounds that will be further presented in the context of the invention having geometrical and stereoisomers are also part of the invention.

The compounds of the present invention may be prepared in a number of ways well-known to those skilled in the art. The compounds can be synthesized, for example, by application or adaptation of the methods known by the skilled person. The appropriate modifications and substitutions will be readily apparent and well known or readily obtainable from the scientific literature to those skilled in the art.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are covered, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes as shown in the examples, which can be adapted by the skilled person based on its knowledge with regard to the desired compounds.

The present invention also relates to pharmaceutical compositions comprising at least one compound as active principle. Preferably, the present invention also relates to pharmaceutical compositions comprising at least one compound as active principle and at least one excipient pharmaceutically acceptable.

According to the invention, the terms "patient" or "patient in need thereof', are intended for an animal such as a valuable animal for breeding, company or preservation purposes, or a human or a human child, being affected or likely to be affected with one or more disesases and conditions described herein. Preferably, the patient is human.

The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

As used herein, a "therapeutically effective amount" refers to an amount of a compound of the present invention which is effective in preventing, reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of formula (I), which is required to achieve the desired biological effect, will vary depending upon a number of factors, including the chemical characteristics of the compounds employed, the potency of the compounds, the type of disease, the species to which the patient belongs, the diseased state of the patient, the route of administration, the bioavailability of the compound by the chosen route, all factors which dictate the required dose amounts, delivery and regimen to be administered.

As used herein, "pharmaceutically acceptable excipient" includes any carriers, diluents, adjuvants, or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well-known in the art. Except insofar as any conventional media or agent is incompatible with the active ingredient, its use in the therapeutic compositions is contemplated. Supplementary active ingredients can also be incorporated into the compositions as suitable therapeutic combinations.

In the context of the invention, the term "treating" or "treatment", as used herein, means reversing, alleviating, inhibiting the progress of, or preventing the disorder or condition to which such term applies, or one or more symptoms of such disorder or condition.

The preferred dosage of drug to be administered is likely to depend on such variables as the type and extent of progression of the disease or disorder, the overall health status of the particular patient, the relative biological efficacy of the compound selected, the formulation of the compound, the route of administration (intravenous, intramuscular, or other), the pharmacokinetic properties of the compound by the chosen delivery route, and the speed (bolus or continuous infusion) and schedule of administrations (number of repetitions in a given period of time).

The compound of the present invention is also capable of being administered in unit dose forms, wherein the expression "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. Compounds provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via trans-dermal patches.

The compositions may conveniently be administered in unit dosage form and may be prepared by any of the methods well-known in the pharmaceutical art.

For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolved, modified-release or sustained-release preparations and formulations, and wherein such sustained-release formulations are preferably bi-modal. Preferred tablets contain lactose, cornstarch, magnesium silicate, croscarmellose sodium, povidone, magnesium stearate, or talc in any combination.

Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for airway administration or inhalation, which include such means as dry powder, aerosol, spray or drops. They may be aqueous solutions or microdroplets containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

In the present invention "compound X for the treatment of Y" is equivalent to "compound X for use in a method for the treatment of Y" or "compound X for use in the therapy of Y".

The present invention also relates to the use of at least one compound of the invention or of the composition of the invention for the preparation of a medicine for the treatment of diseases.

The present invention also relates to a method of treatment of viral diseases comprising the administration of a therapeutically effective amount of at least one compound of the invention or of the composition of the invention to a patient in need thereof.

### BRIEF DESCRIPTION OF THE INVENTION

The present invention relates to polyaromatic and heteromatic compounds belonging to the class of 2-methyl-1,4-naphthoquinone derivatives and more particularly 3-benzylmenadiones, possibly substituted at C-6 (also simply named "menadione" with the CAS number: 58-27-5) corresponding respectively to the following compounds:

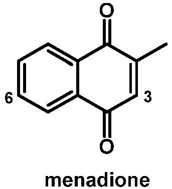

More particularly, the present invention relates to the menadione derivatives defined in the context of the invention by the compound having in common with the known compound named "plasmodione" a benzyl moiety at C-3; plasmodione referring to a compound according to one of these three regiomers:

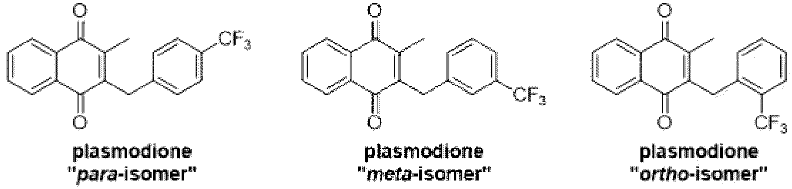

The present invention relates advantageously to a compound of formula (I): 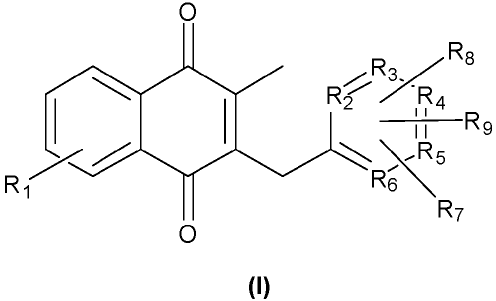 in which:
- R₁ represents -H or one substituent select among:
   a -CN; -NO₂, -NH₂, a methyl group or a halogen atom, and preferably the halogen is a -F atom;
   =R₂- ; =R₃; =R₄- ; =R₅- and =R₆- represent carbon atoms, with the exception that at least one of said carbon atoms is eventually replaced by one heteroatom selected among =P- or =N- , and preferably a =N-;
- R₇ represents -CF₃; and
- R₈ and -R₉ represent independently a substituent comprising at least a functional group selected among an ether group or an amine group, and represent preferably a substituent according to the chemical structure in between the following brackets:

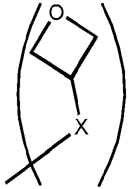

where -X- represents a chemical group comprising a heteroatom such as -NH-; -PH-; -S- or -O-, and preferably -NH or -O-;
or one of its pharmaceutically acceptable salts, hydrates, solvates, esters or one of its optical isomers, racemates, diastereoisomers, enantiomers, tautomers, or a mixture thereof.

Preferably, the compound according to the present invention is a compound according to formula (I'): 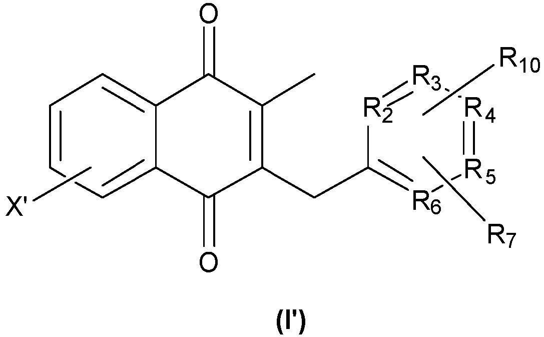 in which =R₂- ; =R₃ ; =R₄- ; =R₅- and =R₆- represent carbon atoms, with the exception that one or two, preferably one, of said carbon atoms is eventually replaced by a =N-;
- X'- represents a -H or a -F;
- R₇ represents -CF₃; and
- R₁₀ represents -NH₂ or a substituent according to the chemical structure in between the following brackets: 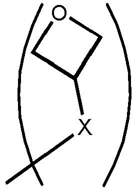
- X- represents -NH or -O-.

Preferably, said compound is according to formula (I') with:
=R₂- ; =R₄- and =R₆- represent carbon atoms, and =R₃ or =R₅- represents a carbon atom or a =N- atom; and preferably =R₂- ; =R₃ ; =R₄- ; =R₅- and =R₆- represent carbon atoms; and
-R₁₀ represents -NH₂ or a substituent according to the chemical structure in between the following brackets: 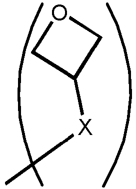 -X- represents -NH-.

Preferably, said compound is according to formula (I') with:
=R₂- ; =R₄- and =R₆- represent carbon atoms, and =Rs- or =R₅- represents a carbon atom or a =N- atom; and preferably =R₂- ; =R₃ ; =R₄- ; =R₅- and =R₆- represent carbon atoms; and
-R₁₀ represents a substituent according to the chemical structure in between the following brackets: 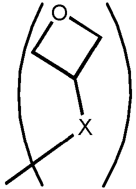
-X- represents -O-.

Preferably, said compound according to the invention has a structure selected among one of the following formulae :

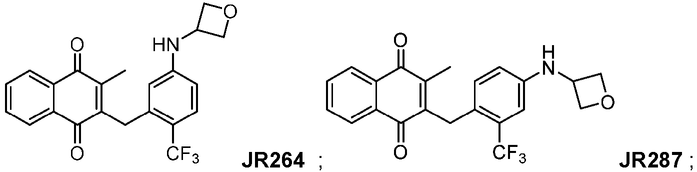

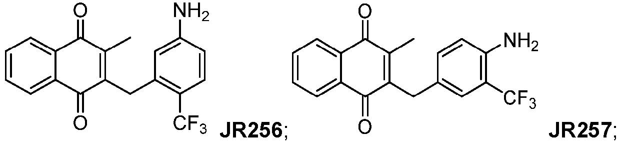

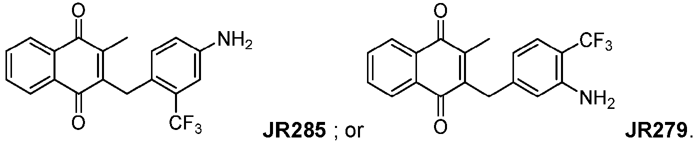

Preferably, said compound of the invention is according to one of the following formulae :

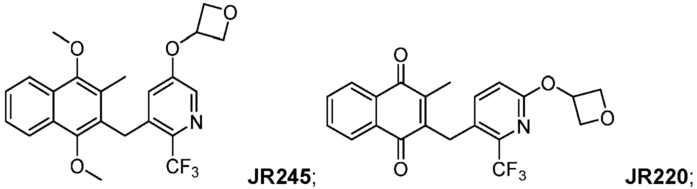

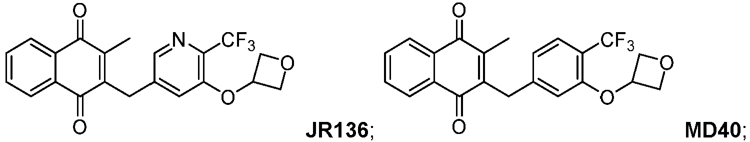

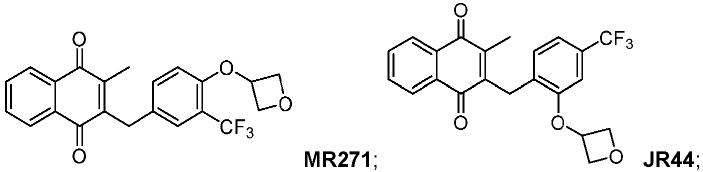

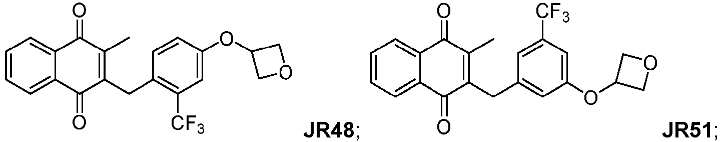

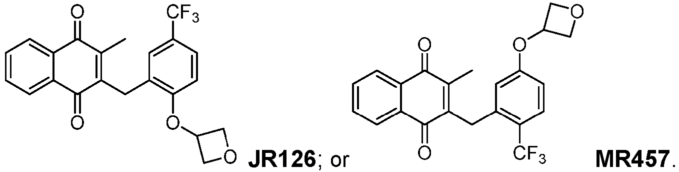

Preferably, said compound is according one of the following formulae:

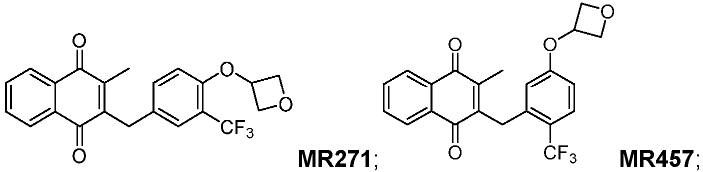

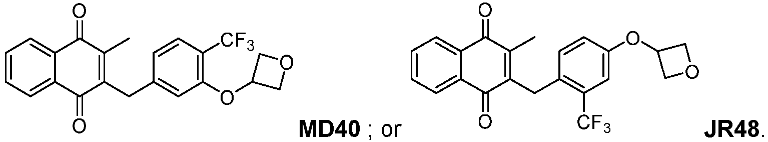

The present invention also relates to a compound according to any compounds precedently presented in the context of the present invention for use as a medicament.

Preferably, said compound is for use in the treatment of a malaria, and preferably in the treatment of a multidrug resistant malaria.

Preferably, said compound is for use in the treatment of an artemisinin-resistant malaria.

Preferably, said compound is for use in the treatment of a disease or disorder implying anti-plasmodial activities, preferably against P. *falciparum* asexual and sexual stages.

The present invention also relates to a pharmaceutical composition comprising at least one compound according to the compound described in the context of the invention, and at least one additional compound selected among the followings:
- an endoperoxide preferably selected among artemisinin or its metabolite dihydroartemisinin (DHA), artemether, arteether, artesunate,
- a peroxide belonging to 1,2,4-trioxanes or 1,2,4-trioxolanes, preferably selected among an ozonide derivative selected among ozonide OZ277 (arterolane) and ozonide OZ439 (artefenomel);
- a peroxide belonging to 1,2,4,5-tetraoxanes, preferably selected among RKA182 or RKA216;
- a natural bicyclic peroxide, preferably selected among 1,2-dioxanes plakortin or G3 factors;
- a compound selected among FR235222, XM172, ladanein (XM184), XM183, miconazole or econazole; and
preferably said additional compound is selected among artemisinin; artesunate arterolane and artefenomel.

For a complete description of the compounds artemisinin, artesunate, OZ277 (arterolane), ozonide OZ439 (artefenomel); RKA182 or RKA216, and more generally for an overview of 1,2,4-trioxanes, 1,2,4-trioxolanes and 1,2,4,5-tetraoxanes compounds, see the article from. Monika Shukla, et al. An overview on the antimalarial activity of 1,2,4-trioxanes, 1,2,4-trioxolanes and 1,2,4,5-tetraoxanes. Med Res Rev. 2024 Jan;44(1):66-137.

For a complete description of the compound named plakortin, see the article from. Fattorusso C, et al. Endoperoxide derivatives from marine organisms: 1,2-dioxanes of the plakortin family as novel antimalarial agents. J Med Chem. 2006 Nov 30;49(24):7088-94. doi: 10.1021/jm060899g; or korokhod et al. Oxidative stress-mediated antimalarial activity of plakortin, a natural endoperoxide from the tropical sponge Plakortis simplex. Free Radic Biol Med. 2015 Dec;89:624-37. doi: 10.1016/j.freeradbiomed.2015.10.399.

For a complete description of the G3 factors, see the article from. Ruiz J, et al. Antimalarial bicyclic peroxides belonging to the G-factor family: mechanistic aspects of their formation and iron (II) induced reduction. Curr Top Med Chem. 2014;14(14):1668-83. doi: 10.2174/1568026614666140808154326

Preferably, said pharmaceutical composition comprises optionally a pharmaceutical acceptable excipient.

### DETAILED DESCRIPTION OF CERTAIN EXEMPLARY EMBODIMENTS OF THE INVENTION

### Part I. Purification and chemical study of the molecules-Biochemical evaluation of the molecules

### Material and methods:

Commercially available reagents and solvents were used as purchased without purification. Dichloromethane was purchased anhydrous. THF was was purchased anhydrous. Column chromatography was performed with silica gel from Merk (silica gel 60, 0.04-0.063 nm).

Proton nuclear magnetic resonance (¹H NMR) spectra were recorded on 400 or 500 MHz Bruker instruments. Data are presented as follows: chemical shift, multiplicity (s = singlet, d = doublet, t = triplet, q = quartet, p = pentuplet, sext = sextet, m = multiplet), coupling constants (J in Hz) and integration. Chemical shifts were determined by taking the solvent as a reference: CHCl₃ (7.26 ppm), MeCN (1.94 ppm), MeOH (3.31 ppm), Me₂SO (2.50 ppm), Me₂CO (2.05 ppm) for ¹H NMR and CHCl₃ (77.2 ppm), MeCN (1.3 ppm), Me₂SO (39.5 ppm), Me₂CO (29.8 ppm) for ¹³C NMR.

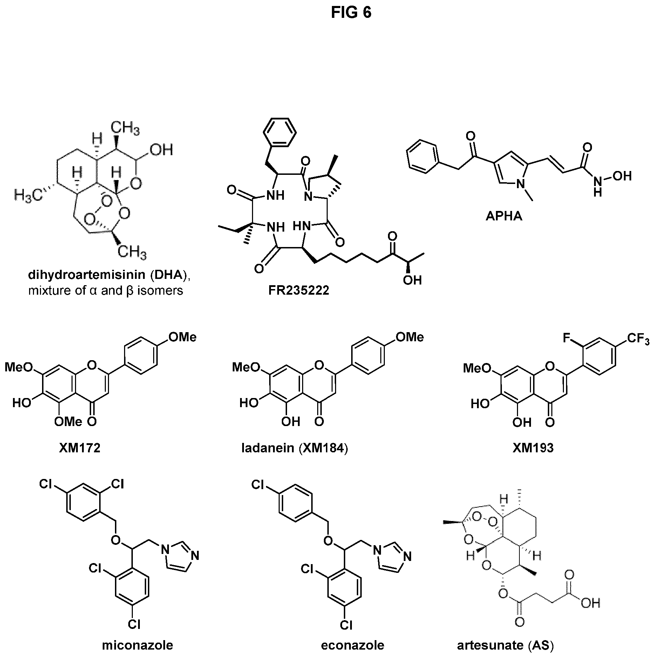

**Compounds: characterisation and dilutions.** Compounds were dissolved in DMSO at 10 mM (STPH) or 6 mM (IBMC) except for methylene blue that was dissolved in water (6 mM). The stocks were kept at 4°C for the time required to perform repeats (usually ≥2 weeks), or stored at -20°C when not used directly. Partner compounds (Fig. 6) were obtained from several laboratories: MD40, Plasmodione, XM172, XM184 and XM193 were synthesized by the Davioud-Charvet lab. PD was included as reference compound(s) in every experiment. Chloroquine Diphosphate (Sigma C6628), Artesunate (Mepha), dihydroartemisinin (Sigma D7439), methylene blue (Sigma M9140), (±)-Miconazole nitrate salt (Sigma-Aldrich M3512) and Econazole nitrate salt (Sigma-Aldrich E4632) were commercially available. APHA and FR235222 were obtained from the Hakimi lab (Institut pour l'Avancée des Biosciences, Grenoble, France).

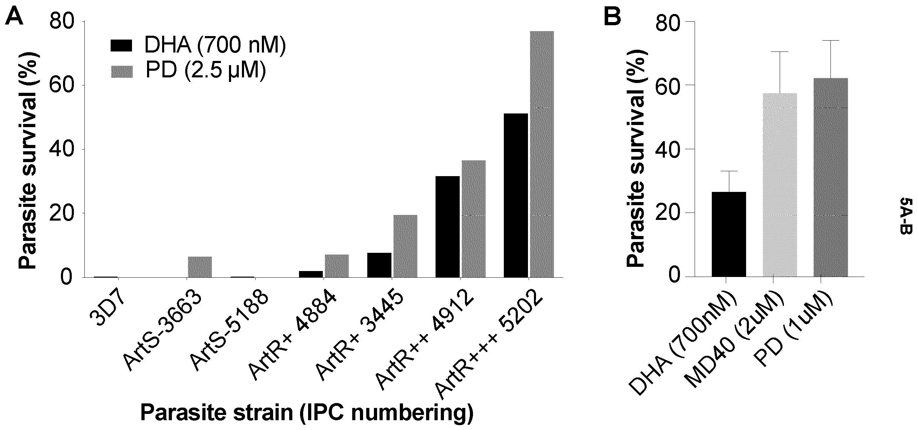

***P. falciparum* culture (IBMC and STPH).** The chloroquine-sensitive *P*. *falciparum* strains NF54 and 3D7 (isolated in the Netherlands and available at MR4), the chloroquine-resistant strain Dd2 (obtained from the Lanzer laboratory, Heidelberg Universität, Germany) and a series of lines isolated in Cambodia (available at MR4) displaying variable sensitivity to artemisinin (see Fig. 5), were grown in continuous culture as described by Trager and Jensen (Trager, W. et AL. Human malaria parasites in continuous culture. Science 193, 673-675 (1976)) with slight modifications (Akoachere, M. et al. In vitro assessment of methylene blue on chloroquine-sensitive and -resistant Plasmodium falciparum strains reveals synergistic action with artemisinins. Antimicrob. Agents. Chemother. 49, 4592-4597 (2005)). Unless otherwise stated, parasites were maintained at 1 to 5% parasitaemia and 2.8 to 3.3% haematocrit in an RPMI 1640 culture medium supplemented with A+ erythrocytes, 0.25% lipid-rich bovine serum albumin (Albumax), 5% human serum, 9 mM (0.16%) glucose, 0.2 mM hypoxanthine, 2.1 mM L-glutamine, and 22 µg/ml gentamicin. All incubations were carried out at 37 °C in 3% O₂, 5% CO₂, and 92% N₂ (STPH) or in 5% O₂, 5% CO₂, and 92% N₂ (IBMC).

***Plasmodium falciparum* antiplasmodial [³H]-hypoxanthine incorporation-based assays with the NF54 strain (STPH, Basel).** The *Plasmodium falciparum* growth inhibition assay was used to determine the 50% inhibitory concentration (IC₅₀) of a given compound. In brief, naive parasites of strain NF54 were exposed to a serial dilution of compounds for 72 hours. The readout method is based on incorporation of radiolabelled [³H]-hypoxanthine into the parasites DNA, which serves as indicator of parasite growth (Desjardins RE, et al. 1979. Quantitative assessment of antimalarial activity in vitro by a semiautomated micro dilution technique. Antimicrob Agents Chemother 16:710-8; Snyder C, Chollet J, et al. 2007. In vitro and in vivo interaction of synthetic peroxide RBx11160 (OZ277) with piperaquine in Plasmodium models. Exp Parasitol 115:296-300).The P. *falciparum* growth inhibition assay method described here was based on the protocol published by Snyder et al. (2007). For the assay, 4-fold concentrated solutions of all compounds were prepared freshly in screening medium. Parasite growth following drug treatment is quantified via the incorporation of [³H]-hypoxanthine as previously described. After 48 h, a [³H]-hypoxanthine (0.25 µCi) solution is added to each well, and the plates are incubated for another 24 h. Plates are harvested with a Betaplate cell harvester (Perkin^{®}, Elmer^{®}, Waltham^{®}, US), which transfers the lysed red blood cells onto a glass fiber filter (Microbeta FilterMate^{™}). The dried filters are inserted into plastic foil with 10 mL of scintillation fluid and counted in a Betaplate liquid scintillation counter (Perkin Elmer^{®}, Waltham^{®}, US). The results are recorded as counts per minute per well at each compound concentration. Data are transferred into a graphics program (e.g., Excel) and expressed as percentage of the values for untreated controls (Radohery GFR, et al. Parasite Viability as a Measure of In Vivo Drug Activity in Preclinical and Early Clinical Antimalarial Drug Assessment. Antimicrob Agents Chemother. 2022, 66(7):e0011422).

The fifty percent inhibitory concentration (IC50) was then calculated by linear interpolation as described (Huber W., et al. A comparison of three methods of estimating EC50 in studies of drug resistance of malaria parasites, Acta Trop 1993, 55, 257-261).

***Plasmodium falciparum* ring culture and antiplasmodial SYBR^{®} green staining-based assays with the Dd2, ArtS and ArtR strain (IBMC).** Antimalarial activity is expressed by the IC₅₀ value, i.e. the drug concentration at which half of the parasites survive. The antimalarial activity of the compounds on asexual stages of parasites was evaluated on three P. *falciparum* strains: Dd2 a chloroquine-resistant strain, and ArtS (IPC5188) and ArtR (IPC5202), that are respectively artemisinin-sensitive and resistant. Parasites were treated for 72 h-drug, starting on 3 h-old ring stage. For this, schizonts of highly synchronous parasite cultures were separated using 60% percoll gradient, and the mature segmented schizonts were incubated with non-infected red blood cells for 3 h to allow reinvasion. The ring stage parasites produced during these 3 h were then synchronized with 5% sorbitol to kill mature stages. These 3 h-old rings were exposed to the compounds for 72 h with decreasing concentrations of the compounds in microtiter plates (0.5% parasitemia, 1.5% haematocrit). Each inhibitor was analysed in a three-fold serial dilution with technical duplicates in at least three independent experiments. Parasite replication was assessed by fluorescent SYBR^{®} green staining of parasitic DNA (Smilkstein M, et al. Simple and inexpensive fluorescence-based technique for highthroughput antimalarial drug screening. Antimicrob Agents Chemother 2004, 48:1803-1806. doi: 10.1128/AAC.48.5.1803-1806.2004) as previously described (Ehrhardt K, et al. The antimalarial activities of methylene blue and the 1,4-naphthoquinone 3-[4-(trifluoromethyl)benzyl]-menadione are not due to inhibition of the mitochondrial electron transport chain. Antimicrob Agents Chemother 2013, 57:2114-2120. doi: 10.1128/AAC.02248-12; Beez D, et al. Genetic predisposition favors the acquisition of stable artemisinin resistance in malaria parasites. Antimicrob Agents Chemother 2011, 55:50-55. doi: 10.1128/AAC.00916-10). After 72 h, the parasites were frozen at -80 °C, thawed and a fluorescent SYBR green assay was run to assess parasite survival by fluorescence on a Varioscan plate reader (excitation at 497 nm and emission at 521 nm). The parasitemia at T0 and T72 was determined by microscopic blood smear analysis after Giemsa staining of non-treated samples to confirm proper growth and multiplication factor of parasites over time. Fluorescence for each sample was expressed as a percentage of the non-treated controls ([[Fₛₐₘₚₗₑ - backgroundₛₐₘₚₗₑ] - [F_{niRBC} - background_{niRBC}]] / [[F_{control} - background_{control}] - [F_{niRBC} - background_{niRBC}]]), and IC₅₀ values were calculated using GraphPad Prism (log(inhibitor) vs. normalized response - Variable slope).

**Cytotoxicity assays with the rat L6 cell line (STPH).** Compounds were also tested against L6 rat skeletal myoblasts to evaluate their selectivity. Cytotoxicity assays for all compounds against mammalian cells (L6 rat skeletal myoblasts) were performed according to established protocols. L6 cells were seeded into RPMI 1640 medium supplemented with L-glutamine 2 mM, HEPES 5.95 g/L, NaHCOs 2 g/L and 10% fetal bovine serum in 96 well microtiter plates (4,000 cells/well). Alamar blue solution (10 µL, 12.5 mg resazurin dissolved in 100 mL distilled water) weas then added to each well and incubation continued for a further 2-4 hours. The plate was then read in a Spectramax Gemini XS microplate fluorometer (Molecular Devices Corporation, Sunnyvale, CA, USA) using an excitation wavelength of 536 nm and emission wavelength of 588 nm. Fluorescence development was measured and expressed as percentage of the control. Data were transferred into the graphic programme Softmax Pro (Molecular Devices) which calculated CC₅₀ values.

### Cytotoxicity assays on human cells.

*Cell culture:* All manipulations are carried out under sterile conditions in a type II category 2 laminar flow hood (FASTER model SafeFAST Top 212s) in a type L2 cell culture laboratory.

The HepG2 cell line (ATTC HB-8065 lot 58492105) is grown in Minimum Essential Medium Eagle (SIGMA M5650) supplemented with 10% undecomplemented Foetal Bovine Serum (INVITROGEN 10270-106), 2 mM L-Glutamine (SIGMA G7513), 100 U/mL Penicillin / 100 µg/mL Streptomycin (SIGMA P0781) and 1 mM Sodium Pyruvate (INVITROGEN 11360-039). Cells are cultured in a humidified 37°C incubator in a 95% air - 5% CO₂ atmosphere. Cells are detached with 1X Trypsin EDTA from a culture flask at 80% cell confluence. Trypsin action is inhibited by adding 2 volumes of complete medium. After centrifugation of the cell suspension, a cell count is performed using a cell counter (TC20 Automated Cell Counter BIORAD). HepG2 cells are diluted and seeded in 96-well culture plates (GREINER 655090) at 100 µL/well (15,000 cells/well). Cells are incubated overnight at 37°C with 5% CO₂.

*Materials used for drug assays:* DMSO SIGMA D4540-100mL lot BCBT0803, Chlorpromazine SIGMA C8138-5G lot 040M1656. All compounds solubilized correctly in their specific solvent at 10-2 M (CQ insoluble in DMSO). *Compound dilutions:* Compounds were prepared 2X concentrated (120 - 60 - 20 - 6 - 2 - 0.6 - 0.2 - 0.06 µM / DMSO 1%) in HepG2 cell-specific complete medium. *Cell treatment:* 100 µL of each compound concentration was applied to cells in plates. Cells were incubated for 72 h at 37°C with 5% CO₂.

*Quantifying cell viability by IncuCyte (ESSEN BIOSCIENCE):* The IncuCyte, a cell imaging device, is used to perform cell proliferation kinetics under standard culture conditions in a controlled atmosphere (37°C; 5% CO₂), quantifying cell confluence (Single, A. and al. (2015). A Comparison of Real-Time and Endpoint Cell Viability Assays for Improved Synthetic Lethal Drug Validation. J Biomol Screen.;20(10):1286-1293.). After treatment, cell plates were placed in the IncuCyte. Phase contrast images are acquired every 4 hours for 72 h at 37°C under 5% CO₂. Cell confluence can be assessed using the instrument's software. Quality control and validation of experiments: All experiments were carried out in an ISO 9001 and NF X50-900 certified environment.

The validity of the test is verified by measuring the statistical factor Z' (ref .3). It is calculated according to the formula: Z'= 1 - (3(ET+ + ET-) / (moy+ - moy-) The test is validated if Z' > 0.5, with ET+ = maximum signal control standard deviation (cells not treated with 0.5% DMSO) and ET- = minimum signal control standard deviation (cells treated with 50 µM Chlorpromazine DMSO 0.5%), moy+ = mean maximum signal control (cells untreated with 0.5% DMSO), moy- = mean minimum signal control (cells treated with 50 µM Chlorpromazine DMSO 0.5%). Chlorpromazine is an internal active control. This molecule is known to be cytotoxic (EC50 on HepG2 ≈ 24 µM). EC50s were calculated with Prism software (v 6.07). Cell confluence was quantified over time (every 4 hours) using IncuCyte. This measurement reflects cell viability. Observation of photos of the cells confirms confirm whether they are alive or not. The values obtained after 72h of treatment are compared with those of controls with solvent (DMSO 0.5%) and expressed as a percentage according to the formula: % confluence/DMSO 0.5% = (value with sample/average control) X 100.

**Gametocytocidal activity against *P*. *falciparum* (IBMC).** Sensitivity of *P*. *falciparum* gametocytes to **MD40** and plasmodione was determined by measuring luciferase activity in luciferase-expressing gametocytes after an exposure of 72 h to different concentrations of the compounds and following the protocol by Adjalley et al. (Adjalley, S.H. et al. Quantitative assessment of Plasmodium falciparum sexual development reveals potent transmission-blocking activity by methylene blue. Proc. Natl. Acad. Sci. U.S.A. 108, E1214-E1223 (2011)) Briefly, the *pfs16-GFP-Luc* (NF54 background) parasite strain was maintained at 1 to 10% parasitaemia in an RPMI 1640 culture medium supplemented with A+ erythrocytes (2% haematocrit), 10% decomplemented human serum, 9 mM (0.16%) glucose, 0.2 mM hypoxanthine, 2.1 mM L-glutamine, and 20 µg/ml gentamicin. Asexual blood stage parasites were first synchronized by 5% D-sorbitol (wt/vol) treatment for 10 min at 37 °C for two or more successive growth cycles. Synchronous parasites at 3% haematocrit were cultured in 10 cm Petri dishes to 10% parasitaemia (ring stage), at which point gametocytogenesis was induced by feeding cultures with a mixture of conditioned and fresh media (1:1). The next day, trophozoite cultures were diluted fourfold. N-acetyl glucosamine (NAG; Sigma-Aldrich) was added to a final concentration of 50 mM after all schizonts had ruptured. NAG treatment was maintained for the next two to three cycles of reinvasion to eliminate all asexual forms from the culture. Immature gametocyte stages (I and II) were purified on a Percoll gradient (Fernandez, V. Separation of Plasmodium falciparum mature stages in Percoll/sorbitol gradients. In Methods in malaria research (eds. Moll, K., Perlmann, H., Scherf, A. & Wahlgren, M.) 26-27 (American Type Culture Collection, Manassas, Virginia, 2008). and incubated directly in 96-well plates at 5-6% gametocytaemia (150 µl/well). Gametocyte cultures were exposed for 72 h to dilutions of **MD40,** plasmodione, methylene blue (MB) or DMSO (0.5%) starting at day 3, 8, or 11 after gametogenesis induction. Following drug exposure, parasites were cultivated for two additional days in normal medium before centrifugation and freezing at -80°C, and luciferase activity was measured. For this, plates were thawed at 37 °C. The cells were lysed at room temperature in 10 µl 1x luciferin lysis buffer (Luciferase Cell Culture Lysis 5x reagent, Promega), and 100 µl luciferase assay substrate was added to each well (Luciferase Assay System, Promega). Luciferase activity was measured on a luminescence plate reader (Mithras LB940 luminometer, Berthold Technologies). The mean of the luciferase activity was calculated for each technical triplicate and normalized as follows: (Lucₓ - Luc_{niRBC})/(Luc_{DMSO} - Luc_{niRBC}) with Lucₓ and LuC_{DMSO} = mean luciferase activity for parasites incubated with compound x and with DMSO, respectively, and Luc_{niRBC} = mean luciferase activity of non-infected red blood cells (background). IC₅₀ values were calculated using Prism (GraphPad, log(inhibitor) vs. normalized response-variable slope) in three independent experiments.

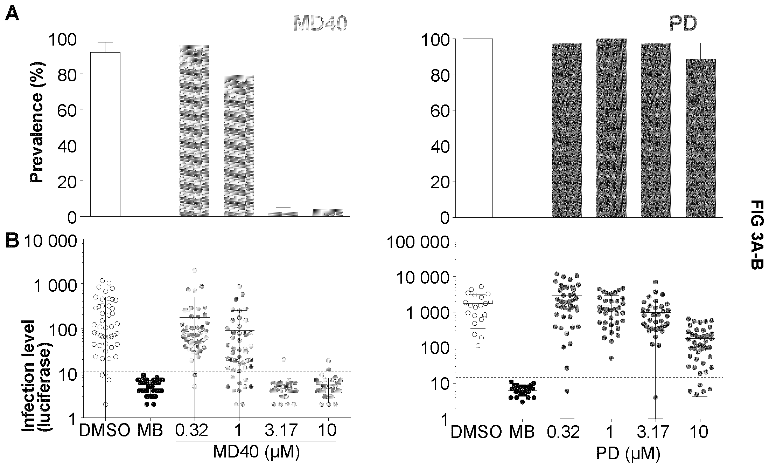

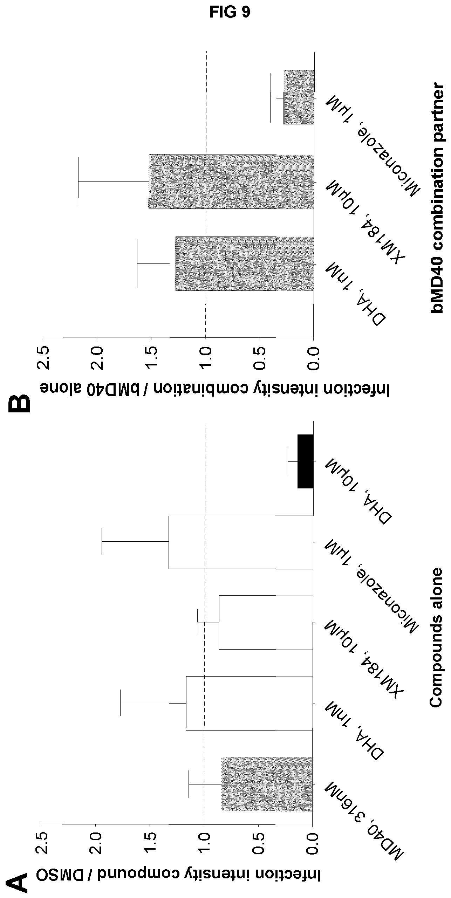

**Transmission blocking activity against P. *falciparum* (TroplQ, Njemegen).** Gametocyte induction and production was achieved using a semi-automated system that automatically changes the culture medium twice a day and maintains cultures under standard conditions of 5% O2, 5% CO2 and 90% N2 for 16-18 days. Stage V gametocytes were used for experimental infections of *A*. *stephensi* mosquitoes using indirect Standard membrane Feeding Assay (iSMFA). Gametocytes were pre-incubated for 24 hours at 37°C with compounds at different concentrations (Fig. 3), with combinations of drugs at selected concentrations (see **Table 5,** **Fig. 9****),** and with controls (MB or DHA at 10 µM) before being fed to mosquitoes (final DMSO concentration of 0.1%). 24h post incubation, the gametocyte samples were mixed with healthy red blood cells (RBCs) and centrifuged for 20 seconds at 10,000g. The pellet was resuspended in human type A serum containing the compound at the appropriate concentration (equal volume of RBC and serum). The mixture was then immediately offered to 50 female mosquitoes by artificial membrane gorging for 10 minutes. After the blood meal, non-fed or partially-fed mosquitoes were removed from the cage, while gorged mosquitoes were kept at 26°C, with 80% humidity. At 8 days post-infection, the presence of oocysts in the midgut of infected mosquitoes was assessed by luciferase assays to determine the prevalence and level of infection of mosquitoes treated with test compounds compared with those treated with controls.

For this, mosquitoes were frozen at -20°C for 24h, then thawed and transferred individually to wells of 96-well plates each containing 60 µl PBS with a complete protease inhibitor cocktail according to the manufacturer's instructions (Roche, Switzerland), and 0.2 grams of 1.0 mm diameter zirconia beads (Lab Services BV). Plates were then placed in a Mini-Beadbeater-96 (Biospec, Bartlesville, OK) and shaken for 2 x15 seconds, with 1-2 minutes on ice between homogenization cycles. At the end of complete homogenization, 45 µl of each well was transferred to wells of a black-and-white 96-well Krystal 2000 plate (Porvair, UK). Next, 45 µl of Bright-Glo luciferase assay substrate (Promega, Madison, WI) was added to each well and the plate was incubated for 3 minutes at room temperature. Luciferase activity was measured using a versatile Synergy 2 plate reader (Biotek, Winooski, VT). The fluorescence background threshold was determined by measuring the signal from uninfected mosquitoes that had undergone the same treatment.

In the case of drug combinations (Fig. 9), MD40 was used at 316 nM and the partner drugs at concentrations indicated in Table 5. The ratio "infection intensity of the combination / infection intensity of MD40 alone" was calculated to identify partner drugs with synergistic effects (ratio <1).

***In vivo* antimalarial activity of the compounds using the *P. berghei* mouse model (STPH).** The Swiss Tropical Institute (Basel, Switzerland) adheres to local and national regulations of laboratory animal welfare in Switzerland. Selected compounds were tested in the murine *P*. *berghei* model essentially as described (Vennerstrom, J. L. et al. Identification of an antimalarial synthetic trioxolane drug development candidate. Nature 2004, 430, 900-904; Ridley RG, et al. Antimalarial activity of the bisquinoline trans-N1,N2-bis-(7-chloroquinolin-4-yl)cyclohexane-1,2-diamine: Comparison of two stereoisomers and detailed evaluation of the S,S enantiomer, Ro 47-7737. Antimicrob. Agents Chemother. 1997, 41, 677-686; Peters, W. Chemotherapy and Drug Resistance in Malaria Vol. 1 (Academic Press, 1987).) The infection was initiated at day 0 with the *P*. *berghei* GFP ANKA malaria strain (donation from AP Waters and CJ Janse, Leiden University). From donor mice with approximately 30% parasitaemia, heparinized blood was taken and diluted in physiological saline to 10⁸ parasitized erythrocytes/ml. An aliquot (0.2 ml) of this suspension was injected intravenously into experimental and control groups of mice. Usually, in untreated control mice parasitaemia rose regularly to ~30% by day 3 post-infection. In these conditions, control mice die between days 6 and 7 post-infection. In the experiments described here, however, control animals (n = 5) were sacrificed on day 4 post-infection for ethical reasons.

The compounds were prepared at an appropriate concentration as a solution/suspension in Tween 80 / alcohol (7:3, Tween 80 and absolute ethanol, respectively), followed by a 10x dilution in water. They were administered to groups of three mice in four doses (4 h, 24 h, 48 h, 72 h post-infection). The route of administration was *per os* (p.o., 50 mg/kg).

The degree of infection (parasitaemia expressed in % of infected erythrocytes) was determined by FACS analysis on day 4 (96 h post-infection). The difference of the mean infection rate of the control group (= 100%) to the test group was calculated and expressed as a percent reduction. For example, activity determination with a mean of 2% parasitaemia in treated mice and a mean of 40% parasitaemia in the control animals s calculated as follows: (40% - 2%) / 40% * 100 = 95% activity. The survival time in days was recorded up to 30 days after infection. A compound was considered curative if the animal survived to day 30 after infection with no detectable parasites (confirmed by light microscopy).

***In vivo* antimalarial properties and transmission blocking activity using the *P*. *berghei* mouse model (IBMC).** The animal experiments described in this part were performed at IBMC using certified facilities and protocols adhering to national regulations of laboratory animal welfare in France. 8-12 week-old male Hsd:ICR mice (bred in-house) were infected with the *P*. *berghei* ANKA malaria strain (Franke-Fayard, B. et al. A Plasmodium berghei reference line that constitutively expresses GFP at a high level throughout the complete life cycle. Mol. Biochem. Parasitol. 137, 23-33 (2004).) constitutively expressing *GFP*. For this, blood was taken by heart puncture from a donor mouse with a parasitaemia of 3-5%, diluted in PBS to 10⁸ parasitized erythrocytes/ml. 0.2 ml of this suspension was injected intravenously into mice. Parasitaemia was monitored daily via FACS analysis (FACSCalibur^{™}, BD Bioscience). *Anopheles gambiae* mosquitoes (G3 strain, donation from the Kafatos laboratory at EMBL in 2002) were bred following standard procedures. Briefly, larvae are fed with grinded fish food (TetraMind Gold) and adult mosquitoes with a 10% sucrose solution. Females are offered a blood meal on anesthetized mice for egg production. For this, mice were anesthetized via an i.p. injection of 5% Rompun^{®} (Xylazine) and 10% Imalgene^{®} (Ketamine) diluted in saline solution (100 µl per 10 g) and exposed to mosquito bites for 15 min. All mosquito stages were maintained at 26°C and 70% humidity with a 12h/12h day/night cycle.

**MD40** and plasmodione were prepared in solution/suspension in Tween 80 / absolute alcohol (7:3), followed by a 10x dilution in water to reach the final concentration of 10 mg/ml. ProveBlue (Inresa Pharma), a heavy metal-free solution of methylene blue (5 mg/ml) was used as a positive control. Solutions were injected intraperitoneally (i.p.) in infected mice as daily doses (24 h, 48 h, and 72 h post passage) (30 mg/kg for **MD40** and plasmodione, and 15 mg/kg for MB). Negative control mice were i.p. injected with an equivalent quantity of solvent. Parasitaemia was determined daily via FACS analysis (FACSCalibur^{™}, BD Bioscience). In parallel, groups of 40 *Anopheles gambiae* mosquitoes (4-7 days old) were allowed to take a blood meal for 15 min on treated and control infected mice at 48 h, 72 h, 96 h, and 120 h post-infection (one group of 40 mosquitoes per mouse per day). Fully engorged mosquitoes were sorted, and their midgut was dissected in 1x PBS at 7 days post-infection. Each midgut was imaged using a Nikon fluorescence microscope SMZ18, and fluorescent parasites (oocyst stage) were counted using the Image J watershed program. The prevalence (percentage of infected mosquitoes) and infection level (average number of parasites per midgut in mosquitoes carrying at least one parasite) were plotted using Prism. Three independent experiments were performed, and significance for differences between treatments was calculated using generalized linear models with treatment, time and repeats as variables using the JMP^{®} software (SAS). Data were modeled with normal distributions and an identity link for parasitemia, a logit link for prevalence, and a log link for the level of infection.

***In vivo* antimalarial activity of the compounds using the humanized *P. falciparum* mouse model (STPH).** The Swiss Tropical Institute (Basel, Switzerland) finally confirmed the therapeutic efficacy of **MD40** (batch LF151) in combination with artesunate in a SCID mouse model of human *P*. *falciparum* malaria. The goal of this study was to evaluate the therapeutic efficacy of **MD40** (batch LF151) alone as well as in combination with artesunate against *P*. *falciparum* Pf3D7^{0081/N9} in NODscidIL2Rγ^{null} mice engrafted with human erythrocytes. Mice are infected intravenously with parasitized red blood cells on day 0. Experimental mice are treated at day 3, 4, 5, and 6 post-infection with an oral dose of the compound and are compared to an infected control group for reduction in parasitemia on day 7.

**MD40** was solubilised in a vehicle consisting of 70% Tween-80 and 30% ethanol, followed by a 10-fold dilution in H2O. The preparations resulted in homogenous, milky suspensions. Artesunate was solubilised in a vehicle consisting of 70% Tween-80 and 30% ethanol, followed by a 10-fold dilution in H2O. The preparations resulted in clear solutions. **MD40** in combination with artesunate was solubilised in a vehicle consisting of 70% Tween-80 and 30% ethanol, followed by a 10-fold dilution in H₂O. The final preparations resulted in homogenous, milky suspensions.

The experimental plan was to assess the antimalarial efficacy in three different ways:
1. Following administration of one oral (p.o.) dose (50 mg/kg) of **MD40** per day on days 3, 4, 5 and 6 post-infection (= 4x 50 mg/kg in total).
2. Following administration of one oral (p.o.) dose (10 mg/kg) of artesunate per day on days 3, 4, 5 and 6 post-infection (= 4x 10 mg/kg in total).
3. Following administration of one oral (p.o.) dose of a mix of **MD40** (50 mg/kg) in combination with artesunate (10 mg/kg) per day on days 3, 4, 5 and 6 post-infection (= 4x 50 mg/kg + 4x 10 mg/kg in total).

The effect on blood parasitemia was measured by flow cytometry (FACS) and by microscopic analysis of Giemsa-stained blood smears (on days 3, 4, 5, 6 and 7 post-infection; and then every Monday, Wednesday and Friday until ≥5-10% parasitemia until day 14, when the mice were euthanized as per the protocol. Control mice were euthanized on day 7. The efficacy estimated in this study was expressed as the reduction (in %) of parasitemia at day 7 after infection (n=2 mice per dose) as compared to the untreated control group (n=4).

### Protocol:

Parasite strain: *Plasmodium falciparum* strain Pf3D7^{0081/N9} (Jiménez-Díaz MB, et al.,
Antimicrob Agents Chemother. 2009, 53(10):4533-4536. doi: 10.1128/AAC.00519-09) Route of infection: Intravenous
Infective dose: 2.5×10⁷ Pf-infected erythrocytes at day 0
Standard vehicle: Compounds are solubilised (suspended) in a solution consisting of 70% Tween-80 (d= 1.08g/ml) and 30% ethanol (d=0.81g/ml), followed by a 10-fold dilution in H₂O.
Volume of administration: 10 ml/kg
Mice: NODscidIL2Rγ^{null} mice, females, 20 - 22 g
Maintenance: Mice are kept in individually ventilated cages (IVC), but otherwise under standard conditions with 22°C and 60 - 70 % relative humidity, pellets (PAB45 - NAFAG 9009, Provimi Kliba AG, CH-4303, Kaiseraugst, Switzerland) and water ad libitum.

### Experimental procedure. Efficacy study:

Day -11 to 6: Mice are engrafted daily with 0.6 mL human blood i.v. in the tail (alternatively 0.8 mL i.p.).
Day -11 to 0: A cryopreserved parasite stock is thawed and parasites in vitro expanded.
Day 0: In vitro cultures with approximately 2-5% parasitemia are diluted in culture medium to 2.5x10⁷ parasitized erythrocytes per 0.1ml. This suspension is injected intravenously (i.v.) into experimental groups and a control group of n=4 mice.
Day 3 - 6: 3-, 4-, 5- and 6-days post-infection, the experimental groups (n=2) are treated with a single daily dose by the oral (p.o.) route. The drug concentration is adjusted in a way that 10 ml/kg has to be injected.
Day 3 - 7: 3, 4-, 5-, 6- and 7-days post-infection, 2 µl tail blood is taken, hematocrit determined by FACS and parasitemia by microscopy on >10'000 red blood cells.

The difference of the mean infection rate of the control group (= 100%) to the test group is calculated and expressed as percent reduction. The results are expressed as reduction of parasitemia at day 7 in % as compared to the untreated control group.

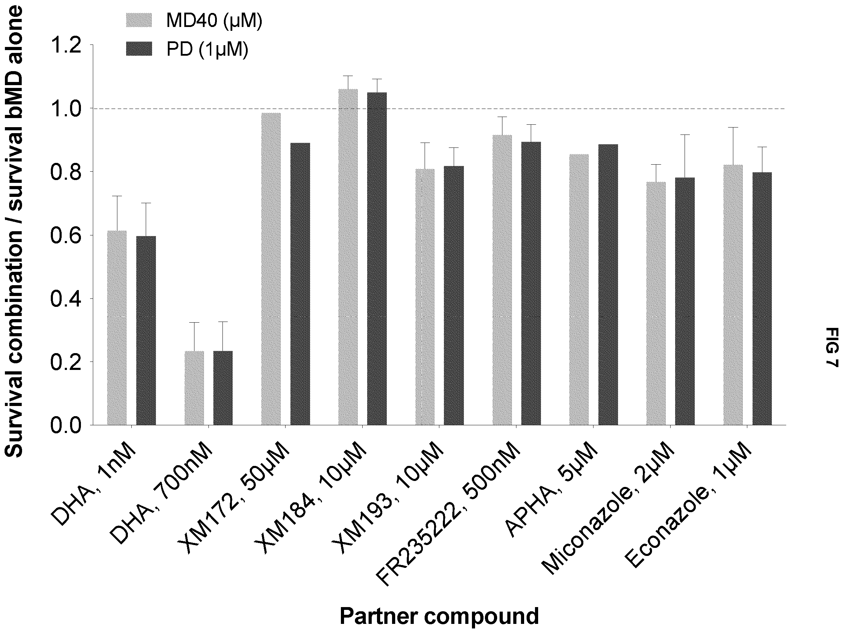

**Ring stage survival assays and drug combination.** Artemisinin resistance is best characterized using a test called Ring stage Survival Assay (RSA) developed at the Institut Pasteur du Cambodge (RSAv1) (B Witkowski, et al. Ring-stage Survival Assays (RSA) to evaluate the in-vitro and ex-vivo susceptibility of Plasmodium falciparum to artemisinins. Institute Pasteur du Cambodge - National Institutes of Health Procedure RSAv1). The resistance of several *P*. *falciparum* strains with variable sensitivity to artemisinin and derivatives, (ranging from fully sensitive (3D7, IPC3663 and IPC5188) to the most resistant (IPC5188)) to PD was evaluated using RSA (see Fig. 5A). All other experiments (Fig. 5B and Fig.7) were performed on ArtS (IPC5188) and ArtR (IPC5202), that are respectively artemisinin-sensitive and resistant. Parasites were treated with drugs for 6h, starting on 3h old ring stage of parasite. For this, schizonts of highly synchronous parasite cultures were separated using 60% percoll gradient, and the mature segmented schizonts were incubated with non-infected red blood cells for 3 h to allow reinvasion. The ring stage parasites produced during these 3 h were then synchronized with 5% sorbitol to kill mature stages. The culture used for the assay was adjusted to 0.5% parasitaemia and 2% haematocrit, and the assay performed in 48-well plates (500 µl/well). Parasites were treated with defined drug concentrations, and compared with a positive control treated with 700 nM DHA and a negative growth control without treatment. Drugs were removed after 6h incubation. For this, the contents of the wells were centrifuged and resuspended in 12 ml of incomplete RPMI (to remove the test compound) and the tubes were centrifuged again. The pellets were resuspended in 500 µl of complete medium each, transferred to a new plate and incubated for an additional 66 hours to allow surviving parasites to develop.

Parasitemia of each well was then assessed by counting the percentage of live parasites in each well, and the survival of parasites upon treatment was determined as follows: Survival upon drug treatment = parasitemia in drug-treated well / parasitemia in non-treated control * 100.

Parasitemia was assessed either by microscopy using Giemsa-stained blood smears (Fig. 5A) as described in procedure RSAv1 (Institut Pasteur du Cambodge, National Institutes of Health), or by flow cytometry following a protocol adapted from Amaratunga *et al.,* 2014¹³ (Fig. 5B and Fig. 7). Briefly, for flow cytometry readout, samples underwent a series of washes and were incubated with SYBR green (SG, 0.8x) and/or MitoTracker Red FM (MT, 0.7µM) following the conditions described by Amaratunga *et al.,* 2014¹³ with some slight modifications. The content of each well was transferred to a 1.5-ml tube and washed by centrifugation with 500 µl of flow cytometry wash solution 3 times. After the third wash, blood pellets were collected and treated with 25µl of fluorophore solution and incubated with open lid in an incubator at 37°C for 45 min. After incubation, 500 µl of flow cytometry wash solution was added to each tube which contents were centrifuged at 2000 rpm for 30 seconds. This wash step was repeated 3 times. At the end of the wash, the pellet is reconstituted in 500 µl of wash solution and 150 µl of this sample is transferred to a new tube containing 500 µl of wash solution. These tubes were then briefly vortexed and immediately read on a flow cytometer (BD AccuryTM C6 Flow cytometer). The use of these two fluorophores makes it possible to detect live parasites (SG⁺, MT⁺) from dead parasites (SG⁺, MT).

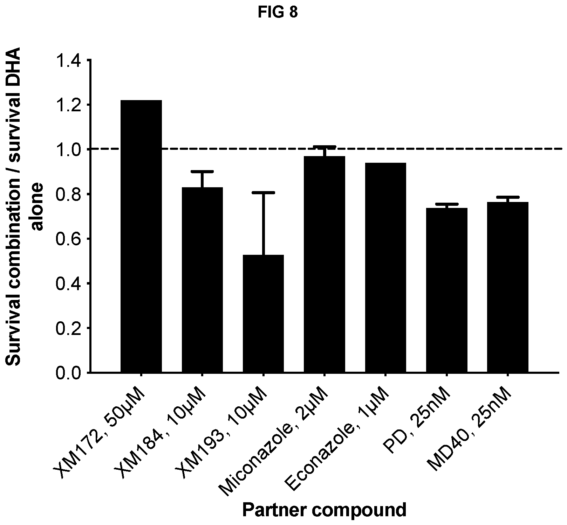

For RSA drug combination tests, a single concentration per drug was selected as follows. 0-3h ring parasites (ArtS = IPC5188 and/or ArtR = IPC5202) were exposed to four concentrations of each drug for 6h, the drug washed and parasites incubated for 66h additional hours. Parasite growth was then measured using the SYBR green assay and data were analyzed with GraphPad Prism as described above. For the combinations, we selected the lowest concentration killing all ArtS parasites (IC₁₀₀) for the main partners, i.e. **MD40** (2 µM) and plasmodione (1 µM) (Fig. 7), or the physiological concentration for DHA (700 nM) (Fig. 8), and the highest concentration of the minor partner drugs that did not affect ArtR growth (IC₀, Fig. 7 and 8, Table 5). Should no effect of the minor partner drug be detected at the highest concentration used, the minor drug was used at 50 µM or at the highest concentration possible for the drug. Survival of ArtR parasites exposed to combinations of **MD40** (2 µM), plasmodione (1 µM) or DHA (700 nM) with partner drugs at the concentrations indicated in **Table 5** was compared to survival of parasites exposed to **MD40** (2 µM), plasmodione (1 µM) or DHA (700 nM) alone, respectively.

**Table 5. Concentrations of minor partner drugs used in Fig. 7 and Fig. 8 (column IC₀ (nM), ArtR 6h), and in Fig. 9 (column "Transmission blocking comb. (nM)"):**

| **Compound class** | **Compounds** | **IC₀ (nM) ArtR 6h** | **Transmission blocking comb. (nM)** |
|---|---|---|---|
| | DHA | 1 | 1 |
| bMDs | PD | 25 | |
| | MD40 | 25 | 316 |
| Iron chelators | XM172 | 50 000 | 10 000 |
| | XM184 | 10 000 | |
| | XM193 | 10 000 | |
| Epigenetic modifiers | FR235222 | 500 | |
| | APHA | 5 000 | |
| Fungicides | Miconazole | 2000 | 1 000 |
| | Econazole | 1 000 | |

### Part II. Syntheses and Characterization

### General procedure (A) for the synthesis of 3-(oxetan-3-yl-oxy)-functionalized plasmodione regioisomers 6 with general formula (I) from diversely 6-substituted menadiones

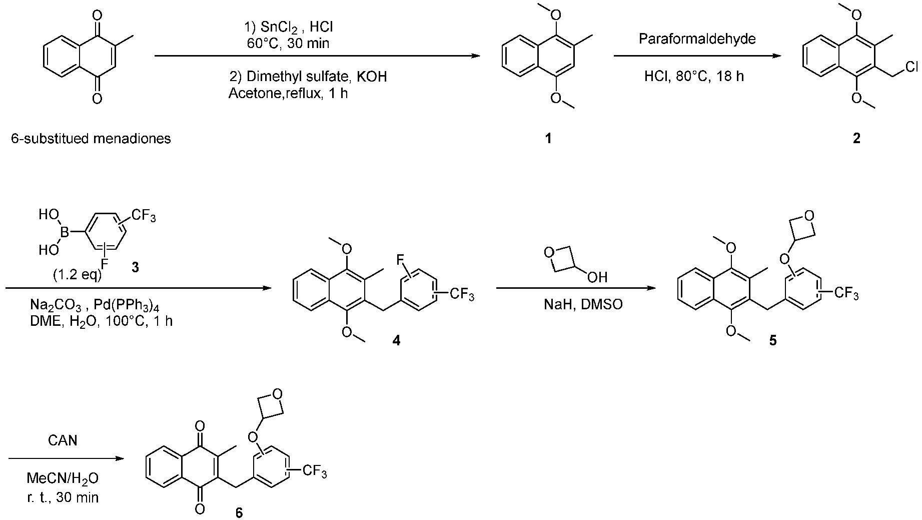

If the boronics acids are not easily accessibles, particularly concerning the heteroarene compounds, the compounds are synthesized according to the next following general procedure (B).

### General procedure (B) for the synthesis of heteroaromatic 3-(oxetan-3-yl-oxy)-functionalized plasmodione analogues 6 from diversely 6- or 7-substituted 2-(chloromethyl)-1,4-dimethoxy-3-methylnaphthalene:

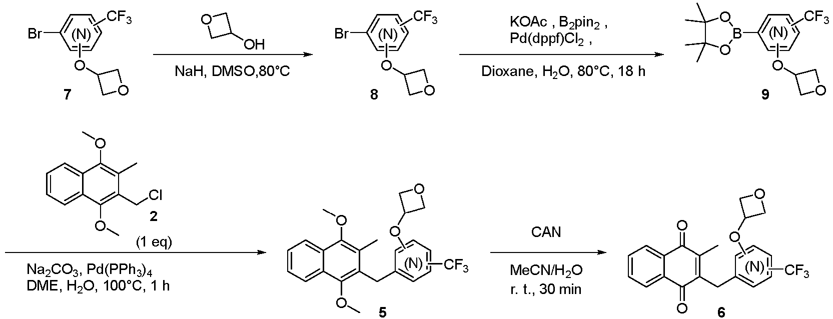

The compounds with amino- and (oxetan-3-yl)-amino-functionnalized plasmodione are prepared according to the next following general procedure (C).

### General procedure (C) for the coupling reaction amino- and (oxetan-3-yl)-amino-functionnalized plasmodione analogues 14 with general formula (I) from diversely 6-or 7-substituted 2-(chloromethyl)-1,4-dimethoxy-3-methylnaphthalene:

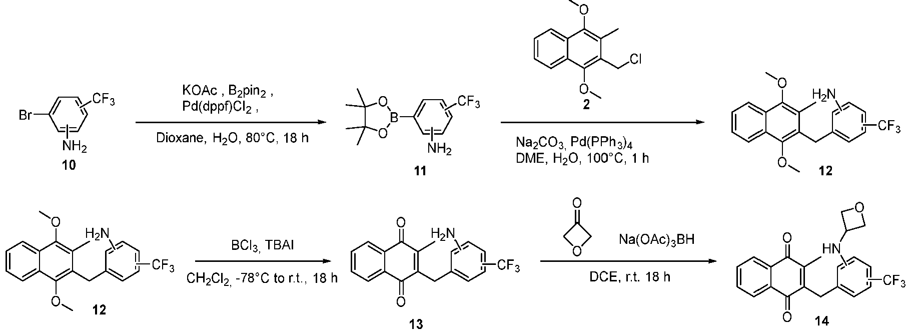

Another method to obtain compounds of formula (IV) was to reduce the corresponding 3-benzylmenadione by Tin(II) chloride and HCl, then methylate the formed dihydro-napthoquinol by dimethylsulfate in the presence of NaOH (or KOH).

### PREPARATION OF COMPOUNDS OF FORMULA (IV) by reduction of menadione, using Tin(II) chloride and HCl, then dimethylsulfate in the presence of NaOH (or KOH). The compounds of formula (IV) are prepared according to the following reaction scheme:

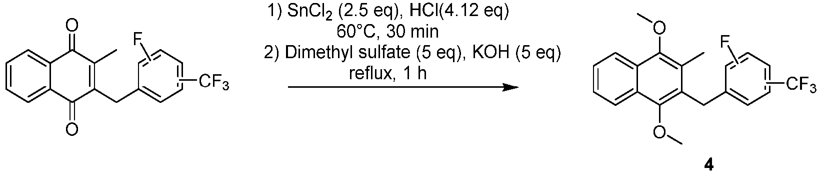

| **3-Benzylmenadiones** | **Compounds of formula (IV)** | **Yield (%)** | **Reference** |
|---|---|---|---|
| 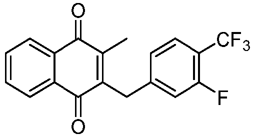 | 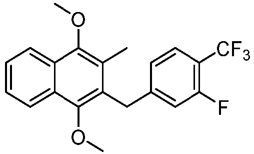 | **95** | **LF137** |
| 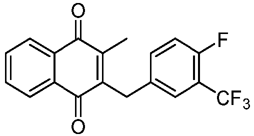 | 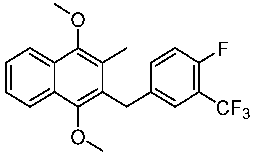 | **38** | **MR265** |

### Preparation of 2-(3-fluoro-4-(trifluoromethyl)benzyl)-1,4-dimethoxy-3-methylnaphthalene (LF137):

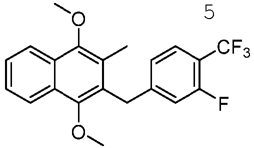

According to this procedure (95% yield, colorless oil) was isolated by purification by flash chromatography on silica gel (Cyclohexane/Toluene, 7/3). ¹H NMR (400 MHz, CDCl₃) δ 8.14-8.05 (m, 2H), 7.56-7.49 (m, 2H), 7.46 (t, 1H, *J =* 7.8 Hz), 7.01 (d, 1H, *J* = 8.2 Hz), 6.94 (d, 1H, *J* = 11.5 Hz), 4.30 (s, 2H), 3.87 (s, 3H), 3.85 (s, 3H), 2.25 (s, 3H). ¹³C NMR (101 MHz, CDCl₃) δ 159.9 (d, *¹_{C-F}* = 256.3 Hz), 151.0 (d, *³J_{C-F}* = 6.5 Hz), 148.4, 148.3, 128.7, 127.8, 127.5, 127.3 (q, *⁴J_{C-F} =* 3.1 Hz), 126.7, 126.5, 126.0, 124.0 (d, *⁴J_{C-F} =* 2.6 Hz), 123.1 (q, *¹J_{C-F}* = 272.4 Hz), 122.9, 122.7, 116.7 (d, *²J_{C-F}* = 20.6 Hz), 116.3 (dd, *²J_{C-F}* = 32.9, 13.0 Hz), 62.6, 61.8, 32.9, 13.0.

¹⁹F NMR (377 MHz, CDCl₃) δ -61.08, -114.9.

HRMS (ESI+) *m*/*z:* [M]⁺ calculated for C₂₁H₁₈F₄O₂: 378.1257, found 378.1237.

### Preparation of 2-(4-fluoro-3-(trifluoromethyl)benzyl)-1,4-dimethoxy-3-methylnaphthalene (MR265):

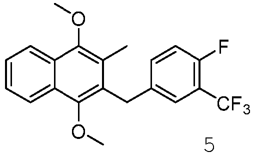

According to this procedure (38% yield, colorless oil) was isolated by purification by flash chromatography on silica gel (Toluene/cyclohexane, gradient from 4/6 to 5/5). ¹H NMR (400 MHz, CDCl₃): *δ* 8.16-8.08 (m, 2H), 7.58-7.50 (m, 2H), 7.45 (dd, *J* = 6.6, 2.3 Hz, 1H), 7.24 (dd, *J* = 5.4, 2.9 Hz, 1H), 7.05 (t, *J =* 9.3 Hz, 1H), 4.28 (s, 2H), 3.88 (d, *J =* 6.3 Hz, 6H), 2.28 (s, 3H). ¹³C NMR (101 MHz, CDCl₃): *δ* 158.3 (qd, *J* = 254.1, 2.0 Hz), 150.8 (2C), 136.8 (d, *⁴J_{C-F} =* 3.8 Hz), 133.4 (d, *³J_{C-F}* = 7.9 Hz), 128.4, 128.1, 127.3, 126.7 (qd, *J* = 4.5, 1.4 Hz), 126.6, 126.2, 125.8, 122.8 (d, *¹J_{C-F}*= 271.5 Hz), 122.7, 122.5, 118.2 (qd, *J* = 32.6, 12.5 Hz), 116.9 (d, *²J_{C-F} =* 20.5 Hz), 62.4, 61.6, 32.0, 12.8.

¹⁹F NMR (377 MHz, CDCl₃): *δ* -61.24, -118.92.

HRMS (APCI+) *m*/*z*: [M]⁺ calculated for C₂₁H₁₈F₄O₂: 378.1237, found 378.1219.

### PREPARATION OF COMPOUNDS OF FORMULA (IV) by Suzuki reaction between 2-methyl-3-chloromethyl-1,4,-dimethoxynaphthalene and boronic acids of formula (III)). The compounds of formula (IV) are prepared according to the following reaction scheme:

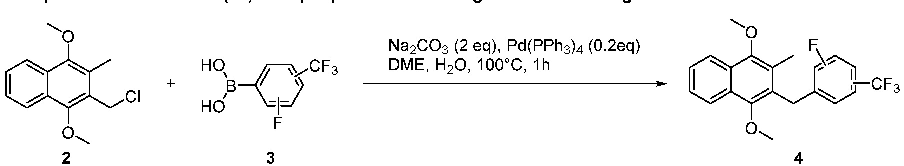

### Synthesis of 2-(chloromethyl)-1,4-dimethoxy-3-methylnaphthalene:

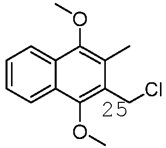

1,4-dimethoxy-2-methylnaphthalene (1 equiv., 5.18 g, 25.61 mmol) , paraformaldehyde (5 equiv., 4.047 g, 3.74 mL, 128.057 mmol) , and HCl (65 mL) were stirred at 80 °C for 18 hours. The mixture was cooled down, diluted with water, and extracted with EtOAc. The organic phase was dried over MgSO₄ and concentrated. The crude product was purified by flash chromatography on silica gel (Toluene/Cyclohexane, 9/1) to afford 2-(chloromethyl)-1,4-dimethoxy-3-methylnaphthalene (5.46 g, 21.78 mmol, 85%).

¹H NMR (400 MHz, CDCl₃) δ 2.54 (s, 3H), 3.89 (s, 3H), 4.05 (s, 3H), 4.93 (s, 2H), 7.45 - 7.58 (m, 2H), 8.09 (dd, *J* = 7.7, 1.8 Hz, 2H)

### General procedure for the Suzuki coupling between 2-(chloromethyl)-1,4-dimethoxy-3-methylnaphthalene and arylboronic acid:

In a flask, 2-(chloromethyl)-1,4-dimethoxy-3-methylnaphthalene (1 equiv), the corresponding boronic acid or boronic acid pinacol ester (1.4 equiv) and sodium carbonate (3 equiv.) were dissolved in a 2:1 mixture of dimethoxyethane:water (0.15M). The mixture was bubbled 15min with argon, and then tetrakis(triphenylphosphine)palladium (5 mol%) was added at once. The mixture was heated 1h at 100°C under stirring. The mixture was then, allowed to cool down to room temperature, diluted with water and extracted three times with dichloromethane. Reunited organic layers were washed with brine, dried over magnesium sulfate and the solvent was removed under reduced pressure to afford a crude which was purified on silica gel chromatography using the adequate eluant system to afford the corresponding coupling product.

| **Boronic acid of formula (III)** | **Compounds of formula (IV)** | **Yield (%)** | **Reference** |
|---|---|---|---|
| 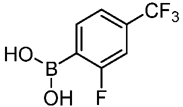 | 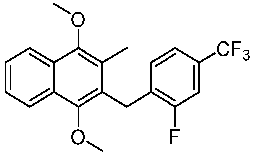 | **73** | **JR35** |
| 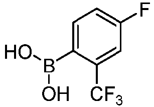 | 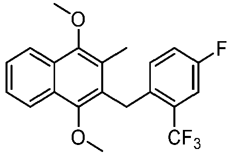 | **92** | **JR36** |
| 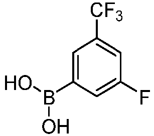 | 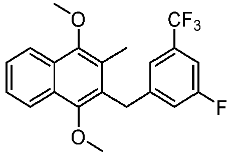 | **82** | **JR37** |
| 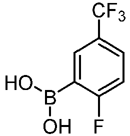 | 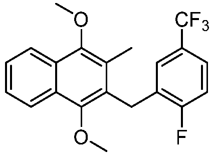 | **93** | **JR120** |
| 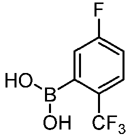 | 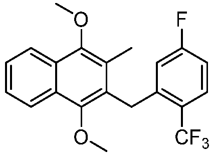 | **90** | **MR450** |

### Preparation of 2-(3-fluoro-5-(trifluoromethyl)benzyl)-1,4-dimethoxy-3-methylnaphthalene (JR35):

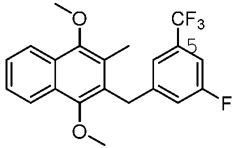

According to this procedure (82% yield, colorless oil) was isolated by purification by flash chromatography on silica gel (Cyclohexane/Toluene, 7/3). ¹H NMR (400 MHz, CDCl₃) δ 8.16 - 8.03 (m, 2H), 7.58 - 7.48 (m, 2H), 7.13 (d, *J* = 8.3 Hz, 1H), 6.94 (d, *J =* 9.4 Hz, 1H), 4.31 (s, 2H), 3.86 (d, *J =* 8.8 Hz, 6H), 2.25 (s, 3H).

¹³C NMR (101 MHz, CDCl₃) δ 163.8, 161.8, 150.8 (d, ³*J*_{C-F} = 10.8 Hz), 144.8 (d, ³*J*_{C-F} = 7.2 Hz), 132.4 (qd, *J* = 33.0, 8.2 Hz), 128.4, 127.4, 127.3, 126.5, 126.2, 125.8, 124.5 (d, *J =* 3.0 Hz), 122.6, 122.4, 120.8 (p, ⁴*J*_{C-F} = 3.6 Hz), 118.6, 118.4, 110.5 (qd, *J* = 24.7, 3.9 Hz), 62.3, 61.5, 32.6, 12.7.

¹⁹F NMR (377 MHz, CDCl₃) δ -62.67, -111.10.

HRMS (ESI+) *m*/*z*: [M+H]⁺ calculated for C₂₁H₁₉F₄O₂: 379.1316, found 379.1332.

### Preparation of 2-(4-fluoro-2-(trifluoromethyl)benzyl)-1,4-dimethoxy-3-methylnaphthalene (JR36):

According to this procedure (92% yield, colorless oil) was isolated by purification by flash 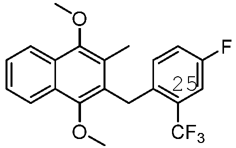 chromatography on silica gel (Cyclohexane/Toluene, 7/3). ¹H NMR (400 MHz, CDCl₃) δ 8.17 - 8.02 (m, 2H), 7.58 - 7.47 (m, 2H), 7.41 (dd, *J* = 2.8, 9.0 Hz, 1H), 6.96 (td, *J* = 2.8, 8.2 Hz, 1H), 6.75 - 6.67 (m, 1H), 4.38 (s, 2H), 3.88 (s, 3H), 3.83 (s, 3H), 2.18 (s, 3H).

¹³C NMR (101 MHz, CDCl₃) δ 161.6, 159.6, 151.1, 150.7, 134.9 (d, 4*J*_{C-F} = 3.5 Hz), 130.7 (d, ³*J*_{C-F} = 7.6 Hz), 129.9 (qd, ²*J*_{C-F} = 31.0, 7.4 Hz), 128.4, 127.4, 127.3, 126.8, 126.2, 125.7, 124.0 (qd, ¹*J*_{C-F} = 274.2, 2.7 Hz), 122.5, 122.4, 118.7, 118.6, 113.6 (qd, ³*J*_{C-F} = 25.1, 6.1 Hz), 62.2, 61.4, 40.8, 28.4 (q, 4*J*_{C-F} = 2.6 Hz), 12.4.

¹⁹F NMR (377 MHz, CDCl₃) δ 61.50, -115.57.

HRMS (ESI+) m/z: [M+H]⁺ calculated for C₂₁H₁₉F₄O₂: 379.1316, found 379.1312.

### Preparation of 2-(2-fluoro-4-(trifluoromethyl)benzyl)-1,4-dimethoxy-3-methylnaphthalene (JR37):

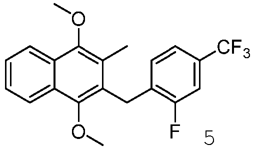

According to this procedure (73% yield, colorless oil) was isolated by purification by flash chromatography on silica gel (Cyclohexane/Toluene, 2/1). ¹H NMR (400 MHz, CDCl₃) δ 8.19 - 8.04 (m, 2H), 7.72 - 7.43 (m, 2H), 7.35 (dd, *J* = 9.7, 2.2 Hz, 2H), 6.87 (t, *J* = 7.8 Hz, 1H), 4.30 (s, 2H), 3.87 (dd, *J =* 10.6, 1.7 Hz, 6H), 2.25 (d, *J =* 2.1 Hz, 3H).

¹³C NMR (101 MHz, CDCl₃) δ 161.7, 159.2, 151.0, 150.6, 137.9, 136.1, 131.8 (d, ²*J*_{C-F} = 15.7 Hz), 130.2 (d, ³*J*_{C-F} = 8.0 Hz), 130.0 (d, ³*J*_{C-F} = 4.7 Hz), 129.1, 128.3, 128.2, 127.2, 126.7, 126.6, 126.2, 125.7, 125.3, 122.5, 122.4, 121.0 (q, ³*J*_{C-F} = 3.9 Hz), 112.4 (qd, *J* = 25.5, 3.8 Hz), 62.3, 61.5, 25.4 (d, *³J_{C-F} =* 4.0 Hz), 12.4.

¹⁹F NMR (377 MHz, CDCl₃) δ -62.51, -115.31.

HRMS (ESI+) *m*/*z*: [M+H]⁺ calculated for C₂₁H₁₉F₄O₂: 379.1316, found 379.1349.

### Preparation of 2-(2-fluoro-5-(trifluoromethyl)benzyl)-1,4-dimethoxy-3-methylnaphthalene (JR120):

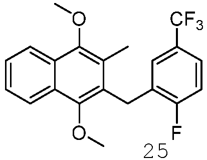

According to this procedure (93% yield, colorless oil) was isolated by purification by flash chromatography on silica gel (Cyclohexane/Toluene, 7/3). ¹H NMR (400 MHz, CDCl₃) δ 8.18 - 8.06 (m, 2H), 7.60 - 7.51 (m, 2H), 7.49 - 7.41 (m, 1H), 7.23 - 7.14 (m, 1H), 7.09 - 7.02 (m, 1H), 4.30 (s, 2H), 3.87 (d, *J* = 13.3 Hz, 6H), 2.25 (s, 3H).

¹³C NMR (101 MHz, CDCl₃) δ 163.9, 161.4, 151.1, 150.7, 129.1, 128.8, 128.6, 128.4, 128.2, 127.2, 127.0 (d, *J =* 3.6 Hz), 126.7 (dt, *J* = 7.5, 3.7 Hz), 126.5, 126.2, 125.7, 125.1 (qd, *J* = 7.8, 3.8 Hz), 122.6, 122.4, 115.6, 115.4, 62.3, 61.4, 25.3 (d, *³J_{C-F}* = 4.0 Hz), 12.5. ¹⁹F NMR (377 MHz, CDCl₃) δ -61.90, -111.91.

HRMS (ESI+) *m*/*z*: [M+H]⁺ calculated for C₂₁H₁₉F₄O₂: 379.1316, found 379.1321.

### Preparation of 2-(5-fluoro-2-(trifluoromethyl)benzyl)-1,4-dimethoxy-3-methylnaphthalene (MR450):

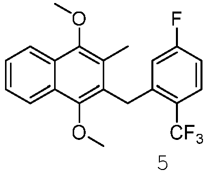

According to this procedure (90% yield, colorless oil) was isolated by purification by flash chromatography on silica gel (Cyclohexane/Toluene, 8/2).

¹H NMR (400 MHz, CDCl₃) δ 8.12 (dd, *J* = 15.9, 7.4 Hz, 2H), 7.78-7.63 (m, 1H), 7.54 (s, 2H), 6.94 (t, *J* = 6.9 Hz, 1H), 6.45 (d, *J* = 9.6 Hz, 1H), 4.43 (s, 2H), 3.89 (s, 3H), 3.84 (s, 3H), 2.20 (s, 3H).

¹³C NMR (101 MHz, CDCl₃) *δ* 165.0 (d, *¹J_{C-F}* = 252.2 Hz), 151.3, 150.9, 143.0 (d, *³J_{C-F} =* 7.2 Hz), 128.5, 128.4 (dd, *J* = 9.3, 6.0 Hz), 127.3, 127.0, 126.7, 126.3, 125.9, 124.7 (qd, *J* = 30.8, 3.2 Hz), 124.6 (q, *¹J_{C-F}* = 273.4 Hz), 122.7, 122.5, 116.2 (d, *²J_{C-F}* = 23.5 Hz), 113.0 (d, *²J_{C-F} =* 22.1 Hz), 62.4, 61.6, 29.2 (d, *⁴J_{C-F} =* 1.6 Hz), 12.6.

¹⁹F NMR (377 MHz, CDCl₃) δ -60.43, -107.38.

HRMS (ESI+) *m*/*z*: [M]⁺ calculated for C₂₁H₁₉F₄O₂: 379.1316, found 379.1316.

### PREPARATION OF COMPOUNDS OF FORMULA (II) (bv nucleophilic aromatic substitution of compounds (II) by oxetan-3-ol. The compounds of formula (IV) are prepared according to the following reaction scheme:

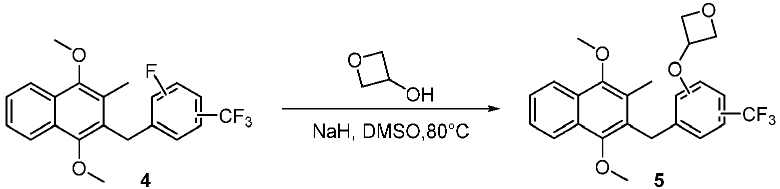

### General procedure for the nucleophilic aromatic substitution of fluoroarenes by oxetan-3-ol:

To a solution of oxetan-3-ol (3 equiv) in anhydrous DMSO (0.13M), NaH (3 equiv.) was added portion-wise under argon atmosphere. The mixture was stirred until homogenous. The resulting mixture was added dropwise to a solution of fluoroarene (1 equiv.) in anhydrous DMSO (0.13M), then the mixture was stirred for 18 h at 20°C for MD40, and at 80 °C for all other MD40 regioisomers. The reaction was quenched with 1M HCl solution and diluted with CH₂Cl₂. The aqueous layer was extracted with CH₂Cl₂. The organic layer was washed with water, brine, dried over MgSO₄ and concentrated under reduced pressure. The crude residue was purified by flash chromatography on silica gel using the adequate eluent.

| **Compounds of formula (II)** | **Compounds of formula (IV)** | **Yield (%)** | **Reference** |
|---|---|---|---|
| 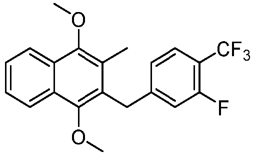 | 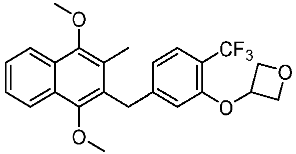 | **80** | **LF149** |
| 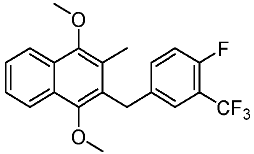 | 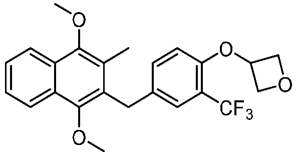 | **49** | **MR268** |
| 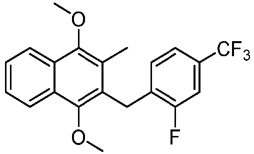 | 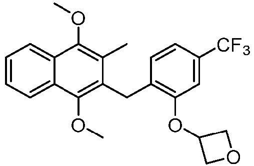 | **29** | **JR39** |
| 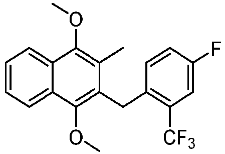 | 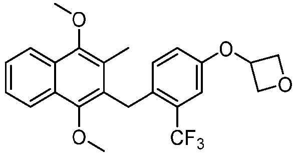 | **62** | **JR40** |
| 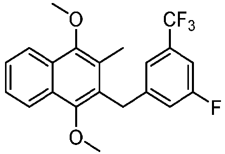 | 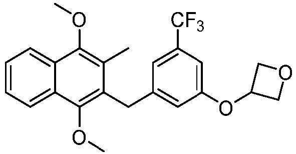 | **50** | **JR49** |
| 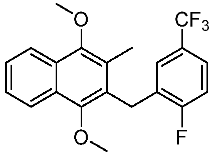 | 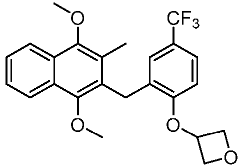 | **76** | **JR124** |
| 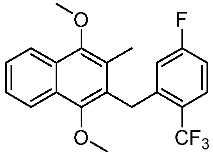 | 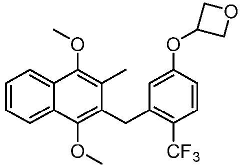 | **74** | **MR453** |

### Preparation of 3-(3-((1,4-dimethoxy-3-methylnaphthalen-2-yl)methyl)-5-(trifluoromethyl)phenoxy) oxetane (JR49):

According to this procedure (50% yield, colorless oil) was isolated by purification by flash 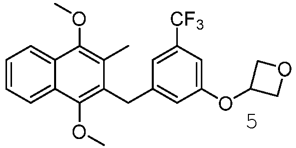 chromatography on silica gel (Cyclohexane/Ethyl acetate, 8/2).

¹H NMR (400 MHz, CDCl₃) δ 8.03 - 7.93 (m, 2H), 7.46 - 7.33 (m, 2H), 7.00 (s, 1H), 6.59 (s, 1H), 6.47 (s, 1H), 4.94 (p, *J =* 6 Hz, 1H), 4.69 (t, *J =* 8Hz, 2H), 4.51 (dd, *J* = 8,4 Hz, 2H), 4.14 (s, 2H), 3.72 (d, *J* = 7.2 Hz, 6H), 2.13 (s, 3H).

¹³C NMR (126 MHz, CDCl₃) δ 157.0, 150.7, 150.6, 143.9, 132.1 (q, *²J_{C-F}* = 32.2 Hz), 129.8 (d, *³J_{C-F} =* 10.4 Hz), 128.3, 127.8, 127.2, 126.6, 126.1, 125.7, 123.8 (q, *¹J_{C-F}* = 272.6 Hz), 122.6, 122.4, 118.2 (q, *³J_{C-F}* = 3.9 Hz), 117.7, 108.7 (d, *³J_{C-F}* = 3.9 Hz), 77.6, 70.3, 62.3, 61.5, 32.7, 12.7.

¹⁹F NMR (377 MHz, CDCl₃) δ -62.55.

HRMS (ESI+) m/z: [M]⁺ calculated for C₂₄H₂₃F₃O₄: 432.1543, found 432.1543.

### Preparation of 3-(4-((1,4-dimethoxy-3-methylnaphthalen-2-yl)methyl)-3-(trifluoromethyl)phenoxy) oxetane (JR40):

According to this procedure (62% yield, white solid) was isolated by purification by flash 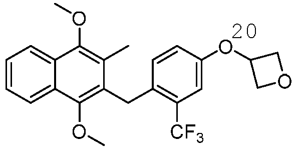 chromatography on silica gel (Cyclohexane/Ethyl acetate, 8/2).

¹H NMR (400 MHz, CDCl₃) δ 8.22 - 8.09 (m, 2H), 7.60 - 7.47 (m, 2H), 7.16 (d, *J =* 2.7 Hz, 1H), 6.71 (d, *J =* 8.6 Hz, 1H), 6.57 (dd, *J* = 8.7, 2.7 Hz, 1H), 5.15 (d, *J* = 5.9 Hz, 1H), 4.92 (d, *J =* 7.2 Hz, 2H), 4.74 (t, *J =* 5.4 Hz, 2H), 4.43 (s, 2H), 3.91 (s, 3H), 3.87 (s, 3H), 2.26 (s, 3H).

¹³C NMR (101 MHz, CDCl₃) δ 154.7, 151.1, 150.7, 131.7, 130.3, 129.7 (q, ²*J*_{C-F} 30.2 Hz), 128.3, 127.9, 127.3, 127.0, 126.1, 125.7, 124.4 (q, ¹*J*_{C-F} 274.6 Hz), 122.6, 122.3, 117.1, 113.0 (q, ³*J*_{C-F} = 6.1 Hz), 77.7 (2C), 70.4, 62.2, 61.4, 28.3, 12.4.

¹⁹F NMR (377 MHz, CDCl₃) δ -61.30.

HRMS (ESI+) *m*/*z*: [M]⁺ calculated for C₂₄H₂₃F₃O₄: 432.1543, found 432.1537. m.p. = 61 °C.

### Preparation of 3-(2-((1,4-dimethoxy-3-methylnaphthalen-2-yl)methyl)-5-(trifluoromethyl)phenoxy) oxetane (JR39):

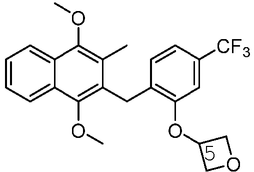

According to this procedure (29% yield, colorless oil) was isolated by purification by flash chromatography on silica gel (Cyclohexane/Ethyl acetate, 8/2).

¹H NMR (400 MHz, CDCl₃) δ 8.1 - 8.0 (m, 2H), 7.6 - 7.5 (m, 2H), 7.5 (d, *J* = 8.0 Hz, 1H), 6.9 - 6.8 (m, 1H), 6.3 (s, 1H), 5.1 (p, *J* = 5.8 Hz, 1H), 4.9 - 4.7 (m, 4H), 4.3 (s, 2H), 3.8 (d, *J* = 7.0 Hz, 6H), 2.2 (s, 3H).

¹³C NMR (126 MHz, CDCl₃) δ 154.3, 151.0, 150.6, 133.5, 129.4 (q, *²J_{C-F}* = 32.5 Hz), 129.1, 128.2, 127.7, 127.3, 126.9, 126.0, 125.7, 124.0 (q, *¹J_{C-F}* = 272.1 Hz), 122.5, 122.3, 118.3 (q, *³J_{C-F} =* 4.1 Hz), 107.1 (q, *³J_{C-F}* = 3.8 Hz), 77.9 (2C), 70.6, 62.4, 61.5, 26.7, 12.5.

¹⁹F NMR (377 MHz, CDCl₃) δ -61.90

HRMS (ESI+) *m*/*z*: [M+H]⁺ calculated for C₂₄H₂₄F₃O₄: 433.1621, found 433.1615.

### Preparation of 3-(2-((1,4-dimethoxy-3-methylnaphthalen-2-yl)methyl)-4-(trifluoromethyl)phenoxy) oxetane (JR124):

According to this procedure (76% yield, colorless oil) was isolated by purification by flash chromatography on silica gel (Cyclohexane/Ethyl acetate, 8/2).

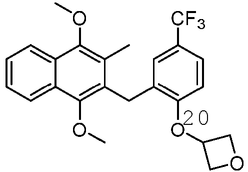

¹H NMR (400 MHz, CDCl₃) δ 8.20 - 8.06 (m, 2H), 7.62 - 7.48 (m, 2H), 7.39 (dd, *J* = 8.4, 2.3 Hz, 1H), 6.97 - 6.92 (m, 1H), 6.47 (d, *J* = 8.5 Hz, 1H), 5.36 (d, *J* = 5.7 Hz, 1H), 5.08 (d, *J =* 6.9 Hz, 2H), 4.87 (dd, *J* = 7.4, 5.2 Hz, 2H), 4.30 (s, 2H), 3.90 (s, 3H), 3.83 (s, 3H), 2.24 (s, 3H).

¹³C NMR (101 MHz, CDCl₃) δ 156.6, 151.1, 150.6, 130.2, 128.2, 127.5, 127.3, 126.8, 126.0, 125.7 (q, ³*J*_{C-F}= 3.7 Hz), 125.6, 124.5 (q, ³*J*_{C-F} = 3.7 Hz), 124.2 (q, ¹*J*_{C-F} = 271.8 Hz) 123.7 (q, ²*J*_{C-F} = 32.6 Hz), 122.6, 122.3, 110.2, 77.8 (2C), 70.6, 62.4, 61.4, 26.7, 12.5.

¹⁹F NMR (377 MHz, CDCl₃) δ -61.44.

HRMS (ESI+) *m*/*z*: [M+K]⁺ calculated for C₂₄H₂₃F₃KO₄: 471.1180, found 471.1195.

### Preparation of 3-(3-((1,4-dimethoxy-3-methylnaphthalen-2-yl)methyl)-4-(trifluoromethyl)phenoxy) oxetane (MR453):

According to this procedure (74% yield, colorless oil) was isolated by purification by flash 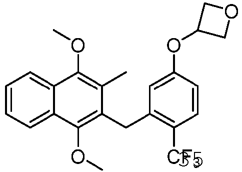 chromatography on silica gel (Cyclohexane/Ethyl acetate, 8/2). ¹H NMR (400 MHz, CDCl₃) δ 8.18-8.08 (m, 2H), 7.62 (d, *J =* 8.6 Hz, 1H), 7.59-7.51 (m, 2H), 6.53-6.46 (m, 1H), 6.10-6.04 (m, 1H), 4.92 (p, *J* = 5.6 Hz, 1H), 4.66 (t, *J* = 6.7 Hz, 2H), 4.54 (dd, *J* = 7.3, 5.2 Hz, 2H), 4.41 (s, 2H), 3.90 (s, 3H), 3.85 (s, 3H), 2.22 (s, 3H).

¹³C NMR (101 MHz, CDCl₃) *δ* 159.2, 151.1, 150.7, 141.8 (q, *³J_{C-F} =* 1.5 Hz), 128.3, 127.9 (q, *³J_{C-F}* = 5.8 Hz), 127.3, 127.2, 126.8, 126.2, 125.8, 124.9 (q, *¹J_{C-F}* = 272.8 Hz), 122.5, 122.4, 121.8 (q, *²J_{C-F} =* 30.4 Hz), 115.3, 110.9, 77.4 (2C), 70.1, 62.3, 61.5, 29.1 (d, *J* =2.4 Hz), 12.4.

¹⁹F NMR (377 MHz, CDCl₃) δ -59.95.

HRMS (ESI+) *m*/*z*: [M+Na]⁺ calculated for C₂₄H₂₃F₃O₄Na: 455.14406, found 455.1425.

### Preparation of 3-(5-((1,4-dimethoxy-3-methylnaphthalen-2-yl)methyl)-2-(trifluoromethyl)phenoxy) oxetane (LF149):

According to this procedure (80% yield, colorless oil) was isolated by purification by flash 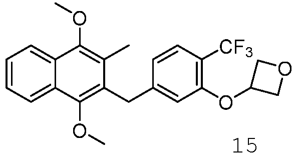 chromatography on silica gel (Cyclohexane/Ethyl acetate, 8/2). ¹H NMR (400 MHz, CDCl₃) δ 8.13-8.05 (m, 2H), 7.56-7.49 (m, 2H), 7.46 (d, *J* = 8.0 Hz, 1H), 6.83 (d, *J* = 7.9 Hz, 1H), 6.25 (s, 1H), 5.08 (m, *J =* 5.7 Hz, 1H), 4.75 (dd, *J* = 7.0, 6.5 Hz), 4.68 (dd, *J* = 7.2, 5.7 Hz, 2H), 4.24 (s, 2H), 3.86 (s, 3H), 3.84 (s, 3H), 2.23 (s, 3H). ¹³C NMR (101 MHz, CDCl₃) δ 154.9, 150.9, 147.1 (2C), 128.5, 128.1, 127.9 (q, ³*J*_{C-F}= 5.4 Hz), 127.5, 126.8, 126.5, 126.1, 123.9 (q, ¹*J*_{C-F} = 274.5 Hz), 122.8, 122.7, 120.9, 116.9 (q, ²*J*_{C-F} = 32.1 Hz), 112.7, 77.8 (2C), 62.6, 61.8, 33.1, 13.0.

¹⁹F NMR (377 MHz, CDCl₃) δ -61.94.

HRMS (ESI+) *m*/*z*: [M]⁺ calculated for C₂₄H₂₃F₃O₄: 432.1543, found 432.1579.

### Preparation of 3-(4-((1,4-dimethoxy-3-methylnaphthalen-2-yl)methyl)-2-(trifluoromethyl)phenoxy) oxetane (MR268):

According to this procedure (49% yield, colorless oil) was isolated by purification by flash chromatography on silica gel (Cyclohexane/Ethyl acetate, 8/2). 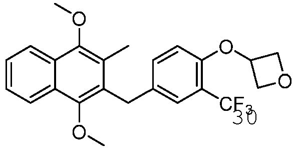 ¹H NMR (400 MHz, CDCl₃) δ 8.09 (dtd, *J* = 7.4, 5.9, 3.6 Hz, 2H), 7.59-7.47 (m, 2H), 7.45 (d, *J* = 1.8 Hz, 1H), 7.08 (dd, *J* = 8.5, 1.7 Hz, 1H), 6.37 (d, *J* = 8.5 Hz, 1H), 5.20 (p, *J* = 5.7 Hz, 1H), 4.92 (t, *J =* 6.8 Hz, 1H), 4.77 (dd, *J* = 7.1, 5.6 Hz, 2H), 4.21 (s, 1H), 3.86 (s, 2H), 3.84 (s, 2H), 2.25 (s, 2H).

¹³C NMR (101 MHz, CDCl₃) δ 152.7, 150.7, 150.6, 133.4, 132.6, 128.4, 128.2, 127.4 (q, ³*J*_{CF} = 5.0 Hz), 127.3, 126.7, 126.1, 125.7, 123.5 (q, ¹*J*_{C-F} = 272.6 Hz), 122.6, 122.4, 119.2 (q, ²*J*_{C-F} = 30.9 Hz), 112.9, 77.7 (2C, overlapping with CDCl3 residual signal and deduced from DEPT experiment), 70.9, 62.4, 61.5, 31.8, 12.7.

¹⁹F NMR (377 MHz, CDCl₃) δ -62.12

HRMS (APCI+) m/z: [M]⁺ calculated for C₂₄H₂₃F₃O₄: 432.1543, found 432.1521.

### PREPARATION OF COMPOUNDS OF FORMULA (VI) (bv nucleophilic aromatic substitution of Fluoroarene by oxetan-3-ol. The compounds of formula (VI) are prepared according to the following reaction scheme:

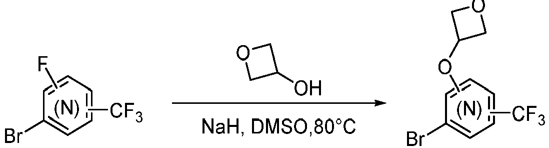

### General procedure for the nucleophilic aromatic substitution of fluoroarenes by oxetan-3-ol:

To a solution of oxetan-3-ol (3 equiv.) in anhydrous DMSO (0.13M), NaH (3 equiv) was added portion-wise under argon atmosphere. The mixture was stirred until homogenous. The resulting mixture was added dropwise to a solution of fluoroarene (1 equiv.) in anhydrous DMSO (0.13M), then the mixture was stirred for 18 h at 80 °C. The reaction was quenched with 1M HCl solution and diluted with CH₂Cl₂. The aqueous layer was extracted with CH₂Cl₂. The organic layer was washed with water, brine, dried over MgSO₄ and concentrated under reduced pressure. The crude residue was purified by flash chromatography on silica gel using the adequate eluent.

| **Fluoroheteroarene** | **Compounds of formula (VI)** | **Yield (%)** | **Reference** |
|---|---|---|---|
| 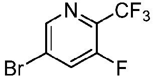 | 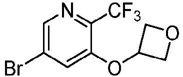 | **99** | **JR128** |
| 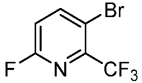 | 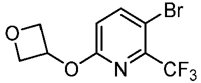 | **92** | **JR212** |
| 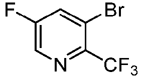 | 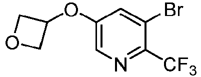 | **99** | **JR236** |

Preparation of 5-bromo-3-(oxetan-3-yloxy)-2-(trifluoromethyl)pyridine (JR128):

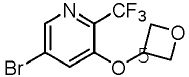

According to this procedure (99% yield, white solid) was isolated by purification by flash chromatography on silica gel (Cyclohexane/Ethyl acetate, 8/2).

¹H NMR (400 MHz, CDCl₃) δ 8.26 (d, *J =* 1.7 Hz, 1H), 7.05 (d, *J =* 1.7 Hz, 1H), 5.24 (p, *J* = 5.3 Hz, 1H), 5.00 - 4.90 (m, 2H), 4.79 - 4.68 (m, 2H).

¹³C NMR (101 MHz, CDCl₃) δ 151.5, 142.1, 135.6 (q, ²*J*_{C-F} = 34.8 Hz), 124.3 (d, ³*J*_{C-F} = 1.3 Hz), 123.7, 121.2 (q, ¹*J*_{C-F} = 274.5 Hz), 76.9 (2C), 71.5.

¹⁹F NMR (377 MHz, CDCl₃) δ -66.30.

HRMS (ESI+) *m*/*z*: [M+H]⁺ calculated for C₉H₈BrF₃NO₂: 297.9685, found 297.9670. m.p. = 68 °C.

### Preparation of 3-bromo-6-(oxetan-3-yloxy)-2-(trifluoromethyl)pyridine (JR212):

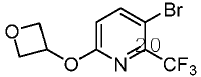

According to this procedure (92% yield, white solid) was isolated by purification by flash chromatography on silica gel (Cyclohexane/Ethyl acetate, 8/2).

¹H NMR (400 MHz, CDCl₃) δ 7.86 (d, *J=* 8.7 Hz, 1H), 6.86 (d, *J=* 8.8 Hz, 1H), 5.57 (p, *J* = 5.9 Hz, 1H), 4.95 (t, *J* = 7.4 Hz, 2H), 4.72 - 4.67 (m, 2H).

¹³C NMR (101 MHz, CDCl₃) δ 160.0, 145.7, 142.9 (q, ²*J*_{C-F} = 34.6 Hz), 139.9, 120.7 (q, ¹*J*_{C-F} = 275.2 Hz), 115.7, 109.7, 77.6 (2C), 70.1.

¹⁹F NMR (377 MHz, CDCl₃) δ -66.28

HRMS (ESI+) *m*/*z*: [M+H]⁺ calculated for C₉H₈BrF₃NO₂: 297.9685, found 297.9688. m.p. = 62 °C.

### Preparation of 3-bromo-5-(oxetan-3-yloxy)-2-(trifluoromethyl)pyridine (JR236):

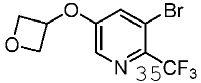

According to this procedure (99% yield, white solid) was isolated by purification by flash chromatography on silica gel (Cyclohexane/Ethyl acetate, 8/2).

¹H NMR (400 MHz, CDCl₃) δ 8.01 (d, *J =* 2.5 Hz, 1H), 7.24 (d, *J =* 2.4 Hz, 1H), 5.21 (p, *J* = 5.4 Hz, 1H), 4.92 (t, *J* = 6.8 Hz, 2H), 4.67 (dd, *J* = 7.6, 4.8 Hz, 2H).

¹³C NMR (101 MHz, CDCl₃) δ 154.6, 138.8 (q, ²*J*_{C-F} = 34.6 Hz), 135.0, 127.3, 121.2 (q, ¹*J*_{C-F} = 274.3 Hz), 118.4, 76.9 (2C), 71.4.

¹⁹F NMR (377 MHz, CDCl₃) δ -64.88.

HRMS (ESI+) *m*/*z*: [M+H]⁺ calculated for C₉H₈BrF₃NO₂: 297.96850, found 297.9686. C₉H₈BrF₃NO₂: 298.96646, found 298.9665.

m.p. = 99 °C.

**PREPARATION OF COMPOUNDS OF FORMULA (II)** (by Miyaura borylation of compounds of formula (VI) which was used with no purification to obtain boronic ester (V)), bv Suzuki reaction between 2-methyl-3-chloromethyl-1,4,-dimethoxynaphthalene and boronic acids of formula (V). The compounds of formula (II) are prepared according to the following reaction scheme

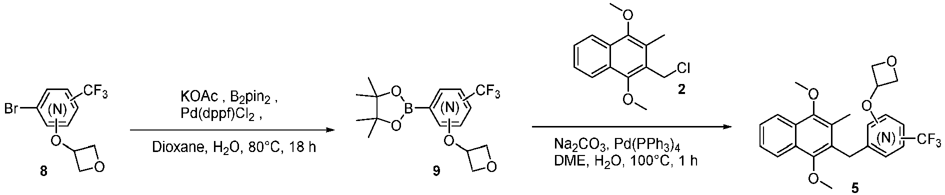

### General procedure for the Miyaura borylation of bromoarenes:

To a mixture of bromoarene (1 equiv.), bis(pinacolato)diboron (1.3 equiv.) and 1,4-dioxane (0.17M) was quickly added KOAc (3 equiv.) and (1,1'-Bis(diphenylphosphino) ferrocene)palladium(II) dichloride (0.05 equiv.). The mixture was stirred vigorously at 100°C. After 18 hours, the mixture was concentrated. The crude was purified by flash chromatography on silica gel using the adequate eluent. This product is used with no other purification.

### General procedure for the Suzuki coupling between 2-(chloromethyl)-1,4-dimethoxy-3-methylnaphthalene and arylboronic acid pinacol ester:

In a flask, 2-(chloromethyl)-1,4-dimethoxy-3-methylnaphthalene 6a (1 equiv), the corresponding boronic acid or boronic acid pinacol ester (1.4 equiv) and sodium carbonate (3 equiv) were dissolved in a 2:1 mixture of dimethoxyethane:water (0.15M). The mixture was bubbled 15min with argon, and then tetrakis(triphenylphosphine)palladium (5 mol%) was added at once. The mixture was heated 1h at 100°C under stirring. The mixture was then, allowed to cool down to room temperature, diluted with water and extracted three times with dichloromethane. Reunited organic layers were washed with brine, dried over magnesium sulfate and the solvent was removed under reduced pressure to afford a crude which was purified on silica gel chromatography using the adequate eluant system to afford the corresponding coupling product.

| **Compounds of formula (VI)** | **Compounds of formula (II)** | **Yield (%)** | **Reference** |
|---|---|---|---|
| 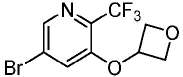 | 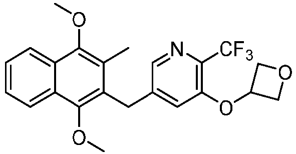 | **61** | **JR131** |
| 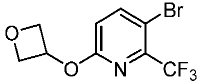 | 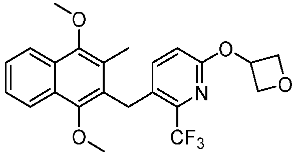 | **35** | **JR217** |
| 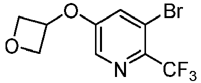 | 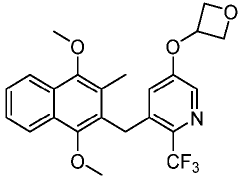 | **61** | **JR239** |
| 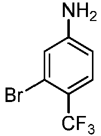 | 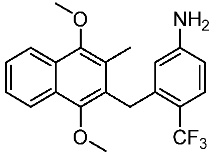 | **44** | **JR250** |
| 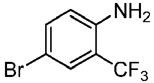 | 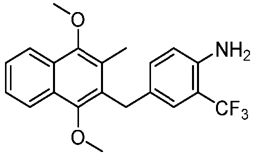 | **41** | **JR251** |
| 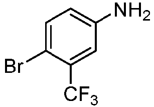 | 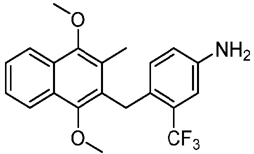 | **45** | **JR280** |
| 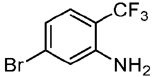 | 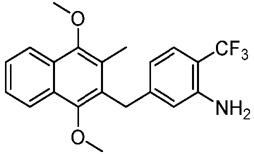 | **77** | **JR275** |

### Preparation of 5-((1,4-dimethoxy-3-methylnaphthalen-2-yl)methyl)-3-(oxetan-3-yloxy)-2-(trifluoromethyl)pyridine (JR131):

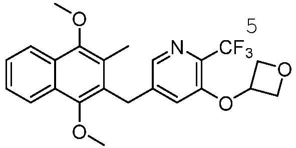

According to this procedure (61% yield, white solid) was isolated by purification by flash chromatography on silica gel (Cyclohexane/Ethyl Acetate, 8/2).

¹H NMR (400 MHz, CDCl₃) δ 8.21 (s, 1H), 8.14 - 8.05 (m, 2H), 7.56 - 7.47 (m, 2H), 6.67 (s, 1H), 5.06 (p, *J* = 5.7 Hz, 1H), 4.72 (d, *J* = 6.8 Hz, 2H), 4.68 - 4.60 (m, 2H), 4.24 (s, 2H), 3.88 (s, 3H), 3.85 (s, 3H), 2.27 (s, 3H).

¹³C NMR (101 MHz, CDCl₃) δ 151.4, 150.8, 150.5, 141.5, 140.9, 134.8 (q, ²*J*_{C-F} = 34.1 Hz), 128.4, 127.1, 127.1, 126.3, 126.0, 125.9, 122.4 (d, ³*J*_{C-F} = 1.9 Hz), 121.7 (q, ¹*J*_{C-F} = 274.4 Hz), 120.6, 77.0 (2C), 70.8, 62.2, 61.5, 29.9, 12.6.

¹⁹F NMR (377 MHz, CDCl₃) δ -65.77.

HRMS (ESI+) *m*/*z:* [M+H]⁺ calculated for C₂₃H₂₃F₃NO₄: 434.1574, found 434.1577. m.p. = 112 °C.

### Preparation of 3-((1,4-dimethoxy-3-methylnaphthalen-2-yl)methyl)-6-(oxetan-3-yloxy)-2-(trifluoromethyl)pyridine (JR217):

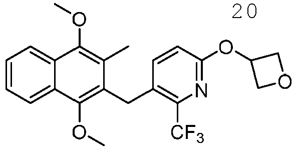

According to this procedure (69% yield, white solid) was isolated by purification by flash chromatography on silica gel (Dichloromethane).

¹H NMR (400 MHz, CDCl₃) δ 8.19 - 8.06 (m, 2H), 7.60 - 7.49 (m, 2H), 7.08 (d, *J* = 8.6 Hz, 1H), 6.74 (d, *J* = 8.6 Hz, 1H), 5.63 (p, *J* = 6.0 Hz, 1H), 5.00 (t, *J* = 6.9 Hz, 2H), 4.79 - 4.71 (m, 2H), 4.38 (s, 2H), 3.88 (d, *J* = 11.8 Hz, 6H), 2.23 (s, 3H).

¹³C NMR (101 MHz, CDCl₃) δ 159.5, 151.1, 150.8, 142.2 (q, ²*J*_{C-F} = 33.5 Hz), 140.7, 128.4, 128.2, 127.2, 126.9, 126.5, 126.3, 125.8, 122.5, 122.4, 122.0 (q, ¹*J*_{C-F} =276.1 Hz), 114.1, 78.0 (2C), 69.6, 62.2, 61.5, 26.9 (q, ⁴*J*_{C-F} = 3.0 Hz), 12.5.

¹⁹F NMR (377 MHz, CDCl₃) δ -64.66.

HRMS (ESI+) *m*/*z:* [M+H]⁺ calculated for C₂₃H₂₃F₃NO₄: 434.1574, found 434.1573. m.p. = 108 °C.

### Preparation of 3-((1,4-dimethoxy-3-methylnaphthalen-2-yl)methyl)-5-(oxetan-3-yloxy)-2-(trifluoromethyl)pyridine (JR239):

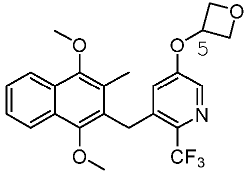

According to this procedure (61% yield, white solid) was isolated by purification by flash chromatography on silica gel (Cyclohexane/Ethyl Acetate, 8/2).

¹H NMR (400 MHz, CDCl₃) δ 7.90 - 7.76 (m, 2H), 7.65 (d, *J =* 2.7 Hz, 1H), 7.33 - 7.22 (m, 2H), 6.12 - 6.07 (m, 1H), 4.70 (p, *J =* 5.5 Hz, 1H), 4.39 (t, *J* = 6.7 Hz, 2H), 4.27 (dd, *J* = 7.6, 4.9 Hz, 2H), 4.12 (s, 2H), 3.59 (d, *J =* 11.6 Hz, 6H), 1.92 (s, 3H).

¹³C NMR (101 MHz, CDCl₃) δ 154.8, 151.1, 150.9, 138.9 (q, ²*J*_{C-F} = 33.5 Hz), 137.1, 133.2, 128.5, 127.1, 126.5, 126.2, 126.1, 126.0, 122.6 (q, ¹*J*_{C-F} = 274.4 Hz), 122.6, 122.5 (d, ³*J*_{C-F} = 3.0 Hz), 77.0, 70.7, 62.3, 61.6, 27.7 (q, 4*J*_{C-F} = 3.0 Hz), 12.5.

¹⁹F NMR (377 MHz, CDCl₃) δ -63.81.

HRMS (ESI+) *m*/*z*: [M+H]⁺ calculated for C₂₃H₂₃F₃NO₄: 434.1574, found 434.1576. m.p. = 66 °C.

### Preparation of 3-[(1,4-dimethoxy-3-methylnaphthalen-2-yl)methyl]-4-(trifluoromethyl)aniline (JR250):

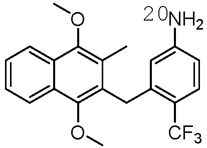

According to this procedure (44% yield, white solid) was isolated by purification by flash chromatography on silica gel (Cyclohexane/ Ethyl acetate, 8/2).

¹H NMR (400 MHz, CDCl₃) δ 8.23 - 8.12 (m, 2H), 7.63 - 7.52 (m, 2H), 7.48 (d, *J* = 8.3 Hz, 1H), 6.43 (d, *J* = 8.4 Hz, 1H), 5.99 (s, 1H), 4.42 (s, 2H), 3.91 (d, *J* = 12.0 Hz, 6H), 3.66 (s, 2H), 2.28 (s, 3H).

¹³C NMR (101 MHz, CDCl₃) δ 151.1, 150.5, 149.7, 140.7 (d, *⁴J_{C-F} =* 1.8 Hz),128.3, 128.1, 127.6 (q, *³J_{C-F}* = 5.8 Hz), 127.4, 127.3, 126.1, 125.7, 125.6 (d, *¹J_{C-F}* = 272.1 Hz), 122.6, 122.3, 118.0 (q, *²J_{C-F} =* 30.1 Hz), 114.5, 111.2, 62.3, 61.5, 29.0 (q, *⁴J_{C-F} =* 2.4 Hz), 12.5. ¹⁹F NMR (377 MHz, CDCl₃) δ -59.00.

HRMS (ESI+) *m*/*z*: [M+H]⁺ calculated for C₂₁H₂₁F₃NO₂: 376.1519, found 376.1538. m.p. = 82 °C.

### Preparation of 4-[(1,4-dimethoxy-3-methylnaphthalen-2-yl)methyl]-2-(trifluoromethyl)aniline (JR251):

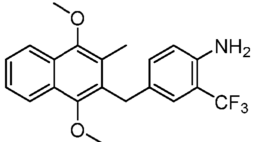

According to this procedure (41% yield, white solid) was isolated by purification by flash chromatography on silica gel (Cyclohexane/Toluene, 8/2).

¹H NMR (400 MHz, CDCl₃) δ 8.26 - 8.16 (m, 2H), 7.64 - 7.53 (m, 2H), 7.36 (d, *J* = 2.1 Hz, 1H), 7.07 (d, *J* = 7.9 Hz, 1H), 6.61 (d, *J* = 8.3 Hz, 1H), 4.26 (s, 2H), 4.07 (s, 2H), 3.93 (d, *J* = 7.4 Hz, 6H), 2.37 (s, 3H).

¹³C NMR (101 MHz, CDCl₃) δ 150.6, 150.6, 142.7 (q, *⁴J_{C-F} =* 1.9 Hz), 132.6, 129.7, 129.1, 128.2, 127.4, 127.1, 126.0 (q, *³J_{C-F}* = 4.8 Hz), 126.0, 125.6, 125.2 (q, *¹J_{C-F}* = 272.4 Hz), 122.6, 122.4, 117.6, 113.8 (q, *²J_{C-F}*= 29.7 Hz), 62.3, 61.4, 31.7, 12.7.

¹⁹F NMR (377 MHz, CDCl₃) δ -62.29.

HRMS (ESI+) *m*/*z*: [M+Na]⁺ calculated for C₂₁H₂₀F₃NnaO₂: 398.1338, found 398.1363. m.p. = 63 °C.

### Preparation of 4-[(1,4-dimethoxy-3-methylnaphthalen-2-yl)methyl]-3-(trifluoromethyl)aniline (JR280):

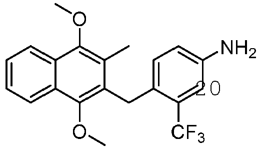

According to this procedure (45%, colorless oil) was isolated by purification by flash chromatography on silica gel (Cyclohexane/ EtOAc, 9/1).

¹H NMR (400 MHz, CDCl3) δ 8.15 - 8.02 (m, 2H), 7.53 - 7.42 (m, 2H), 6.96 (d, *J* = 2.1 Hz, 1H), 6.52 - 6.42 (m, 2H), 4.30 (s, 2H), 3.85 (s, 3H), 3.79 (s, 3H), 2.18 (s, 3H).

¹³C NMR (101 MHz, CDCl₃) δ 151.0, 150.5, 148.4, 144.5, 137.3, 129.7, 129.0 (q, *²J_{C-F} =* 29.6 Hz), 128.5, 128.1, 128.0 (d, *⁴J_{C-F} =* 1.5 Hz), 127.3, 125.9, 125.6, 124.8 (q, , *¹J_{C-F} =* 274.1 Hz), 122.5, 122.3, 118.0, 112.7 (q, , *³J_{C-F}* = 5.9 Hz), 62.3, 61.4, 28.2 (q, , *⁴J_{C-F}* = 12.4 Hz), 12.4.

¹⁹F NMR (377 MHz, CDCl₃) δ -61.17.

HRMS (ESI+) *m*/*z*: [M+H]⁺ calculated for C₂₁H₂₁F₃NO₂: 376.1519, found 376.1505. m.p. = 87 °C.

### Preparation of 5-[(1,4-dimethoxy-3-methylnaphthalen-2-yl)methyl]-2-(trifluoromethyl)aniline (JR275):

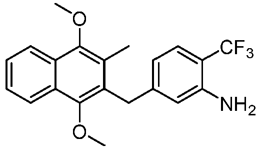

According to this procedure (77%, yellow oil) was isolated by purification by flash chromatography on silica gel (Cyclohexane/Toluene, 8/2).

¹H NMR (400 MHz, CDCl₃) δ 8.30 - 8.19 (m, 2H), 7.66 - 7.55 (m, 2H), 7.41 (d, *J =* 8.1 Hz, 1H), 6.67 (d, *J =* 8.1 Hz, 1H), 6.52 (s, 1H), 4.29 (s, 2H), 4.12 (s, 2H), 3.95 (d, *J* = 5.6 Hz, 6H), 2.38 (s, 3H).

¹³C NMR (101 MHz, CDCl₃) δ 150.8, 150.6, 146.1, 144.9 (q, *³J_{C-F} =* 1.9 Hz), 128.5, 128.3, 127.4, 127.2, 126.7 (q, *³J_{C-F} =* 5.2 Hz), 126.1, 125.8, 125.5 (q, *¹J_{C-F}* = 271.8 Hz), 122.7, 122.4, 117.7, 116.5, 111.6 (q, *²J_{C-F}* = 29.9 Hz), 62.3, 61.4, 32.6, 12.7.

¹⁹F NMR (377 MHz, CDCl₃) δ -61.96.

HRMS (ESI+) m/z: [M+Na]⁺ calculated for C₂₁H₂₀F₃NnaO₂: 398.1338, found 398.1319. m.p. = 79 °C.

**PREPARATION OF COMPOUNDS OF FORMULA (I)** (by oxidative deprotection using CAN from the compounds of formula (IV) or compounds of formula (II)). The compounds of formula (I) are prepared according to the following reaction scheme:

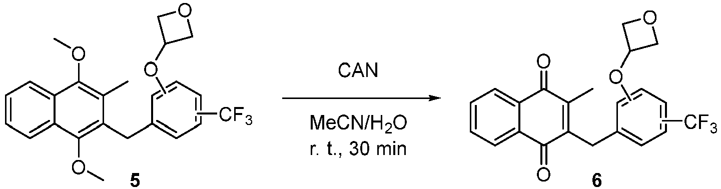

### General procedure for the oxidative deprotection:

Suzuki coupling derivative 3 (1 equiv.) was dissolved in stirring acetonitrile. Then, at room temperature, CAN (2.1 equiv.) dissolved in water was added drop by drop (ratio ACN/H₂O 3:1, 0.05M). The mixture was stirred at room temperature during 30 minutes. The aqueous layer was extracted three times with dichloromethane. Combined organic layers were dried over MgSO4 and the solvent was removed under reduced pressure. Purification by silica gel chromatography was performed using the adequate eluent.

| **Compounds of formula (IV) or (II)** | **Compounds of formula (I)** | **Yield (%)** | **Reference** |
|---|---|---|---|
| 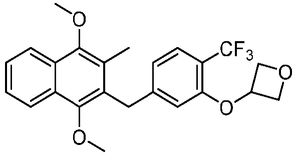 | 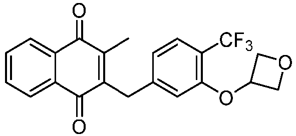 | **78** | **MD40 (batches: LF148, LF151)** |
| 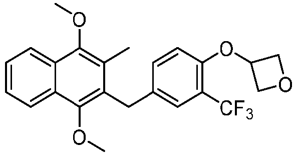 | 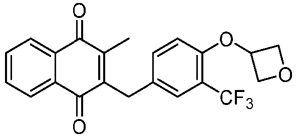 | **73** | **MR271** |
| 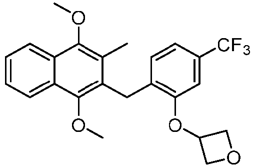 | 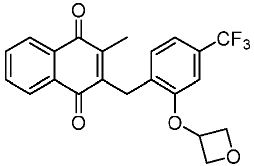 | **73** | **J R44** |
| 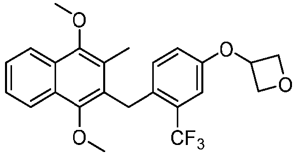 | 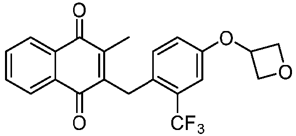 | **97** | **JR48** |
| 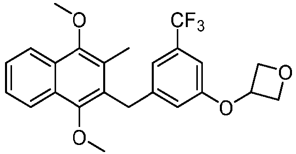 | 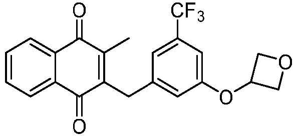 | **60** | **JR51** |
| 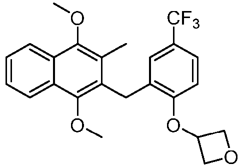 | 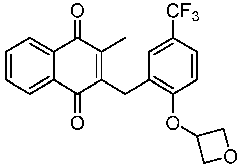 | **93** | **JR126** |
| 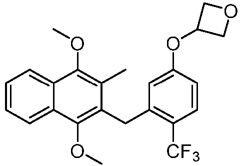 | 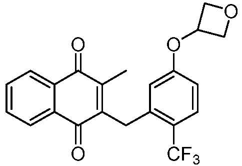 | **77** | **MR457** |
| 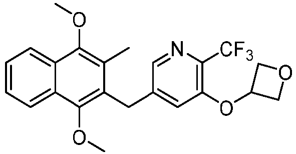 | 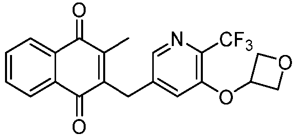 | **88** | **JR136** |
| 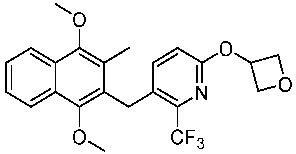 | 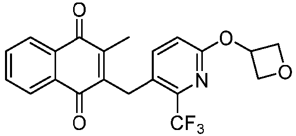 | **60** | **JR220** |
| 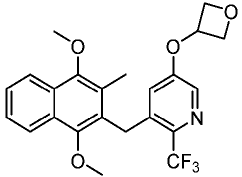 | 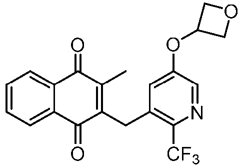 | **69** | **JR245** |

### Preparation of 2-methyl-3-(3-(oxetan-3-yloxy)-5-(trifluoromethyl)benzyl)naphthalene-1,4-dione (JR51):

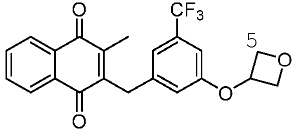

According to this procedure (60% yield, yellow solid) was isolated by purification by flash chromatography on silica gel (Cyclohexane/Ethyl acetate, 8/2).

¹H NMR (400 MHz, CDCl₃) δ 8.13 - 8.04 (m, 2H), 7.75 - 7.66 (m, 2H), 7.12 (s, 1H), 6.79 (s, 1H), 6.70 (s, 1H), 5.19 (q, *J =* 5.1 Hz, 1H), 4.94 (t, *J =* 7.1 Hz, 2H), 4.70 (m, 2H), 4.02 (s, 2H), 2.25 (s, 3H).

¹³C NMR (101 MHz, CDCl₃) δ 185.0, 184.5, 157.0, 144.9, 144.0, 141.1, 133.7 (d, ⁴*J*_{C-F} = 2.6 Hz), 132.3 (q, ²*J*_{C-F}= 32.3 Hz), 132.1, 131.8, 126.5, 126.4, 123.6 (q, ¹*J*_{C-F} = 272.6 Hz), 118.5 (q, ³*J*_{C-F} = 4.1 Hz), 109.1 (q, ³*J*_{*C*-F} = 3.9 Hz), 77.6(2C), 70.4, 32.3, 13.4.

¹⁹F NMR (377 MHz, CDCl₃) δ -62.68.

HRMS (ESI+) *m*/*z:* [M+Na]⁺ calculated for C₂₂H₁₇F₃NaO₄: 425.0971, found 425.0977. m.p. = 107 °C.

### Preparation of 2-methyl-3-(4-(oxetan-3-yloxy)-2-(trifluoromethyl)benzyl)naphthalene-1,4-dione (JR48):

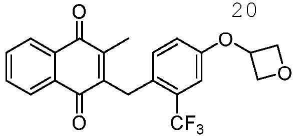

According to this procedure (97% yield, yellow solid) was isolated by purification by flash chromatography on silica gel (Cyclohexane/Ethyl acetate, 8/2).

¹H NMR (500 MHz, CDCl₃) δ 8.11 - 7.99 (m, 2H), 7.73 - 7.64 (m, 2H), 7.04 (d, *J =* 2.7 Hz, 1H), 6.84 (d, *J =* 8.6 Hz, 1H), 6.63 (dd, *J* = 8.6, 2.8 Hz, 1H), 5.15 (p, *J =* 5.0 Hz, 1H), 4.90 (d, *J* = 7.0 Hz, 2H), 4.72 - 4.65 (m, 2H), 4.10 (s, 2H), 2.07 (s, 3H).

¹³C NMR (126 MHz, CDCl₃) δ 184.9, 184.3, 154.9, 146.2, 144.2, 133.7 (2C), 132.2, 131.9, 129.9 (q, ²*J*_{C-F} = 30.1 Hz), 129.8, 129.1, 126.5, 126.4, 124.1 (q, ¹*J*_{C-F} = 274.2 Hz), 117.3, 113.3 (q, ³*J*_{C-F} = 6.1 Hz), 77.6 (2C), 70.4, 27.9, 13.0.

¹⁹F NMR (377 MHz, CDCl₃) δ -61.19.

HRMS (ESI+) *m*/*z:* [M+Na]⁺ calculated for C₂₂H₁₇F₃NaO₄: 425.0971, found 425.0983. m.p. = 110 °C.

### Preparation of 2-methyl-3-(2-(oxetan-3-yloxy)-4-(trifluoromethyl)benzyl)naphthalene-1,4-dione (JR44):

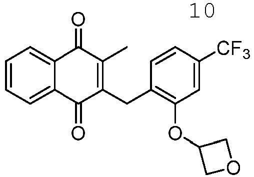

According to this procedure (73% yield, yellow solid) was isolated by purification by flash chromatography on silica gel (Cyclohexane/Ethyl acetate, 8/2).

¹H NMR (400 MHz, CDCl₃) δ 8.05 (dt, *J* = 5.3, 3.1 Hz, 2H), 7.74 - 7.64 (m, 2H), 7.45 (d, *J* = 7.9 Hz, 1H), 6.88 (d, *J* = 8.0 Hz, 1H), 6.48 (s, 1H), 5.24 (p, *J =* 5.6 Hz, 1H), 4.92 (t, 2H), 4.74 (dd, *J* = 7.8, 5.6 Hz, 2H), 3.98 (s, 2H), 2.23 (s, 3H).

¹³C NMR (101 MHz, CDCl₃) δ 184.9, 184.5, 154.6 (q, ³*J*_{C-F} = 1.8 Hz), 144.8, 144.1, 144.0, 133.7 (d, 4*J*_{C-F} = 3.5 Hz), 132.0, 131.8, 127.8 (q, ³*J*_{C-F} = 5.1 Hz), 126.4 (d, ⁴*J*_{C-F} = 2.3 Hz), 123.4 (q, *J* = 272.3 Hz), 120.9, 117.5 (q, ¹*J*_{C-F} = 31.4 Hz), 113.4, 77.5 (2C), 70.7, 32.5, 13.3.

¹⁹F NMR (377 MHz, CDCl₃) δ -62.05.

HRMS (ESI+) *m*/*z*: [M+K]⁺ calculated for C₂₂H₁₇F₃KO₄: 441,0711 found 441.0711. m.p. = 129 °C.

### Preparation of 2-methyl-3-(2-(oxetan-3-yloxy)-5-(trifluoromethyl)benzyl)naphthalene-1,4-dione (JR126):

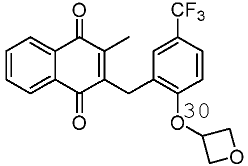

According to this procedure (93% yield, yellow solid) was isolated by purification by flash chromatography on silica gel (Cyclohexane/Ethyl acetate, 8/2).

¹H NMR (400 MHz, CDCl₃) δ 8.14 - 8.04 (m, 2H), 7.71 (dd, *J* = 5.8, 3.3 Hz, 2H), 7.39 (dd, *J* = 8.7, 2.3 Hz, 1H), 7.31 (d, *J =* 2.3 Hz, 1H), 6.43 (d, *J* = 8.5 Hz, 1H), 5.27 (p, *J* = 5.4 Hz, 1H), 4.97 (t, *J* = 7.0 Hz, 2H), 4.73 (dd, *J* = 7.9, 5.0 Hz, 2H), 4.09 (s, 2H), 2.17 (s, 3H).

¹³C NMR (101 MHz, CDCl₃) δ 185.0, 184.3, 156.6, 145.4, 144.3, 133.6 (d, ⁴*J*_{C-F} = 4.8 Hz), 132.1, 132.0, 127.6, 126.8 (q, ³*J*_{C-F} = 3.7 Hz), 126.5, 126.4, 125.1 (q, ³*J*_{C-F} = 4.0 Hz), 123.7 (q, ²*J*_{C-F} = 33.0 Hz), 121.4 (q, ¹*J*_{C-F} = 271.9 Hz), 110.8, 77.5 (2C), 70.7, 26.8, 13.3.

¹⁹F NMR (377 MHz, CDCl₃) δ -61.55.

HRMS (ESI+) *m*/*z*: [M+K]⁺ calculated for C₂₂H₁₇F₃KO₄: 441.0711, found 441.0715. m.p. = 165 °C.

### Preparation of 2-methyl-3-(5-(oxetan-3-yloxy)-2-(trifluoromethyl)benzyl)naphthalene-1,4-dione (MR457):

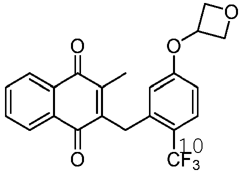

According to this procedure (77% yield, yellow solid) was isolated by purification by flash chromatography on silica gel (Cyclohexane/Ethyl acetate, 8/2).

¹H NMR (400 MHz, CDCl₃) δ 8.20 - 8.06 (m, 2H), 7.80 - 7.70 (m, 2H), 7.60 (d, *J =* 8.6 Hz, 1H), 6.43 (dd, *J*= 8.7, 2.5 Hz, 1H), 6.37 (d, *J =* 2.5 Hz, 1H), 5.10 (p, *J =* 5.5 Hz, 1H), 4.83 (d, *J* = 6.7 Hz, 2H), 4.63 (dd, *J* = 7.4, 5.2 Hz, 2H), 4.19 (s, 2H), 2.12 (s, 3H).

¹³C NMR (101 MHz, CDCl₃) *δ* 184.9, 184.3, 159.1, 146.4, 143.8, 139.1, 133.8 (2C), 132.2, 131.9, 128.3 (q, *³J_{C-F}* = 5.9 Hz), 126.6, 126.5, 124.6 (q, *¹J_{C-F}* = 272.5 Hz), 121.9 (q, *²J_{C-F} =* 30.4 Hz), 115.9, 110.1, 77.4 (2C), 70.3, 28.7 (q, *⁴J_{C-F} =* 2.8 Hz), 13.1.

¹⁹F NMR (377 MHz, CDCl₃) *δ* -59.81.

HRMS (ESI+) *m*/*z*: [M+Na]⁺ calculated for C₂₂H₁₇F₃O₄Na: 425.09711, found 425.0960. M.p. = 148-150 °C.

### Preparation of 2-methyl-3-(3-(oxetan-3-yloxy)-4-(trifluoromethyl)benzyl)naphthalene-1,4-dione (MD40):

According to this procedure (% yield, yellow solid) was isolated by purification by flash 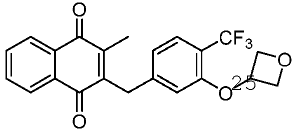 chromatography on silica gel (Cyclohexane/Ethyl acetate, 8/2).

¹H NMR (400 MHz, CDCl₃) δ 8.13-8.05 (m, 2H), 7.76-7.68 (m, 2H), 7.48 (d, *J =* 8.0 Hz, 1H), 6.88 (d, *J =* 8.0 Hz, 1H), 6.48 (s, 1H), 5.25 (m, *J* = 5.8 Hz, 1H), 4.94 (t, *J* = 6.9 Hz, 2H), 4.76 (dd, *J* = 7.5, 5.7 Hz, 2H), 4.00 (s, 2H), 2.26 (s, 3H).

¹³C NMR (101 MHz, CDCl₃) δ 185.3, 184.9, 154.9, 145.1, 144.4, 144.3, 134.1, 134.1, 132.3, 132.1, 128.14 (q, ³*J*_{C-F} = 5.1 Hz), 126.8, 123.7 (q, ¹*J*_{C-F} = 271.5 Hz), 121.2, 117.9 (q, ²*J*_{C-F} = 31.3 Hz), 113.6, 77.9 (2C), 70.9, 32.9, 13.7.

¹⁹F NMR (377 MHz, CDCl₃) δ -62.14

HRMS (ESI+) *m*/*z:* [M+H]⁺ calculated for C₂₂H₁₈F₃O₄: 403.1152, found 403.1169. M.p. = 130-131 °C.

### Preparation of 2-methyl-3-(4-(oxetan-3-yloxy)-3-(trifluoromethyl)benzyl)naphthalene-1,4-dione (MR271):

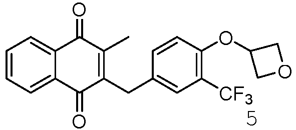

According to this procedure (73% yield, yellow solid) was isolated by purification by flash chromatography on silica gel (Cyclohexane/Ethyl acetate, 7/3).

¹H NMR (400 MHz, CDCl₃) δ 8.09 (ddd, *J* = 6.2, 5.0, 3.2 Hz, 2H), 7.80-7.65 (m, 2H), 7.47 (d, *J* = 2.0 Hz, 1H), 7.37-7.28 (m, 1H), 6.43 (d, *J* = 8.5 Hz, 1H), 5.29-5.14 (m, 1H), 4.94 (t, *J* = 6.8 Hz, 2H), 4.85-4.75 (m, 2H), 3.99 (s, 2H), 2.26 (s, 3H).

¹³C NMR (101 MHz, CDCl₃) δ 185.2, 184.6, 153.1, 144.6, 144.6, 133.7, 133.7, 133.3, 132.1, 131.9, 130.9, 127.8 (q, ³*J*_{C-F} = 4.7 Hz), 126.6, 126.4, 123.3 (q, ¹*J*_{C-F} = 272.7 Hz), 119.4 (q, ²*J*_{C-F} = 31.1 Hz), 113.0, 77.4 (2C, overlapping with CDCl3 residual signal and deduced from DEPT experiment), 71.1, 31.6, 13.4.

¹⁹F NMR (377 MHz, CDCl₃) δ -62.23.

HRMS (APCI+) m/z: [M]⁺ calculated for C₂₂H₁₇F₃O₄: 402.1073, found 402.1058. m.p. = 142 °C.

### Preparation of 2-methyl-3-((5-(oxetan-3-yloxy)-6-(trifluoromethyl)51yridine-3-yl)methyl)naphthalene -1,4-dione (JR136):

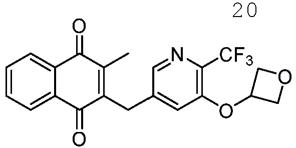

According to this procedure (88% yield, yellow solid) was isolated by purification by flash chromatography on silica gel (Cyclohexane/Ethyl acetate, 7/3).

¹H NMR (400 MHz, CDCl₃) δ 8.11 (s, 1H), 8.01 - 7.90 (m, 2H), 7.68 - 7.58 (m, 2H), 6.89 (s, 1H), 5.28 - 5.15 (m, 1H), 4.87 (t, *J* = 6.8 Hz, 2H), 4.67 (dd, *J* = 7.8, 5.3 Hz, 2H), 3.94 (s, 2H), 2.20 (s, 3H).

¹³C NMR (101 MHz, CDCl₃) δ 184.6, 184.4, 151.2, 145.0, 143.0, 140.9, 139.0, 135.2 (q, ²*J*_{C-F} = 34.2 Hz), 133.9, 133.8, 131.8, 131.6, 126.4 (d, ³*J*_{C-F} = 6.1 Hz), 121.5, 121.5 (q, ¹*J*_{C-F} = 274.4 Hz), 77.1 (2C), 71.0, 29.8, 13.2.

¹⁹F NMR (377 MHz, CDCl₃) δ -66.00.

HRMS (ESI+) *m*/*z*: [M+H]⁺ calculated for C₂₁H₁₇F₃NO₄: 404.1104, found 404.1096. m.p. = 81 °C.

### Preparation of 2-methyl-3-((6-(oxetan-3-yloxy)-2-(trifluoromethyl)51yridine-3-yl)methyl)naphthalene -1,4-dione (JR220):

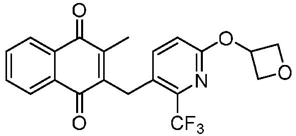

According to this procedure (60% yield, yellow solid) was isolated by purification by flash chromatography on silica gel (Dichloromethane).

¹H NMR (400 MHz, CDCl₃) δ 8.19 - 8.05 (m, 2H), 7.80 - 7.70 (m, 2H), 7.28 (d, *J* = 8.9 Hz, 1H), 6.84 (d, *J =* 8.6 Hz, 1H), 5.61 (p, *J =* 6.0 Hz, 1H), 4.98 (t, *J* = 7.0 Hz, 2H), 4.77 - 4.69 (m, 2H), 4.15 (s, 2H), 2.13 (s, 3H).

¹³C NMR (101 MHz, CDCl₃) δ 184.7, 184.4, 159.6, 146.4, 143.4, 142.4 (q, ²*J*_{C-F} = 33.6 Hz), 140.3, 133.8 (d, ³*J*_{C-F} = 3.8 Hz), 132.1, 131.8, 126.6 (d, ³*J*_{C-F} = 7.1 Hz), 125.9, 121.9 (q, *¹J*_{C-F} = 275.3 Hz), 114.2, 77.9 (2C), 69.7, 26.7 (q, ⁴*J*_{C-F} = 3.0 Hz), 13.1.

¹⁹F NMR (377 MHz, CDCl₃) δ -64.49.

HRMS (ESI+) m/z: [M+H]⁺ calculated for C₂₁H₁₇F₃NO₄: 404.1104, found 404.1105. m.p. = 124 °C.

### Preparation of 2-methyl-3-((5-(oxetan-3-yloxy)-2-(trifluoromethyl)52yridine-3-yl)methyl)naphthalene -1,4-dione (JR245):

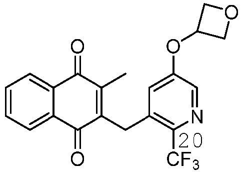

According to this procedure (69% yield, yellow solid) was isolated by purification by flash chromatography on silica gel (Dichloromethane).

¹H NMR (400 MHz, CDCl₃) δ 8.12 - 7.99 (m, 2H), 7.85 (d, *J =* 2.6 Hz, 1H), 7.79 - 7.66 (m, 2H), 6.70 (d, *J* = 2.4 Hz, 1H), 5.17

(p, *J =* 5.4 Hz, 1H), 4.82 (t, *J =* 6.7 Hz, 2H), 4.61 (dd, *J* = 7.5, 4.7 Hz, 2H), 4.16 (s, 2H), 2.09 (s, 3H).

¹³C NMR (101 MHz, CDCl₃) δ 184.5, 184.2, 154.7, 146.7, 142.7, 139.0 (q, ²*J*_{C-F} = 33.5 Hz), 134.7, 133.9 (d, ³*J*_{C-F} = 5.7 Hz), 132.4, 132.0, 131.7, 126.6, 123.3, 122.3 (q, ¹*J*_{C-F} = 274.7 Hz), 77.1, 71.0, 27.4 (q, 4*J*_{C-F}= 2.9 Hz), 13.2.

¹⁹F NMR (377 MHz, CDCl₃) δ -63.62.

HRMS (ESI+) m/z: [M+H]⁺ calculated for C₂₁H₁₇F₃NO₄: 404.1104, found 404.1118. m.p. = 191 °C.

### PREPARATION OF COMPOUNDS OF FORMULA (V) by oxidative deprotection using boron trichloride and TBAI from the compounds of formula (IV) or compounds of formula (II)). The compounds of formula (I) are prepared according to the following reaction scheme:

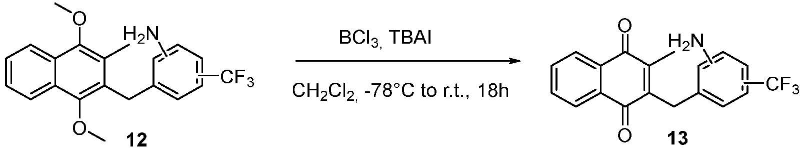

### General procedure for the oxidative deprotection:

To a stirred solution of the Suzuki coupling derivative **12** (1 equiv.) and tetrabutylammonium iodide (2 equiv.) in dry dichloromethane (0.032M) was added dropwise boron trichloride (6 equiv.) at -78 °C. The reaction mixture was allowed to warm up slowly to room temperature and stirred for 18 hours. The reaction mixture was quenched with water and the organic phase was separated. The aqueous phase was further extracted with dichloromethane. The combined organic phases were washed with brine then dried over MgSO₄, filtered, and evaporated under reduced pressure. Purification by silica gel chromatography was performed using the adequate eluent.

| **Compounds of formula (IV) or (II)** | **Compounds of formula (V)** | **Yield (%)** | **Reference** |
|---|---|---|---|
| 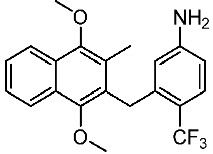 | 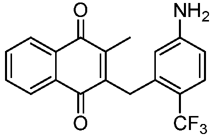 | **97** | **JR256** |
| 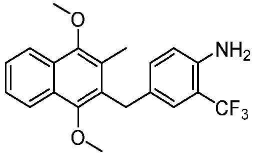 | 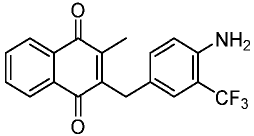 | **89** | **JR257** |
| 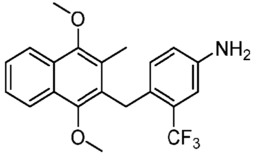 | 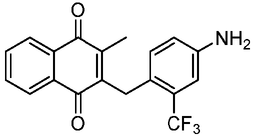 | **69** | **JR285** |
| 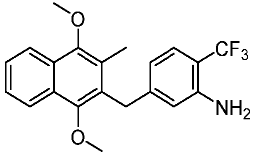 | 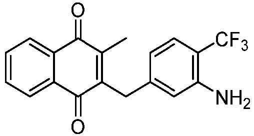 | **89** | **JR279** |

### Preparation of 2-{[5-amino-2-(trifluoromethyl)phenyl]methyl]-3-methyl-1,4-dihydronaphthalene-1,4-dione (JR256):

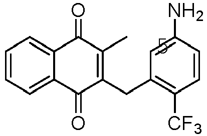

According to this procedure (97% yield, red solid) was isolated by purification by flash chromatography on silica gel (EtOAc/Cyclohexane/Net₃, 90/9/1).

¹H NMR (400 MHz, CDCl₃) δ 8.12 - 8.02 (m, 2H), 7.75 - 7.64 (m, 2H), 7.42 (d, *J =* 8.4 Hz, 1H), 6.48 (d, *J =* 2.3 Hz, 1H), 6.16 (s, 1H), 4.11 (s, 2H), 3.66 (s, 2H), 2.08 (s, 3H).

¹³C NMR (101 MHz, CDCl₃) δ 185.1, 184.4, 149.7, 146.3, 144.5, 138.1 (d, 2C), 133.7 (d, *⁴J_{C-F}* = 2.3 Hz), 132.2, 131.9, 127.9 (q, ³*J*_{C-F} = 5.8 Hz), 126.6, 126.4, 122.5 (q, *¹J_{C-F}* = 272.2 Hz), 118.0 (q, *²J_{C-F}* = 30.1 Hz), 113.9, 111.6, 28.5 (q, *⁴J_{C-F}* = 4.0 Hz), 13.0.

¹⁹F NMR (377 MHz, CDCl₃) δ -58.97.

HRMS (ESI+) m/z: [M+Na]⁺ calculated for C₁₉H₁₄F₃NO₂Na: 368.08688, found 368.0857. m.p. = 162 °C.

### Preparation of 2-{(4-amino-3-(trifluoromethyl)phenyl]methyl}-3-methyl-1,4-dihydronaphthalene-1,4-dione (JR257):

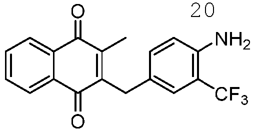

According to this procedure (89% yield, red solid) was isolated by purification by flash chromatography on silica gel (EtOAc/Cyclohexane/Net₃, 90/9/1).

¹H NMR (500 MHz, CDCl₃) δ 8.14 - 8.04 (m, 2H), 7.76 - 7.66 (m, 2H), 7.31 (s, 1H), 7.18 (d, *J* = 8.4 Hz, 1H), 6.74 (d, *J =* 8.4 Hz, 1H), 3.93 (s, 2H), 2.25 (s, 3H).

¹³C NMR (126 MHz, CDCl₃) δ 185.4, 184.7, 145.0, 144.3, 133.6, 133.6, 133.2, 132.1, 132.0, 126.6, 126.5, 126.4, 123.6, 118.2, 31.4, 13.3.

¹⁹F NMR (471 MHz, CDCl₃) δ -62.44.

HRMS (ESI+) m/z: [M+Na]⁺ calculated for C₁₉H₁₄F₃NO₂Na: 368.08688*,* found 368.0854. m.p. = 111 °C.

### Preparation of 2-{[4-amino-2-(trifluoromethyl)phenyl]methyl}-3-methyl-1,4-dihydronaphthalene-1,4-dione (JR285):

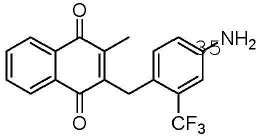

According to this procedure (69%, orange solid) was isolated by purification by flash chromatography on silica gel (Cyclohexane/ EtOAc, 8/2).

¹H NMR (400 MHz, CDCl₃) δ 8.17 - 8.05 (m, 2H), 7.78 - 7.65 (m, 2H), 6.98 (d, *J =* 2.5 Hz, 1H), 6.69 (d, *J =* 8.3 Hz, 1H), 6.61 (dd, *J* = 8.3, 2.5 Hz, 1H), 4.09 (s, 2H), 3.77 (s, 2H), 2.09 (s, 3H).

¹³C NMR (101 MHz, CDCl₃) δ 185.1, 184.5, 146.0, 144.8, 144.8, 133.6, 133.6, 132.2, 132.0, 129.2, 129.2 (q, *²J_{C-F}* = 30.2 Hz), 126.6, 126.4, 125.4 (q, ³*J*_{C-F} *=* 1.5 Hz), 124.5 (q, *¹J_{C-F}* = 274.0 Hz), 117.9, 113.0 (q, ³*J*_{C-F} = 5.9 Hz), 27.8 (q, *⁴J_{C-F}* = 3.6 Hz), 13.0.

¹⁹F NMR (377 MHz, CDCl₃) δ -61.06.

HRMS (ESI+) m/z: [M+H]⁺ calculated for C₁₉H₁₅F₃NO₂: 346.1049, found 346.1043. m.p. = 168 °C.

### Preparation of 2-{[3-amino-4-(trifluoromethyl)phenyl]methyl}-3-methyl-1,4-dihydronaphthalene-1,4-dione (JR279):

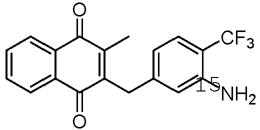

According to this procedure (89%, orange solid) was isolated by purification by flash chromatography on silica gel (Cyclohexane/ EtOAc, 9/1).

¹H NMR (400 MHz, CDCl₃) δ 8.10 - 8.01 (m, 2H), 7.72 - 7.63 (m, 2H), 7.30 (d, *J =* 8.1 Hz, 1H), 6.63 (d, *J =* 8.2 Hz, 1H), 6.59 (s, 1H), 4.12 (s, 2H), 3.94 (s, 2H), 2.22 (s, 3H).

¹³C NMR (101 MHz, CDCl₃) δ 185.2, 184.5, 144.8, 144.8 (q, ³*J*_{C-F} *=* 1.9 Hz), 144.5, 143.3, 133.6, 133.6, 132.1, 131.9, 126.9 (q, ³*J*_{C-F} *=* 5.1 Hz), 126.5, 126.3, 125.0 (q, ¹*J*_{C-F} = 271.8 Hz), 117.9, 116.9, 112.1 (q, *²J_{C-F} =* 30.1 Hz), 32.2, 13.3.

¹⁹F NMR (377 MHz, CDCl₃) δ -62.40.

HRMS (ESI+) m/z: [M+Na]⁺ calculated for C₁₉H₁₄F₃NnaO₂: 368.0869, found 368.0860. m.p. = 142 °C.

### PREPARATION OF COMPOUNDS OF FORMULA (V) by reductive amination of compounds of formula (IV) or (II). The compounds of formula (V) are prepared according to the following reaction scheme:

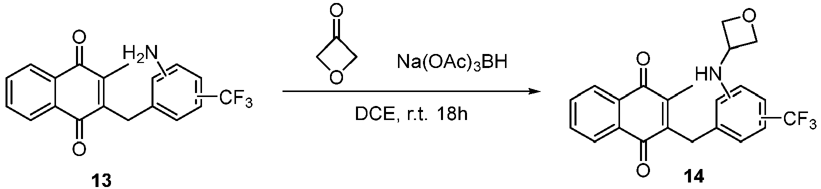

Amino-functionnalized 3-benzyl-menadiones **13** (1 equiv.) and oxetan-3-one (3 equiv.) were dissolved in DCE (0.049M). Acetic acid (5 equiv.) was added, and the mixture was stirred for 5 min. Then sodium triacetoxyborohydride (4 equiv.) were added and the mixture was stirred for 18 hours at room temperature. The reaction mixture was quenched with saturated aqueous NaHCOs and the organic phase was separated. The aqueous phase was further extracted with dichloromethane. The combined organic phases were washed with water and brine then dried over MgSO₄, filtered, and evaporated under reduced pressure. Purification by silica gel chromatography was performed using the adequate eluent.

| **Compounds of formula (IV) or (II)** | **Compounds of formula (V)** | **Yield** (%) | **Reference** |
|---|---|---|---|
| 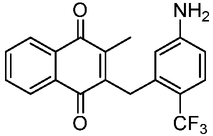 | 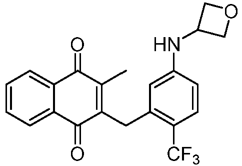 | **31** | **JR264** |
| 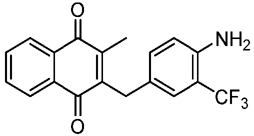 | 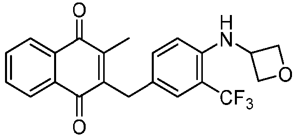 | **0** | |
| 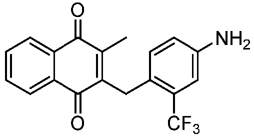 | 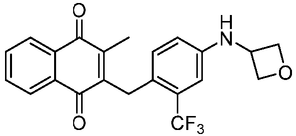 | **58** | **JR287** |
| 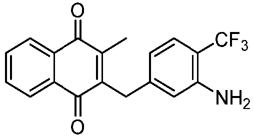 | 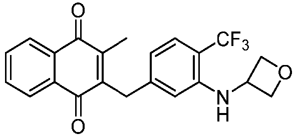 | **0** | |

### Preparation of 2-methyl-3-({5-[(oxetan-3-yl)amino]-2-(trifluoromethyl)phenyl)methyl)-1,4-dihydronaphthalene-1,4-dione (JR264):

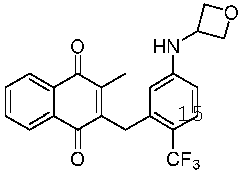

According to this procedure (31% yield, orange solid) was isolated by purification by flash chromatography on silica gel (Cyclohexane/ethyl acetate, 8/2).

¹H NMR (500 MHz, CDCl₃) δ 8.18 - 7.92 (m, 2H), 7.73 - 7.65 (m, 2H), 7.46 (d, *J =* 8.5 Hz, 1H), 6.22 (dd, *J =* 8.6, 2.3 Hz, 1H), 6.05 (d, *J* = 2.3 Hz, 1H), 4.80 (t, *J* = 6.7 Hz, 2H), 4.57 (s, 1H), 4.47 (q, *J* = 6.0 Hz, 1H), 4.36 (t, *J* = 6.2 Hz, 2H), 4.11 (s, 2H), 2.08 (s, 3H).

¹³C NMR (126 MHz, CDCl₃) δ 185.0, 184.4, 148.9, 146.3, 144.4, 138.1, 133.8 (d, *J* = 2.2 Hz, 2C), 132.1, 131.9, 128.2 (q, *³J_{C-F}* = 5.8 Hz), 126.6, 126.4, 125.2 (q, ¹*J*_{C-F} = 272.0 Hz), 118.0 (q, *²J_{C-F} =* 30.4 Hz), 113.1, 108.3, 78.5 (2C), 47.9, 28.6 (q, *⁴J_{C-F}* = 2.6 Hz), 13.1.

¹⁹F NMR (471 MHz, CDCl₃) δ -58.97.

HRMS (ESI+) m/z: [M+Na]⁺ calculated for C₂₂H₁₈F₃NO₃Na: 424.11310, found 424.1119. m.p. = 142 °C.

### Preparation of 2-methyl-3-({4-[(oxetan-3-yl)amino]-2-(trifluoromethyl)phenyl}methyl)-1,4-dihydronaphthalene-1,4-dione (JR287):

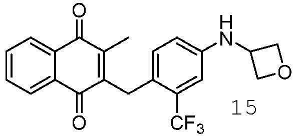

According to this procedure (69%, orange solid) was isolated by purification by flash chromatography on silica gel (Cyclohexane/ EtOAc, 8/2).

¹H NMR (500 MHz, CDCl₃) δ 8.16 - 8.04 (m, 2H), 7.77 - 7.66 (m, *J* = 5.0 Hz, 2H), 6.81 (d, *J* = 2.6 Hz, 1H), 6.73 (d,

*J* = 8.4 Hz, 1H), 6.43 (dd, *J* = 8.4, 2.5 Hz, 1H), 4.96 (t, *J =* 6.6 Hz, 2H), 4.58 (p, *J* = 6.0 Hz, 1H), 4.48 (t, *J* = 6.1 Hz, 2H), 4.09 (s, 2H), 2.10 (s, 3H). ¹³C NMR (126 MHz, CDCl₃) δ 184.1, 183.5, 145.0, 143.7, 143.4, 132.6 (d, *J* = 4.0 Hz, 2C), 131.2, 131.0, 128.4, 128.4 (d, *²J_{C-F}* = 29.5 Hz), 125.5, 125.4, 124.4, 123.4 (q, *¹J_{C-F}* = 273.8 Hz), 114.6, 110.1 (q, ³*J*_{C-F} *=* 6.0 Hz), 77.8 (2C), 47.4, 26.8 (d, *⁴J_{C-F}* = 2.9 Hz), 12.0.

¹⁹F NMR (471 MHz, CDCl₃) δ -61.06.

HRMS (ESI+) m/z: [M+H]⁺ calculated for C₂₂H₁₉F₃NO₃: 402.1312, found 402.1292. m.p. = 145 °C.

### Part III. Biological Activity of the compounds

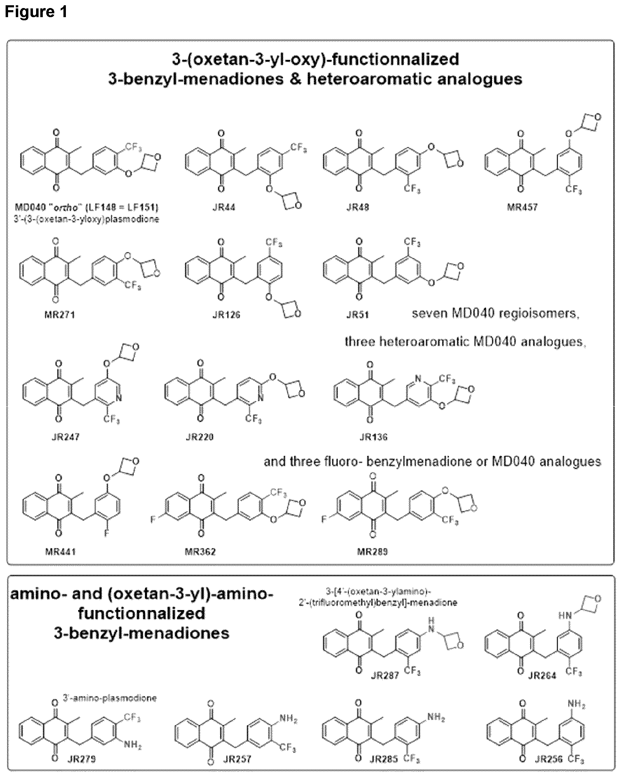

### Pharmacokinetic properties of 3-(oxetan-3-yl-oxy)-functionalized 3-benzyl-menadiones & heteroaromatic analogues (= MD40 and derivatives) (see compounds from figure 1.)

**Table 1. Pharmacokinetic properties of compounds of formula (I):**

| **Code product** | **MW** | **clogP^{a}** | **tPSA^{a}** | **Solubility in in PBS -10% DMSO (µM)^{b}** |
|---|---|---|---|---|
| **MD40** (LF148-LF151) | 402.37 | 5.18 | 52.6 | 3.7 ± 0.7 |
| **MR457** | 402.37 | 5.18 | 52.6 | 0.02 ± 0.01 |
| **JR48** | 402.37 | 5.18 | 52.6 | 0.05 ± 0.02 |
| **MR271** | 402.37 | 5.18 | 52.6 | |
| **JR51** | 402.37 | 5.18 | 52.6 | |
| **JR44** | 402.37 | 5.18 | 52.6 | |
| **JR126** | 402.37 | 5.18 | 52.6 | |
| **JR136** | 403.36 | 403 | 64.9 | |
| **JR220** | 403.36 | 403 | 64.9 | |
| **JR245** | 403.36 | 403 | 64.9 | |
| **MR441** | 352.36 | 4.33 | 69.5 | |
| **MR362** | 420.36 | 5.36 | 52.6 | |
| **MR289** | 420.36 | 5.36 | 52.6 | |
| **atovaquone** | 366.84 | 6.35 | 54.4 | |
| **chloroquine** | 31988 | 5.06 | 27.6 | |
| **artesunate** | 384.43 | 2.93 | 100.5 | |
| **Plasmodione (PD)** | 33031 | 5.42 | 34.1 | 0.44 ± 0.05 |
| ^{a}: ClogP and tPSA were predicted using PerkinElmer Chemdraw as software. | | | | |
| ^{b}: experimental values from TechMed, Illkirch, France | | | | |

The addition of an oxetan-3-yl-oxy- group in *ortho* to the CF₃ group in the original plasmodione scaffold increased the solubility of the molecule **MD40** by 8 fold in aqueous media **(Table 1).**

### 3-(oxetan-3-yl-oxy)-functionalized 3-benzyl-menadiones & heteroaromatic analogues display potent anti-plasmodial activities against P. falciparum asexual stages in vitro

**Table 2. In vitro antimalarial properties of compounds of formula (I) on different P. falciparum lines. Dd2 is chloroquine resistant. ArtS and ArtR are lines originating from Cambodia that are sensitive and resistant to artemisinin, respectively, with ArtS = IPC5188 and ArtR = IPC5202 (see also Figure 3):**

| **Code product** | **IC₅₀** (n: number of repeats) **72h (nM) - drug assays on different *P*. *falciparum* lines** | | | | **rat L6 cells, CC₅₀ (µM)^{a}** | **Human cells, EC₅₀ (µM)^{c}** |
|---|---|---|---|---|---|---|
| | **NF54 IC₅₀** mean ± MAD (n)^{a} | **Dd2 IC₅₀** ± SD (n) ^{b} | **ArtS IC₅₀** ± SD (n)^{b} | **ArtR IC₅₀** ± SD (n)^{b} | | |
| **MD40** (LF148-LF151 batches) | 131 ± 3 (2) - 107 - 143± 20 (4)^{d} | 91.8 ± 12.1 (4) | 42.7 ± 10.3 (8) | 240.2 ± 20.9 (3) | 65.5 | 26 (HepG2) |
| **MR457** | 78 ± 0.9 (2) - 101 ± 23 (4)^{d} | | | | 65.05 | |
| **JR48** | 57 ± 9.5 (2) - 79 ± 24 (4)d | | | | 30.95 | |
| **MR271** | 70 ± 5.5 (2) - 102 - 109± 38 (4)^{d} | | | | 28.9 | |
| **JR51** | 487 ± 44 (2) | | | | 54.65 | |
| **JR44** | 545 ± 61 (2) | | | | 23.5 | |
| **JR126** | 3246 ± 454(2) | | | | >100 | |
| **JR136** | 392 ±58 (3) | | | | 19.12 | |
| **JR220** | 155 ± 19 (4) | | | | 15.16 | |
| **JR245** | 220 ± 11(2) | | | | 15.66 | |
| **JR256** | 114 ± 37 (2) | | | | 66.44 | |
| **JR257** | 150 ± 35 (2) | | | | 33.96 | |
| **JR279** | 65 ± 17 (2) | | | | 45.79 | |
| **JR285** | 188 ± 18 (2) | | | | 61.74 | |
| **JR264** | 93 ± 19 (2) | | | | 97.15 | |
| **JR287** | 120 ± 9.7 (2) | | | | 57.26 | |
| **MR362** | 422 ± 5.4 (2) | | | | | |
| **MR289** | 177± 27 (4) - 164.5 ± 12.95 (2) | | | | 26.35 | |
| **MR441** | 145 ± 4.8 (2) | | | | 69.5 | |
| **Plasmodione (PD)** | 7.5 | 48.2 ± 2.7 (3) | 24.3 ± 8.9 (8) | 135.2 ± 25.6 (3) | 141.7 | 74 (HepG2) 48 (MRC-5) |
| **Chloroquine** | 3.9 (3) - 5.4 (2) | 188.6 ± 12.0 (3) | 95.3 ± 43.2 (7) | 210.4 ± 47.5 (3) | 22.8 | 34 (HepG2) |
| **artesunate** | 1.9(3) - 3.7 (2) | | | | 5.2 | |
| **dihydroartemisinin** | 1.8 ± 0.12 (3) | 0.7 ± 0.2 (5) | 0.9 ± 0.2 (8) | 6.0 ± 0.2 (3) | ND | |
| **Methylene Blue** | | 7.1 ± 0.3 (3) | 5.1 ± 3.3 (8) | 10.9 ± 0.7 (3) | | |
| **podophyllotoxin** | | | | | 0.018 | |
| ^{a}: data from STPH, Basel, Switzerland. The IC₅₀ values mean ± median absolute deviation (MAD) from 2-4 experiments (n) of the antimalarial drugs in the ³H-hypoxanthine incorporation-based assay using the P. *falciparum* NF54 strain with chloroquine, artesunate as drug references. Dihydroartemisinin is the major artemisinin metabolite. Number of repeats in duplicate is given in brakets. The color code indicates the IC₅₀ data made in parallel in the same experiment. | | | | | | |
| ^{b}: data from IBMC, Strasbourg, France. Mean ± standard deviation (SD) from 3-8 experiments (n); | | | | | | |
| ^{c}: data from Plateforme de chimie biologique intégrative de Strasbourg (PCBiS) - UAR 3286, Illkirch, France; the cell line used for the assay is indicated in brakets; | | | | | | |
| ^{d}: Mean ± SEM | | | | | | |
| ^{e}: preliminary data, only one measure | | | | | | |

### Results:

The antimalarial properties of **MD40** and several derivatives are tested in growth assays where *Plasmodium falciparum* parasite cultures (NF54, Dd2, ArtS and ArtR) were exposed to the drugs for 72h, and the 50% inhibitory concentration determined (IC₅₀, Table 2). The sensitivity of mammalian cells to these compounds was also determined. Plasmodione (PD), the original lead benzylmenadione (bMD), and several known antimalarial drugs are used as controls.

### Plasmodium falciparum antiplasmodial [³H]-hypoxanthine incorporation-based assays with the NF54 strain (STPH, Basel).

**MD40** and several derivatives (MR457, JR48, MR271, JR220 and JR245) present IC₅₀s below 250 nM, with several of them consistently under 150 nM **(MD40,** MR457, JR48, MR271).

### SYBR^{®} green staining-based antimalarial assays performed on ring stages of three

***Plasmodium falciparum* strains, Dd2, ArtS and ArtR (IBMC).** We demonstrated that **MD40** is active *in vitro* on all tested P. *falciparum* strains, albeit twice less so than PD. Please note that a 2-3 fold decrease in sensibility is observed for the two bMDs we have tested with the artemisinin-resistant ArtR line **(MD40** and PD, please also see § below on cross-resistance with artemisinin).

### Cytotoxicity assays on rat L6 cell line (STPH) and human HepG2 cells (PCBiS).

The selectivity index of **MD40,** ie ratio between the IC50 on rat L6 cells / that on parasites is 458, indicates a good selectivity of **MD40** against parasites.

In human cells, **MD40** (LF151.F1) is cytotoxic from 30 µM (27% confluence at 72 h of treatment) on HepG2 cells (IC₅₀ = 26 µM) . PD is cytotoxic from 60 µM (51% confluence at 72 h of treatment) on HepG2 cells (IC₅₀ = 74 µM). By comparison, chloroquine (CQ) is cytotoxic from 10 µM (72% confluence at 72h treatment) on HepG2 cells (IC₅₀ = 34 µM).

In conclusion, the selectivity index (ratio between the IC₅₀ on mammalian cells / that on parasites) of **MD40** and derivatives for which we currently have data is 250-1000, indicating a good selectivity of the drugs against parasites.

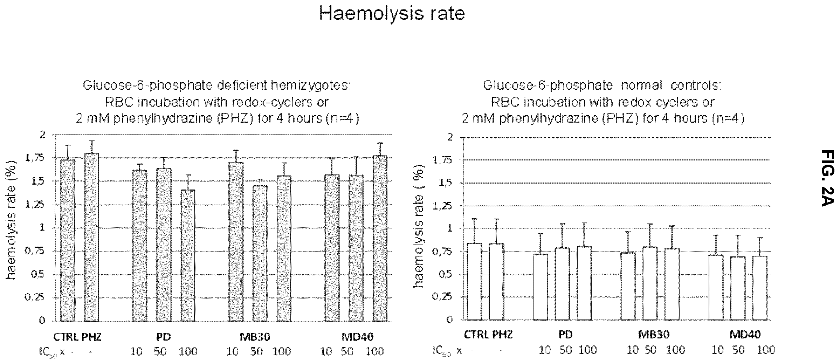

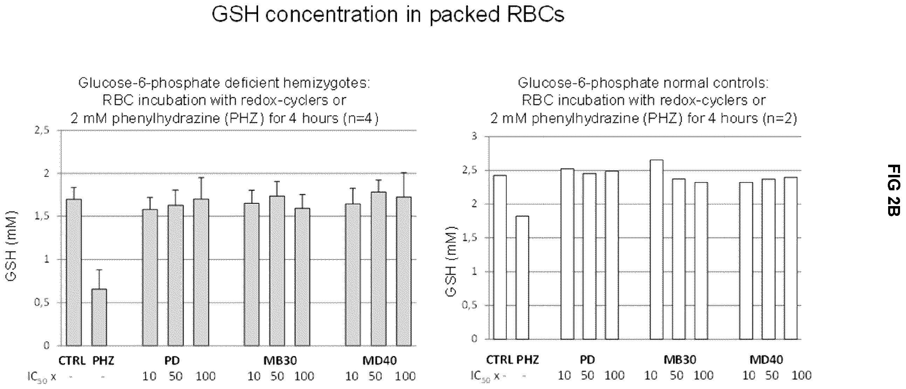

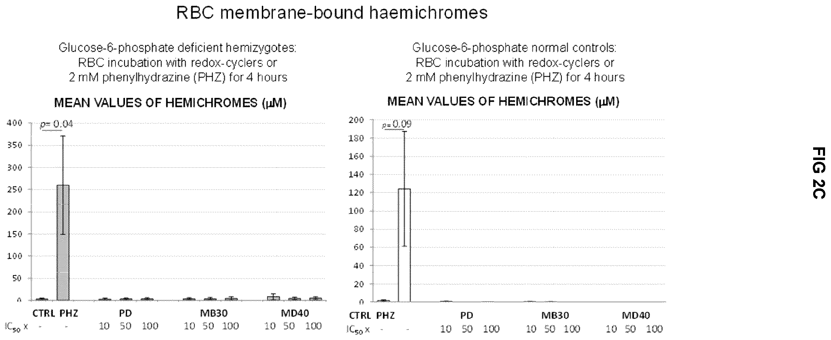

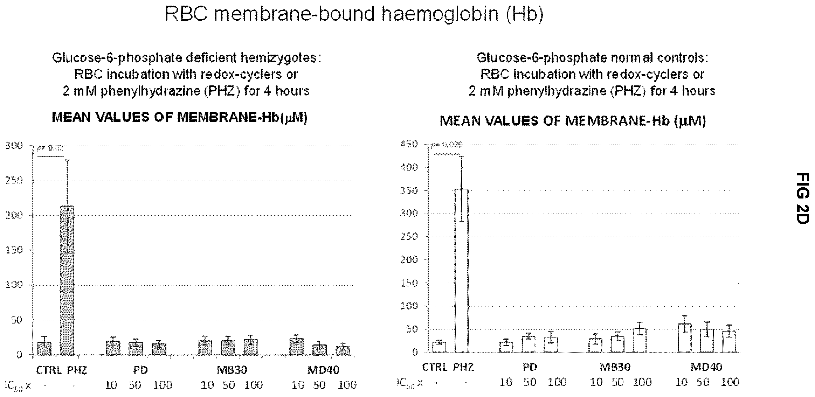

**Figure 2****.** shows the evaluation of the toxicity of PD, **MD30** and **MD40** on G6PD-normal and -deficient red blood cells (RBCs). Both types of RBCs were exposed to different concentrations of the drugs (10x, 50x and 100x IC₅₀) for 4h. Several parameters were then evaluated: **A.** RBC hemolysis ; **B.** Glutathione (GSH) concentration in packed RBCs ; C. RBC membrane-bound hemichromes ; and **D.** RBC membrane-bound hemoglobin. PH means phenylhydrazine; Control means no drug. MB30 is the 6-fluoroplasmodione (Rodo et al. *platform of regioselective methodologies to access to polysubstituted 2-methyl-1,4-naphthoquinones derivatives: scope and limitations.* Eur. J. Org. Chem. **2016,** 11, 1982-1993. doi: 10.1002/ejoc.201600144).

Red blood cells (RBCs) from G6PD-normal and -deficient patients were incubated with redox cyclers PD, MD030 and **MD40** at three different concentrations (10x IC₅₀, 50x IC₅₀, and 100x IC₅₀) or with phenylhydrazine (PH) at 2mM as control for 4 hours (n=4). Incubation with the redox cyclers did not induce an increased hemolysis rate **(****fig. 2A****).** Similarly, and in contrast to PH treatement, incubation of G6PD-normal and -deficient RBCs with PD, MD30 and **MD40** had no effect (1) on glutathione (GSH) concentration which reduction indicates oxidative stress **(****fig. 2B****),** (2) on the level of RBC membrane-bound hemichromes, a marker of RBC senescence **(****fig. 2C****)** and (3) on the level of RBC membrane-bound hemoglobin **(****fig. 2D****).** Taken together, these results suggest that **MD40** could be safely used in G6PD-deficient patients.

### In vivo activity of MD40 and analogues on P. berghei asexual stages.

**Table 3. In vivo antimalarial properties and transmission blocking activity of compounds of formula (I) on P. berghei in mice.**

| **Code product** | Reduction of *P*. *berghei* parasitemia | | Reduction of *P*. *berghei* transmission^{a} | |
|---|---|---|---|---|
| | ip, 3 doses at 30 mg/kg^{a} | *per* os, 4 doses at 50 mg/kg^{b} | Prevalence | Parasite # per infected mosquito |
| **MD40** (LF148-LF151 batches) | - 90% | - 96% | - 87% | - 91% |
| **MR457** | | 0 | | |
| **JR48** | | 0 | | |
| **JR220** | | 0 | | |
| **JR245** | | 0 | | |
| **plasmodione (PD)** | - 50% | 0 | - 45% | - 60% |
| **chloroquine** | | 99.9%^{c} | | |
| **(dihydro)artemisinin** | | 99.1%^{c} | | |
| **Methylene Blue** | - 97% | | - 93% | - 97% |
| a: data from IBMC, Strasbourg (see Fig.4); | | | | |
| b: data from STPH, Basel. | | | | |
| c: 4 x30 mg/kg | | | | |

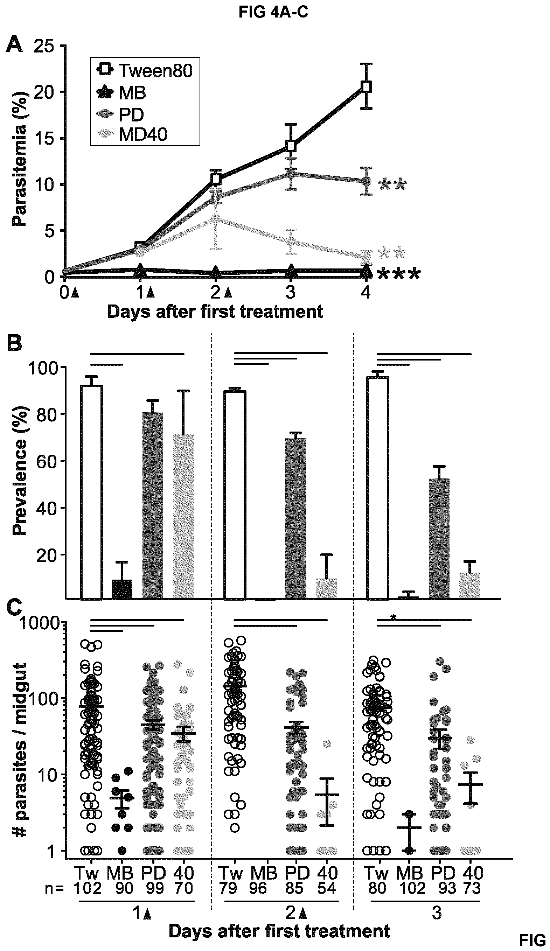

**MD40** and several analogues have also been tested *in vivo,* in parallel to plasmodione (PD) **(Table 3** and **Fig. 4A****). MD40** decreases parasitemia in mice by 90% after 3 intraperitoneal treatments on three consecutive days, and by 96% after 4 daily doses *per os.* In contrast, plasmodione is twice less effective ip and has no activity *per os,* indicating that the oxetane modification is important to either increase bMD bioavailability, decrease its metabolism, or increase its antimalarial activity *in vivo,* or a combination of these. Importantly, mouse ip treatment with **MD40** and PD also reduced parasite transmission to mosquitoes (see specific paragraph below).

### 3-Benzylmenadiones display potent anti-gametocyte activity against P. falciparum stages transmissible to mosquitoes (in vitro assays)

**Table 4. In vitro gametocytocidal activity of MD40, plasmodione (PD) and methylene blue (MB) on P. falciparum gametocytes at different stages of their development. The transgenic parasite line pfs16-GFP-Luc (NF54 background) expressing luciferase during gametocytogenesis was used following the same protocol as in Adjalley et al, PNAS 2011 (Adjalley, S.H. et al. Quantitative assessment of Plasmodium falciparum sexual development reveals potent transmission-blocking activity by methylene blue. Proc. Natl. Acad. Sci. U.S.A. 108, E1214-E1223 (2011)) Experiments performed at IBMC, Strasbourg.**

| | **IC₅₀ ± SD (nM) for *P. falciparum* NF54 gametocytes** | | |
|---|---|---|---|
| **Product code** | **early stages** | **mid-late stages** | **mature stages** |
| **MD40** | 4.3 ± 4.7 | 82.8 ± 20 | 228 ± 58 |
| **PD** | 20.8 ± 26 | 3,6 ± 4 | >10,000 |
| **MB** | 24 ± 10.5 | 157 ± 181 | 97 ± 17.7 |

The gametocytocidal activity of **MD40** and **PD** was tested on P. *falciparum* gametocytes (sexual stages that are the only ones transmitted to mosquitoes), using a transgenic parasite line expressing luciferase during gametocytogenesis *(pfs16-GFP-Luc,* NF54 background) and following the same protocol as in Adjalley et al, PNAS 2011 (Table 4**).** in Methylene blue (MB), a known gametocyte killer, was used as positive control. Gametocytes at different stages (early: stages II-III, mid-late: stage IV, and mature: stage V) were exposed to the compounds for 72h, and gametocytemia was determined by a luciferase assay after parasite lysis. Similarly to methylene blue, **MD40** presents a clear gametocytocidal activity on all gametocyte stages, including on mature stages that are resistant to most drugs used to treat malaria. In contrast, PD activity is restricted to early gametocytes.

### 3-Benzylmenadiones reduce transmission of P. falciparum to mosquitoes in indirect standard membrane feeding assay.

**Figure 3** shows the transmission blocking-activity of MD40 and PD on P. *falciparum* using an indirect standard membrane feeding assay. A culture of P. *falciparum* gametocyte stage V (transgenic strain expressing luciferase at mosquito stages) was pre-incubated for 24h with different concentrations (uM) of **MD40** (light gray), plasmodione (PD, dark gray), with the vehicle (DMSO, white) or with gametocyticidal methylene blue (10 uM, MB, black) as a positive control, and then offered to mosquitoes for a blood meal. **A.** Prevalence (percentage of infected mosquitoes) and **B.** level of infection (measured by the intensity of luciferase activity) were determined 7 days post blood feeding. In B., each dot shows the luciferase activity in a single mosquito, and the mean and standard error of the mean are plotted. The light dotted line represents the threshold above which mosquitoes are considered infected. Please note that the overall infection was very high in the PD experiment, leading to a prevalence of ~ 100% for all doses. Experiments planned at IBMC Strasbourg and performed at TroplQ in Njemegen, the Netherlands.

The ability of **MD40** and **PD** to reduce parasite transmission to mosquitoes is assessed. For this, gametocytes were incubated for 24h together with the compounds at different concentrations, or with methylene blue at 1 µM as a positive control. DMSO (0.1%), the vehicle, served as negative control. Mosquitoes were then allowed to feed on treated gametocytes, and maintained for 7 days to allow parasites to develop until the oocyst stage. Both molecules display a dose-dependent effect, with a decrease in one or two of the infection parameters. Indeed, both prevalence (i.e. the percentage of mosquitoes that became infected) and infection levels (i.e. the average amount of parasites in infected mosquitoes, measured using luciferase activity as a readout) were decreased when mosquitoes were exposed to **MD40,** with an almost complete transmission blocking at 3.17 and 10 µM. The effect of **PD** was less visible: there was no significant change in prevalence, however a dose-dependent decrease in infection levels was observed. The seemingly lower activity of **PD** compared to **MD40** could be explained by a lower efficiency of this compound gametocytes or early parasite stages in mosquitoes (coherent with a lower gametocytocidal activity), and/or by technical differences in the infection parameters between the two experiments (the luciferase activity of controls in the **PD** experiment was about 10 times higher than that with **MD40).**

### 3-Benzylmenadiones reduce both parasitemia in mice and transmission of P. berghei to mosquitoes (in vivo)

**Figure 4****.** shows the effect of 3-benzylmenadiones on the parasitemia of mice infected with the murine malaria parasite *P. berghei,* and on its transmission from mice to mosquitoes. Naïve mice were injected i.v. with blood infected with a transgenic line of parasites expressing constitutively GFP at all stages (*PbGFPcon*)*,* and treated daily for 3 consecutive days, starting 24h post passage, with plasmodione (PD) and **MD40** (30 mg/kg) or with methylene blue (MB, 15 mg/kg) and vehicle (Tw80) as positive and negative controls, respectively. Parasitemia was monitored daily and groups of mosquitoes were fed on mice 24 h after each treatment. A. Parasitemia (mean ± standard deviation of four independent experiments) are plotted for each treatment. After 3 treatments, parasitemia was reduced by 50% for PD, by 90% for **MD40** and by 97% for MB as compared to the negative control Tw. **B.** Prevalence (percentage of infected mosquitoes), and C. infection intensity (mean number of parasites per infected mosquito, shown on log scale) were determined 7 days post infectious blood feedings. For this, mosquitoes that blood fed on infected and treated mice were dissected, and the number of parasites counted. The results of four independent experiments were pooled and the mean ± standard error of the mean are shown (B, C). Significance for differences between treatments was calculated using generalized linear models with treatment, time, and repeats as variables (A-C). n, number of mosquitoes. The reduction of parasitemia in mice (A) and of prevalence (B) and level of infection (C) in mosquitoes is also summarised in **Table 3.** Experiments performed at IBMC, Strasbourg.

In this set of experiments **(****Figure 4****),** mice were infected with a P. *berghei* line constitutively expressing *GFP* (*PbGFPcon*)*,* and treated once a day starting 24h after mouse infection with **MD40, PD** or methylene blue (MB) as a positive control. Tween80 is the vehicle for bMDs and was used as negative control. We followed mouse parasitemia daily **(****Fig. 4A****)** and groups of mosquitoes were fed on these mice 24h after the first, second or third treatment. They were dissected at 7-8 days post infection and parasites were counted to measure prevalence (ie. percentage of infected mosquitoes, **Fig. 4B****)** and infection levels (ie. the average number of parasites per infected mosquito, **Fig. 4C****).** Both **MD40** and PD reduce mouse parasitemia, reaching 90% and 50% reduction with **MD40** and **PD,** respectively. Similarly, **MD40** was more efficient than **PD** at reducing mosquito infection, reaching 87% and 91% decrease in prevalence and infection levels, respectively, as compared to 45% and 60% with **PD (Table 3).**

### Cross-resistance of MD40 and plasmodione with Artemisinin / DHA

**Figure 5****.** Cross-resistance of plasmodione (PD) and **MD40** with dihydroartemisinin (DHA) demonstrated by Ring stage Survival Assay (RSA) on multiple lines with different sensitivity to artemisinin (ArtS to ArtR+++, IPC numbers of the different lines are given) using microscopy as a readout (A) and on IPC5202 (the most resistant line) using cytometry **(B).** Experiments performed at Institut Pasteur du Cambodge **(A)** and IBMC **(B).**

Artemisinin-resistance is measurable by an increased survival of ring stages to short Art pulses at high concentrations (RSA: 6h, 700 nM DHA). To evaluate the cross-resistance of PD or **MD40** with artemisinin, we first performed a classical RSA on multiple Cambodian lines with microscopic readout (Witkowski B, et al. Novel phenotypic assays for the detection of artemisinin-resistant Plasmodium falciparum malaria in Cambodia: in-vitro and ex-vivo drug-response studies. Lancet Infect Dis. 2013, 13(12):1043-9. doi: 10.1016/S1473-3099(13)70252-4) after Giemsa staining **(****fig. 5A****),** and then established a readout by flow cytometry as in Amaratunga *et al.,* 2014 (Amaratunga, C. et al. (2014). Flow Cytometry-Based Analysis of Artemisinin-Resistant Plasmodium falciparum in the Ring-Stage Survival Assay. Antimicrobial Agents and Chemotherapy, 58(8), 4938-4940. https://doi.org/10.1128/AAC.02902-14). For this **(****fig. 5B****),** we selected the lowest concentration of the two drugs that killed all ArtS (IPC 5188) parasites upon a 6h exposure on 0-3h post invasion rings: 700 nM DHA as a control, 1 µM for PD and 2 µM for **MD40** and used these on ArtR (IPC 5202) parasites and measured their survival by flow cytometry using both SYBR green (to detect all infected red blood cells) and Mitotracker (to detect live parasites only). In both cases, tightly synchronised parasites (0-3hpi rings) were exposed to the drugs for 6h, the drug was then removed and parasites allowed to grow for another 66 hours. The percentage of live parasites as compared to untreated samples was determined. The clear correlation between the level of resistance to Art and to PD and MD40 indicates the existence of cross-resistance of PD or **MD40** with Art **(****Fig. 5****).**

### Several drug combinations demonstrate synergy between MD40 and several partners in both ring stage survival and transmission-blocking assays

**Figure 7** shows RSA on ArtR (IPC5202) to test drug combinations of benzylmenadiones, **MD40** (light gray) or plasmodione (PD, dark gray), with partner molecules using flow cytometry as a readout. Except for DHA at 700nM, all partners were tested at concentrations that have no effect on ArtR when applied alone (see **Table 5).** The ratio survival [bMD + partner] / survival [bMD alone] is plotted for each combination (mean and standard deviation for combinations tested more than once). A ratio below 1 is characteristic of synergistic partners, above 1, of antagonist partners, and the ratio is 1 when there is no interaction between the two compounds at the concentrations tested (additive effects). **XM172** and APHA were tested once, **XM184,** twice and all others three times. Experiments performed at IBMC, Strasbourg.

To identify partner molecules that could synergise with benzylmenadiones to reduce resistance in ArtR parasites (IPC5202), drug combinaisons were tested as explained in **figure 7****.** For this, the highest concentration at which a given partner molecule **(****Figure 6****)** does not affect ArtR parasite survival upon a 6 h exposure was first determined **(Table 5),** and then tested at this concentration in combinaison with PD (1uM) or **MD40** (2uM). We plotted the ratio survival [bMD + partner] / survival [bMD alone] **(****Figure 7****).** Several compounds acting synergically with PD and **MD40** were detected : (1) DHA, (2) **XM193,** an iron chelator, (3) 2 epigenetic modifiers (HDAC inhibitors): **FR235222** and **APHA,** and (4) 2 antifungal compounds: miconazole and econazole. Further work is required to determine concentrations of bMDs and synergistic partners for optimal synergism.

**Figure 8****.** shows RSA on ArtR (IPC5202) to test drug combinations with DHA using flow cytometry as a readout. Same protocol as in **Fig. 7****,** but with DHA at 700 nM as the main drug in all samples, and PD and **MD40** used as combination partners at 25nM. The ratio survival [DHA + partner] / survival [DHA alone] is plotted for each combination (mean and standard deviation for combinations tested more than once). Experiments performed at IBMC, Strasbourg.

A few of the partner molecules are also tested in combination with DHA to assess whether they might as well be synergistic with DHA **(****Figure 8****).** The same strategy as previously described was used, this time with DHA at 700 nM (physiological concentration), PD and **MD40** at 25 nM (a concentration that does not kill ArtR parasites when exposed to the drugs for 6h in RSA) and a few selected partner drugs at the same concentration as in Fig. 7. We confirmed that PD and **MD40** act synergistically with DHA in this reciprocal configuration. In our assay, one compound acts synergistically with both DHA and bMDs: **XM193;** one is synergistic with DHA only: **XM184,** while the two antifungal compounds (miconazole and econazole) are synergistic with bMDs only. These data strongly indicate that bMDs and DHA have different modes of action.

**Figure 9** shows transmission blocking test performed as in **Fig. 3****,** with **MD40** at 316 nM and partner molecules at concentrations indicated on the graphs and in Table 5. **A.** Reduction of infection due to the compound alone as compared to DMSO. The ratio infection intensity upon drug treatment / infection intensity of DMSO is plotted (MD40 in gray without border, partner drugs in white with black borders), DHA 10 µM is used as a positive control (black bar). A ratio below 1 indicates that the drug is effective on its own at the chosen concentration. **B.** For each combination of **MD40** with partner, the ratio infection intensity upon drug combination / infection intensity upon **MD40** alone is plotted (gray with black borders). A ratio below 1 indicates synergy between the 2 drugs, a ratio above 1, antagonism, and a ratio of 1, additive effects. Mean and standard errors of the mean are indicated. Experiments performed in duplicates at TroplQ in Njemegen, the Netherlands with drugs and concentrations selected at IBMC, Strasbourg.

Some of the drug combinations with **MD40** were also tested in the P. *falciparum* transmission blocking assay **(****Figure 9****). MD40** was used at 316 nM, a concentration that has no effect on parasite transmission **(****Figure 3****).** Partner drugs are tested alone in parallel to confirm that they have no effect on their own at the chosen concentration **(****Figure 9A****).** We detected synergy between **MD40** and the antifungal miconazole in this transmission-blocking assay **(****Figure 9B****).** In contrast **MD40** activity was not boosted by the presence of DHA and **XM184** at the chosen concentrations (please note the high variability between duplicates). This could be due to a lack of synergism between **MD40** and DHA or **XM184** in this specific assay, or to selected concentrations that were not optimal.

Taken together, these data demonstrate that miconazole is synergistic with **MD40** in all tests that we have performed, and thus represents an interesting partner of 3-benzylmenadiones.

### MD40 and artesunate act synergistically in vivo to kill P. falciparum in a humanised mouse model

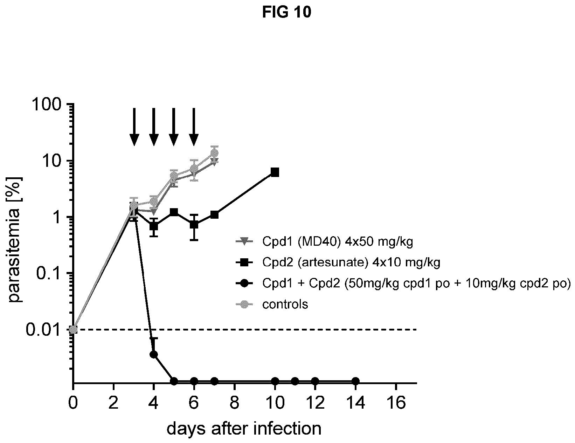

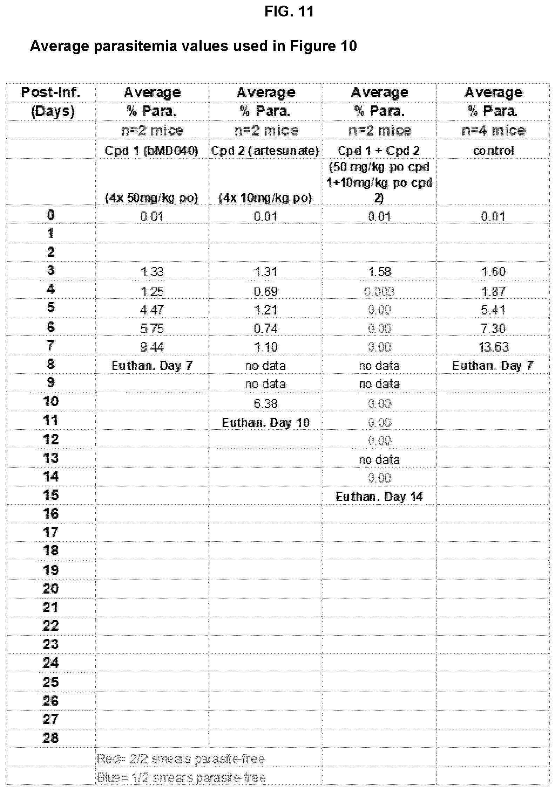

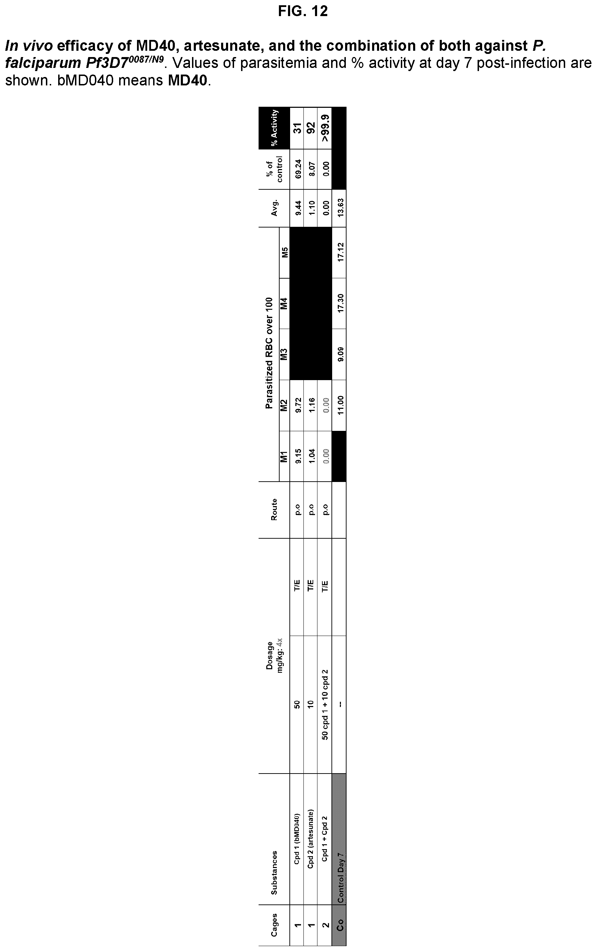

**Figure 10** shows the *in vivo* therapeutic efficacy of MD40 in combination with artesunate in a humanised mouse model infected with P. *falciparum* strain Pf3D70087/N9. Infected mice were treated daily for 4 days per os with **MD40** (4x 50mg/kg p.o.), artesunate (4x 10mg/kg p.o.) or the combination of both **(MD40** 4x 50mg/kg and artesunate 4x 10mg/kg). Arrows indicate the days of treatment in the 4-day Peters' test. The values correspond to the average parasitemia in peripheral blood of n=2 mice (n=4 mice for the control mice). Data for Figure 10 are summarised in Tables of Figures 11 and 12.

On day 7 post-infection, **MD40** in combination with artesunate showed >99.9% efficacy against *P*. *falciparum* (Table of figure 12). Both mice were parasite-free on day 5 post-infection. When dosed separately, the two compounds were much less effective (31% and 92% reduction upon MD40 and artesunate treatment, respectively).

*Parasite recrudescence data:* On day 14, when the experiment was stopped, both mice treated with **MD40** in combination with artesunate showed still no signs of recrudescence (p= 0.00 and 0.00%).

In summary, **MD40** at 4x 50 mg/kg p.o. in combination with artesunate at 4x 10/mg/kg po induced temporary clearance of parasites from peripheral blood at least until day 14 post-infection.

While no drug alone could cure infected mice, treatment with a combination of **MD40** and artesunate led to complete parasite clearance within 2 days. These data indicate a strong synergism between the two molecules *in vivo.* The concentrations of the partners should be optimized, and other endoperoxide drugs should also be tested *in vivo* in combination with **MD40.**

## Claims

1. Compound of formula (I): 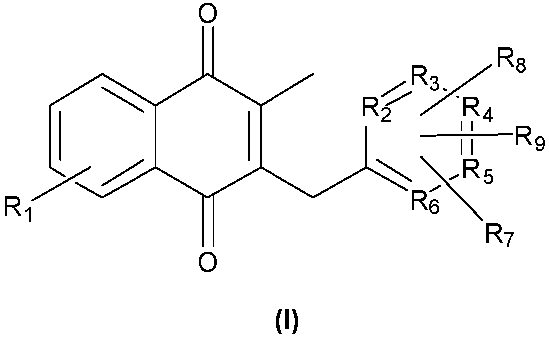 in which:
-R₁ represents -H or one substituent select among:
a -CN; -NO₂, -NH₂, a methyl group or a halogen atom, and preferably the halogen is a -F atom;
=R₂- ; =R₃ ; =R₄- ; =R₅- and =R₆- represent carbon atoms, with the exception that at least one of said carbon atoms is eventually replaced by one heteroatom selected among =P- or =N- , and preferably a =N-;
-R₇ represents -CF₃ -; and
-R₈ and -R₉ represent independently a substituent comprising at least a functional group selected among an ether group or an amine group, and represent preferably a substituent according to the chemical structure in between the following brackets: 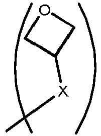
where -X- represents a chemical group comprising a heteroatom such as -NH-; -PH-; -S-or -O-, and preferably -NH or -O-;
or one of its pharmaceutically acceptable salts, hydrates, solvates, esters or one of its optical isomers, racemates, diastereoisomers, enantiomers, tautomers, or a mixture thereof.

2. Compound according to claim 1, wherein said compound is a compound according to formula (I'): 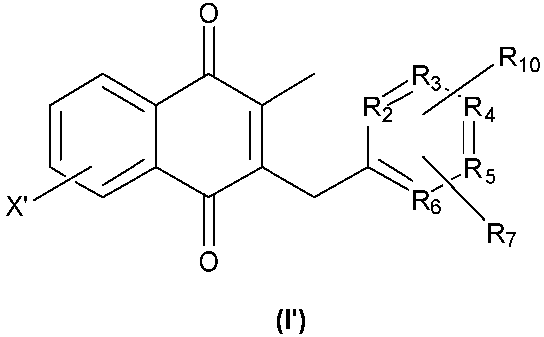 in which =R₂-; =R₃; =R₄-; =R₅- and =R₆- represent carbon atoms, with the exception that one or two, preferably one, of said carbon atoms is eventually replaced by a =N-;
-X'- represents a -H or a -F;
-R₇ represents -CF₃; and
-R₁₀ represents -NH₂ or a substituent according to the chemical structure in between the following brackets: 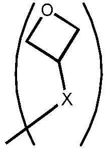
-X- represents -NH or -O-.

3. Compound according to claim 2, wherein said compound is according to formula (I') with:
=R₂- ; =R₄- and =R₆- represent carbon atoms, and =R₃ or =R₅- represents a carbon atom or a =N- atom; and preferably =R₂- ; =R₃ ; =R₄- ; =R₅- and =R₆- represent carbon atoms; and
-R₁₀ represents -NH₂ or a substituent according to the chemical structure in between the following brackets: 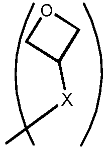
-X- represents -NH-.

4. Compound according to claim 2, wherein said compound is according to formula (I') with:
=R₂- ; =R₄- and =R₆- represent carbon atoms, and =R₃- or =R₅- represents a carbon atom or a =N- atom; and preferably =R₂- ; =R₃- ; =R₄- ; =R₅- and =R₆- represent carbon atoms; and
-R₁₀ represents a substituent according to the chemical structure in between the following brackets: 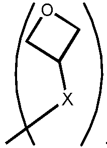 -X- represents -O-.

5. Compound according to claim 3, wherein said compound is according to one of the following formulae:

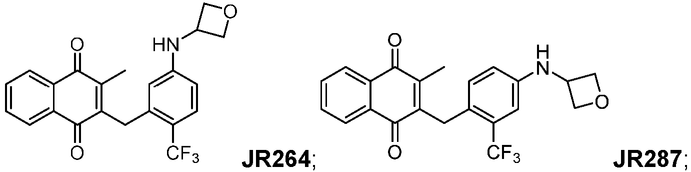

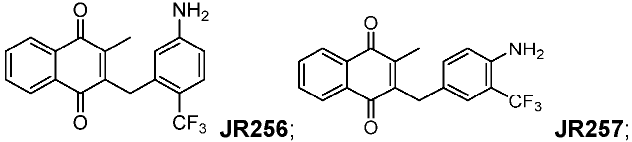

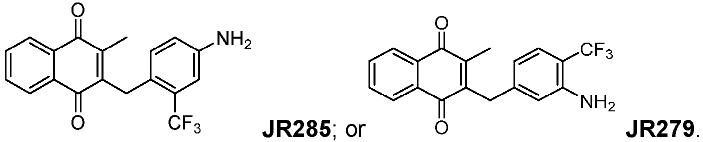

6. Compound according to claim 4, wherein said compound is according to one of the following formulae :

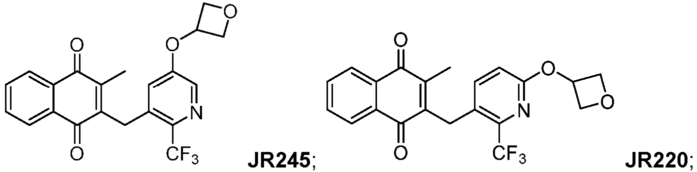

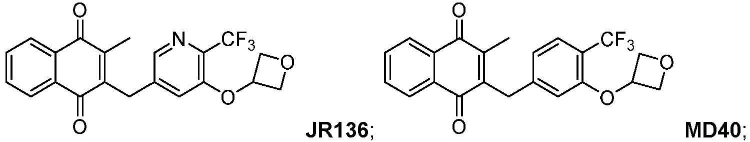

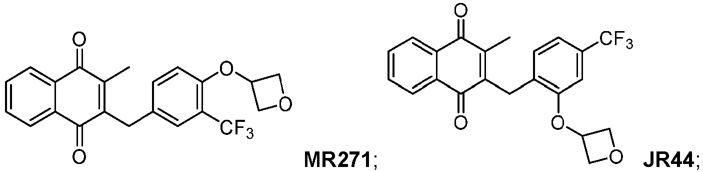

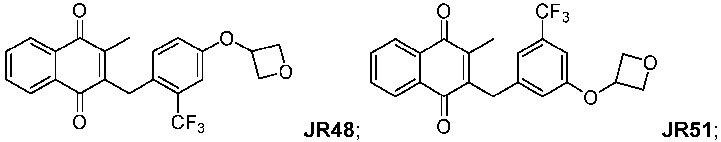

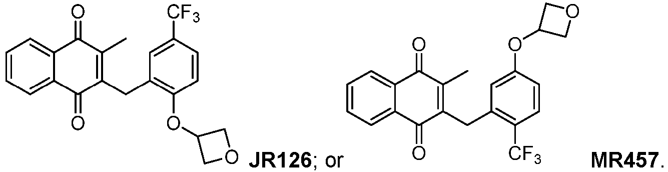

7. Compound according to claim 5 or 6, said compound having a structure according to the following formulae :

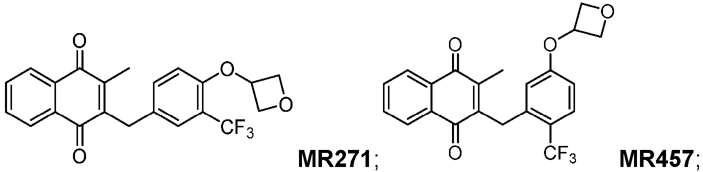

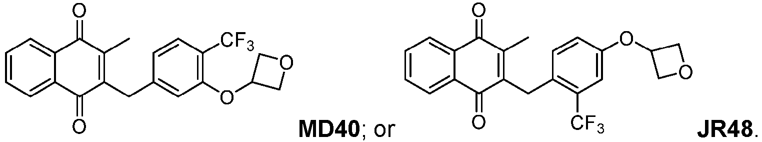

8. A compound according to any preceding claims for use as a medicament.

9. The compound according to claim 8 for the use in the treatment of a malaria, and preferably in the treatment of a multidrug resistant malaria.

10. The compound according to claim 8 or 9 for use in the treatment of artemisinin-resistant malaria.

11. The compound according to any of claims 8 to 10 for use in the treatment of a disease or disorder implying anti-plasmodial activities, preferably against P. *falciparum* asexual and sexual stages.

12. Pharmaceutical composition comprising at least one compound according to any one of claims 1 to 7 as active principle, and at least one additional compound selected among the followings:
- an endoperoxide, preferably selected among artemisinin or its metabolite dihydroartemisinin (DHA), artemether, arteether, artesunate;
- a peroxide belonging to 1,2,4-trioxanes or 1,2,4-trioxolanes, preferably selected among an ozonide derivative advantageously selected among ozonide OZ277 (arterolane) and ozonide OZ439 (artefenomel);
- a peroxide belonging to 1,2,4,5-tetraoxanes, preferably selected among RKA182 or RKA216;
- a natural bicyclic peroxides, preferably selected among 1,2-dioxanes plakortin or G3 factors;
- a compound selected among FR235222, XM172, ladanein (XM184), XM183, miconazole or econazole; and
preferably said additional compound is selected among artemisinin; artesunate arterolane and artefenomel.

13. Pharmaceutical composition according to claim 12, comprising optionally a pharmaceutically acceptable excipient.
